# EUROPEAN PATENT SPECIFICATION

(11) **EP 4 324 833 B1**
(45) Date of publication and mention of the grant of the patent: **09.12.2026**
(21) Application number: 21936693.7
(22) Date of filing: 09.09.2021
(51) Int. Cl.: C07D 487/04, A61K 31/495, A61P 35/00

(54) **N-PHENYL-3-(IMIDAZO[1,2-B]PYRIDAZIN-3-YL-ETHYNYL)-BENZAMIDE DERIVATIVES AS TRK (TROPOMYOSIN RECEPTOR KINASE) INHIBITORS FOR THE TREATMENT OF CANCER**
N-PHENYL-3-(IMIDAZO[1,2-B]PYRIDAZIN-3-YL-ETHYNYL)-BENZAMID-DERIVATE ALS TRK (TROPOMYOSIN-REZEPTOR-KINASE)-INHIBITOREN ZUR BEHANDLUNG VON KREBS
DÉRIVÉS DE N-PHÉNYL-3-(IMIDAZO[1,2-B]PYRIDAZIN-3-YL-ÉTHYNYL)-BENZAMIDE EN TANT QU'INHIBITEURS DE LA TRK (TROPOMYOSINE RECEPTOR KINASE) POUR LE TRAITEMENT DU CANCER

(30) Priority: 13.04.2021 CN 202110395086
(43) Date of publication of application: 21.02.2024
(73) Proprietor: Shenzhen Newdel Biotech Co., Ltd., Shenzhen, Guangdong 518000 (CN)
(72) Inventor: LU, Xiaoyun, Guangzhou, Guangdong 510632 (CN); ZHANG, Zhang, Guangzhou, Guangdong 510632 (CN); DING, Ke, Guangzhou, Guangdong 510632 (CN); XIANG, Shuang, Guangzhou, Guangdong 510632 (CN); WANG, Jie, Guangzhou, Guangdong 510632 (CN); TU, Zhengchao, Guangzhou, Guangdong 510632 (CN); ZHANG, Zhimin, Guangzhou, Guangdong 510632 (CN); TANG, Xia, Guangzhou, Guangdong 510632 (CN); WANG, Zuqin, Guangzhou, Guangdong 510632 (CN); HE, Xun, Shenzhen, Guangdong 518110 (CN); JIN, Feng, Shenzhen, Guangdong 518110 (CN); ZHANG, Shuihua, Shenzhen, Guangdong 518110 (CN); LI, Zhenwei, Shenzhen, Guangdong 518110 (CN)
(74) Representative: Mewburn Ellis LLP
(86) International application number: PCT/CN2021/117495
(87) International publication number: WO 2022/217821

(56) References cited:
- WO-A1-2013/101281
- WO-A1-2014/019338
- WO-A1-2021/047584
- WO-A2-2015/108490

## Description

### CROSS-REFERENCE TO RELATED APPLICATION

This application is a continuation of international application of PCT application serial no. PCT/CN2021/117495 filed on September 9, 2021, which claims the priority benefit of China application no. 202110395086.2 filed on April 13, 2021.

### BACKGROUND OF THE INVENTION

### 1. Technical Field

The present disclosure relates to the chemical and pharmaceutical technology, in particular to a kind of Alkynylphenylbenzamide compounds and the applications thereof.

### 2. Background Art

TRK (tropomyosin receptor kinase) belongs to the receptor tyrosine kinase (RTK) family, and has three subtypes: TRKA, TRKB, and TRKC, which are encoded respectively by NTRK1, NTRK2, and NTRK3 genes. TRK is a kind of transmembrane protein, which is composed of extracellular ligand binding domain, transmembrane domain (TM) and intracellular domain. TRK plays its role mainly by binding neurotrophic factors (NTs), which are a class of protein molecules that are produced by nerve-innervated tissues (such as muscles) and astrocytes and are necessary for neuronal growth and survival. At present, four main neurotrophic factors have been found, including NGF (nerve growth factor), brain-derived neurotrophic factor (BDNF), neurotrophic factor 3 (NT-3) and neurotrophic factor 4 (NT-4), among which NGF binds to TRKA, BDNF and NT-4 bind to TRKB, and NT-3 can bind to the three TRK proteins, but its binding ability to TRKC is stronger. When activated by signal induction, TRK activates downstream signaling pathways in turn through self-dimerization and phosphorylation to achieve various cellular physiological functions. The downstream signaling pathways of TRK include MAPK, PI3K/AKT, PLCγ/PKC pathways, which regulate physiological processes such as cell proliferation, differentiation, migration, and apoptosis, as well as various physiological activities related to neurons such as the elasticity of neural synapses, the growth and repair of neural dendrites, the prevention and repair of neuronal degradation, and the maintenance of sensory neurons.

Numerous studies have shown that TRK overexpression, gene fusion and single nucleotide changes are closely related to the occurrence and development of various types of tumors, such as non-small cell lung cancer, breast cancer, colon cancer, prostate cancer, thyroid cancer, malignant melanoma, neuroblastoma and breast-like secretory carcinoma, etc. Among the mechanisms of abnormal activation of TRK, the most prevalent mechanism is the gene fusion of TRK. The earliest NTRK fusion gene discovered in medical research was the TPM3-NTRK1 fusion gene found in colon cancer samples. With further in-depth research, researchers have successively discovered various types of fusion genes such as CD74-NTRK1, ETV6-NTRK2, QKI-NTRK2, and ETV6-NTRK3. The TRK fusion protein expressed by the NTRK fusion gene can continuously activate downstream signaling pathways independent of ligand binding, thereby inducing abnormal cell proliferation and promotes the occurrence and development of tumors. Therefore, TRK is regarded as an effective anticancer therapeutic target.

At present, a TRK selective inhibitor, Larotrectinib, developed by LOXO in the United States, was approved by the FDA in 2018; and Entrectinib, a TRK inhibitor developed by Roche Pharmaceuticals, was launched in Japan in June 2019; and Belizatinib, developed by TESARO, is undergoing clinical studies. In addition, multi-target inhibitors such as Cabozanitinib, Sitravatinib, and Altiratinib also have good TRK inhibitory activities.

The point mutation of NTRK gene caused by continuous use of TRK inhibitors is the key reason for the development of drug resistance in tumors. Clinical studies have successively discovered the mutations in G595R, G667C, F589L, G667S of NTRK1, and G623R and G696A of NTRK3. However, there are currently no inhibitors targeting these mutations on the market, and the second-generation of TRK inhibitors LOXO-195, TPX-0005 and ONO-5390556 are in clinical studies.

WO 2014/019338 A1 describes novel alkynyl derivatives with inhibitory activity on protein kinases and a pharmaceutical composition for preventing or treating diseases involving abnormal cell growth.

WO 2015/108490 A2 describes certain heteroaryl compounds that act as kinase inhibitors, for the prevention and treatment of diseases (e.g., proliferative diseases (e.g., cancer), inflammatory diseases, autoimmune disease, metabolic diseases, neurodegenerative diseases (e.g., Alzheimer's disease), or neurodevelopmental disorders (e.g., autism)).

WO 2013/101281 A1 describes methods and compositions for treating or preventing Parkinson's disease by administering an Abl inhibitor or a pharmaceutically acceptable salt thereof.

WO 2021/047584 A1 describes compounds which can inhibit TRK family protein tyrosine kinases and may be useful for the treatment of hyper-proliferative diseases like cancer and inflammation, or immune and autoimmune diseases.

### SUMMARY OF THE INVENTION

Based on the above, the present disclosure provides a new class of Alkynylphenylbenzamide compounds or their pharmaceutically acceptable salts or stereoisomers, which can be used as protein kinase inhibitors, and can effectively inhibit the activity of TRK protein kinase as well as the proliferation, migration and invasion of various tumor cells, especially with good pharmacokinetic properties and anti-drug resistance.

The detailed technical solutions are as follows:

The Alkynylphenylbenzamide compounds with the structure shown in Formula (I) or their pharmaceutically acceptable salts or stereoisomers: 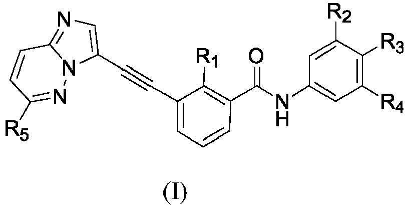
wherein, R₁ is selected from: C₁~C₂₀ alkyl;
R₂ is selected from: H, halogen, C₁~C₂₀ alkyl, C₁~C₂₀ alkoxy and halogen-substituted C₁~C₂₀ alkyl;
R₃ is selected from: H, difluoromethyl, difluoroethyl, trifluoromethyl and trifluoroethyl;
R₄ is selected from: H, halogen, nitro, C₁~C₈ alkyl, halogen-substituted C₁~C₈ alkyl, C₁~C₈ alkoxy, halogen-substituted C₁~C₈ alkoxy, -(CH₂)ₓNR₁₇R₁₈, 5-6 membered heterocyclyl group containing 1-3 N ring atoms and substituted or unsubstituted by 1-5 R₁₉, and 5-6 membered heteroaryl containing 1-3 N ring atoms and substituted or unsubstituted by 1-5 R₁₉; wherein, x is an integer from 1 to 5; R₁₇ and R₁₈ together with the attached nitrogen atom form a 1-5 R₁₉ substituted or unsubstituted morpholinyl, pyrrolidinyl, piperidinyl or piperazinyl; each R₁₉ is independently selected from: C₁-C₅ alkyl;
R₅ is -NR₆R₇;
   wherein, R₆ and R₇ are independently selected from: -(CH₂)ₘNR₈R₉, -(CH₂)ₙCR₁₀R₁₁R₁₂, and - (CH₂)ₚOR₁₂; or R₆ and R₇ together with the attached nitrogen atom form a 3-15 membered monocyclic ring, fused ring, spiro ring or bridged ring containing 1-3 heteroatoms and substituted or unsubstituted by 1-5 R₁₃, wherein, the heteroatoms are selected from: O, N, S;
R₈ and R₉ are independently selected from: H, and C₁-C₅ alkyl; or R₈ and R₉ together with the attached nitrogen atom form a 3-10 membered monocyclic ring, fused ring, spiro ring or bridged ring containing 1-3 heteroatoms and substituted or unsubstituted by 1-5 R₁₃, wherein, the heteroatoms are selected from: O, and N;
R₁₀ and R₁₁ together with the attached carbon atoms form a 3-10 membered monocyclic ring, fused ring, spiro ring or bridged ring containing 1-3 heteroatoms and substituted or unsubstituted by 1-5 R₁₃, wherein the heteroatoms are selected from: O, and N;
R₁₂ is selected from: H, and C₁-C₅ alkyl;
each R₁₃ is independently selected from: H, C₁-C₅ alkyl, C₁-C₅ alkanoyl, hydroxy, hydroxy-substituted C₁-C₅ alkyl, amino-substituted C₁-C₅ alkyl, amino-substituted C₁-C₅ alkoxy, C₃-C₇ cycloalkyl-substituted C₁-C₃ alkyl, -NR₁₅R₁₆, and 3-10 membered monocyclic ring, fused ring, spiro ring or bridged ring substituted or unsubstituted by 1-5 R₁₄ and containing 1-3 heteroatoms, wherein, the heteroatoms are selected from: O, and N;
R₁₄, R₁₅ and R₁₆ are independently selected from: H, and C₁-C₅ alkyl;
m, n and p are each independently selected from: an integer from 0 to 5.

In some embodiments, R₄ is selected from: H, halogen, nitro, C₁~C₄ alkyl, halogen-substituted C₁~C₄ alkyl, C₁~C₄ alkoxy, halogen-substituted C₁~C₄ alkoxy, -(CH₂)ₓNR₁₇R₁₈, 5-6 membered heterocyclyl group containing 1-3 N ring atoms and substituted or unsubstituted by 1-3 R₁₉, and 5-6-membered heteroaryl containing 1-3 N ring atoms and substituted or unsubstituted by 1-3 R₁₉; wherein, x is an integer from 1 to 5.

In some embodiments, R₄ is selected from: H, halogen, nitro, C₁~C₄ alkyl, halogen-substituted C₁~C₄ alkyl, C₁~C₄ alkoxy, halogen-substituted C₁~C₄ alkoxy, -(CH₂)ₓNR₁₇R₁₈, and 1-3 R₁₉ substituted or unsubstituted imidazolyl; wherein, x is 1, 2 or 3;
R₁₇ and R₁₈ together with the attached nitrogen atom form a piperazinyl substituted or unsubstituted by 1-3 R₁₉;
each R₁₉ is independently selected from: C₁-C₅ alkyl.

In some embodiments, R₄ is selected from: H, halogen, nitro, methyl, ethyl, propyl, methoxy, ethoxy, propoxy, trifluoromethyl, trifluoroethyl, 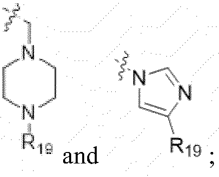 each R₁₉ is independently selected from: methyl, ethyl, and propyl.

In some embodiments, R₆ and R₇ are independently selected from: -(CH₂)ₘNR₈R₉, and - (CH₂)ₚOR₁₂; or R₆ and R₇ together with the attached nitrogen atom form a 1-3 R₁₃ substituted or unsubstituted morpholinyl, pyrrolidinyl, piperidinyl or piperazinyl;
R₈ and R₉ are independently selected from: H, and C₁-C₅ alkyl; or R₈ and R₉ together with the attached nitrogen atom form a 1-5 R₁₃ substituted or unsubstituted morpholinyl, pyrrolidinyl, piperidinyl or piperazinyl;
R₁₂ is selected from: H, and C₁-C₅ alkyl;
each R₁₃ is independently selected from: H, C₁-C₅ alkyl, C₁-C₅ alkanoyl, hydroxy, -NR₁₅R₁₆, oxetanyl substituted or unsubstituted by 1-2 R₁₄, and morpholinyl substituted or unsubstituted by1-4 R₁₄;
R₁₄, R₁₅ and R₁₆ are independently selected from: H, and C₁-C₃ alkyl;
m and p are independently selected from: 1, 2, 3, 4 and 5.

In some embodiments, R₅ is selected from any of the following groups:

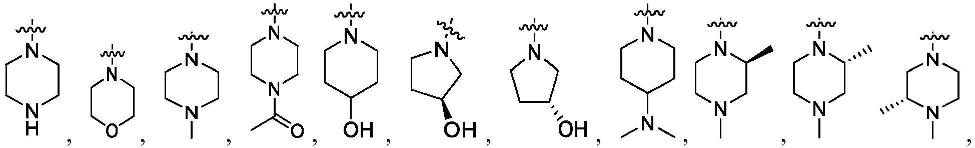

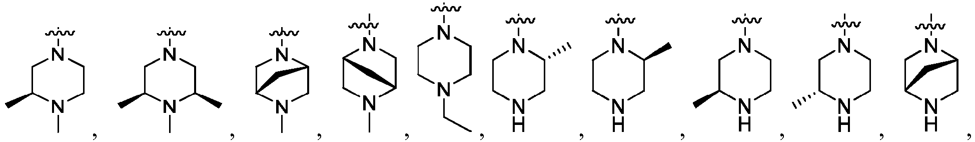

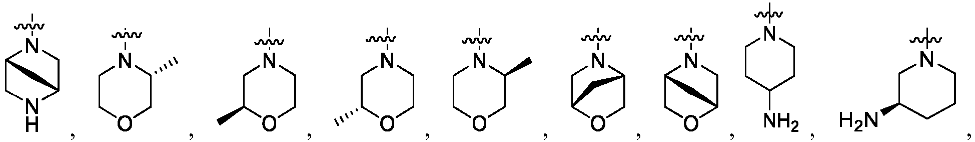

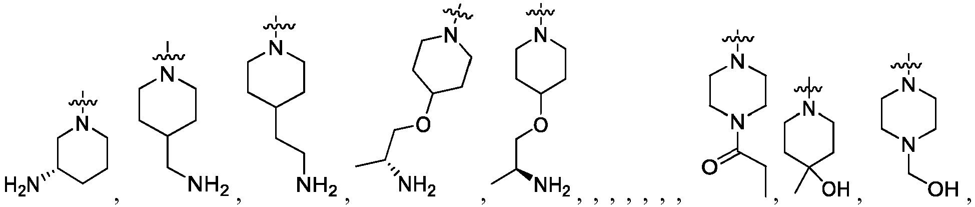

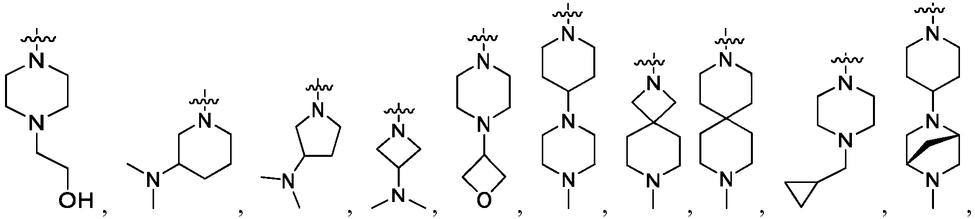

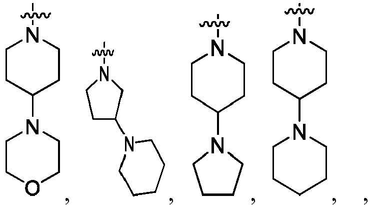

In some embodiments, R₄ is halogen; R₅ is -NR₆R₇;
R₆ and R₇ are independently selected from: -(CH₂)ₘNR₈R₉, and -(CH₂)ₚOR₁₂; or R₆ and R₇ together with the attached nitrogen atom form a 1-3 R₁₃ substituted or unsubstituted morpholinyl, pyrrolidinyl, piperidinyl or piperazinyl ;
R₈ and R₉ are independently selected from: H, and C₁-C₃ alkyl; or R₈ and R₉ together with the attached nitrogen atom form a 1-2 R₁₃ substituted or unsubstituted morpholinyl, pyrrolidinyl, piperidinyl or piperazinyl;
R₁₂ is selected from: H, and C₁-C₃ alkyl;
each R₁₃ is independently selected from: H, C₁-C₃ alkyl, acetyl, hydroxyl, -NR₁₅R₁₆, oxetanyl, and morpholinyl;
R₁₅ and R₁₆ are independently selected from: H, and C₁-C₃ alkyl;
m and p are independently selected from: 2, 3 and 4.

In some embodiments, R₄ is Cl,
R₅ is selected from:

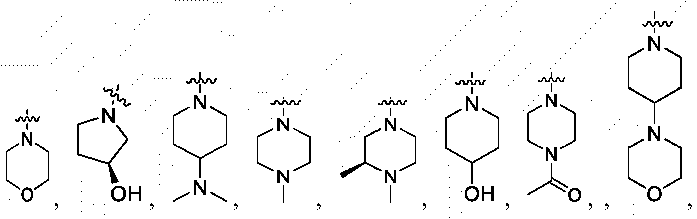

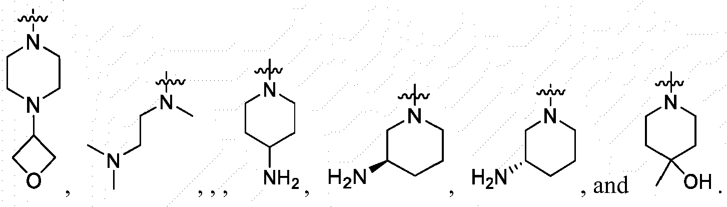

In some embodiments, R₄ is selected from: H, halogen, methyl, methoxy, trifluoromethyl, nitro, and 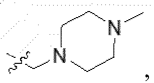 and R₅ is

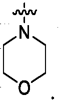

In some embodiments, R₄ is selected from H, and 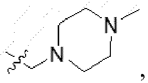 and R₅ is selected from

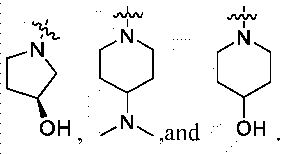

In some embodiments, R4 is 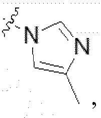 and R₅ is selected from

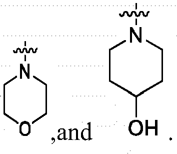

In some embodiments, R₁ is selected from: C₁~C₁₀ alkyl.

In some embodiments, R₁ is selected from: C₁∼C₄ alkyl.

In some embodiments, R₁ is selected from: methyl, ethyl, isopropyl, and tert-butyl.

In some embodiments, R₂ is selected from: H, halogen, C₁-C₁₀ alkyl, and halogen-substituted C₁-C₁₀ alkyl.

In some embodiments, R₂ is selected from: H, halogen, C₁-C₄ alkyl, halogen-substituted C₁-C₄ alkyl, and C₁-C₄ alkoxy.

In some embodiments, R₂ is selected from: hydrogen, fluorine, methyl, ethyl, isopropyl, tert-butyl, difluoromethyl, difluoroethyl, trifluoromethyl and trifluoroethyl.

In some embodiments, the Alkynylphenylbenzamide compounds have the structure shown in Formula (II):

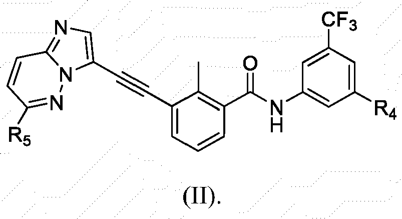

Another object of the present disclosure is to provide use of the above-mentioned Alkynylphenylbenzamide compounds or their pharmaceutically acceptable salts or stereoisomers in the preparation of TRK inhibitors.

Another object of the present disclosure is to provide use of the above-mentioned Alkynylphenylbenzamide compounds or their pharmaceutically acceptable salts or stereoisomers in the preparation of medicaments for preventing and/or treating the diseases mediated by TRK tyrosine kinases.

In some embodiments, the disease mediated by TRK tyrosine kinase is tumor, preferably non-small cell lung cancer, breast cancer, colon cancer, prostate cancer, thyroid cancer, malignant melanoma, nerve blastoma and breast-like secretory carcinoma.

Another object of the present disclosure is to provide a pharmaceutical composition for preventing and/or treating tumors, comprising an active ingredient and a pharmaceutically acceptable adjuvant, wherein the active ingredient comprises the above-mentioned Alkynylphenylbenzamide compounds or their pharmaceutically acceptable salts or stereoisomers.

The Alkynylphenylbenzamide compounds provided by the present disclosure have strong inhibitory activity on TRKs kinase, and have strong inhibitory activity on the proliferation of wild-type and drug-resistant cells of Ba/F3-TRKs stable strain. It can be used for preparation of medicaments for preventing or treating diseases mediated by TRK tyrosine kinase, such as non-small cell lung cancer, breast cancer, colon cancer, prostate cancer, thyroid cancer, malignant melanoma, nerve Blastoma and breast-like secretory carcinoma. It also has good pharmacokinetic properties and low toxicity.

### BRIEF DESCRIPTION OF THE DRAWINGS

Fig. 1 shows the in vivo antitumor activity of compound XS3-55.
Fig. 2 shows the effect of compound XS3-55 on the body weight of mice.

### DETAILED DESCRIPTION OF THE INVENTION

The experimental methods without indicating specific conditions in the following examples of the present disclosure are usually in accordance with conventional conditions, or in accordance with the conditions suggested by the manufacturer. Various common chemical reagents used in the examples are all commercially available products.

Unless otherwise defined, all technical and scientific terms as used herein have the same meanings as those commonly understood by one skilled in the art of the present disclosure. The terms used in the description of the present disclosure are for description of the specific embodiments only and are not intended to limit the present disclosure.

The terms "comprising" and "having" and any variations thereof of the present disclosure are intended to cover a non-exclusive inclusion. For example, a process, method, apparatus, product or device comprising a series of steps is not limited to the steps or modules listed, but optionally also includes steps not listed, or optionally includes other steps inherent to the process, method, product, or apparatus.

"Plurality" mentioned in the present disclosure means two or more. "And/or", which describes the association relationship of the associated objects, means that there can be three kinds of relationships, for example, A and/or B, which can mean that A exists alone, A and B exist at the same time, and B exists alone. The character "/" generally indicates that the associated objects are an "or" relationship.

In the compounds of the present disclosure, when any variable (eg, R₁₀, R₁₁, etc.) occurs more than once in any component, its definition at each occurrence is independent of the definition at each other occurrences. Likewise, combinations of substituents and variables are permissible as long as such combinations stabilize the compound. The line drawn into a ring system from a substituent indicates that the indicated bond may be attached to any substitutable ring atom. If the ring system is polycyclic, it means that such bonds are only attached to any suitable carbon atoms adjacent to the ring. It should be appreciated that an ordinary skilled in the art can select substituents and substitution patterns for the compounds of the present disclosure to provide compounds that are chemically stable and readily synthesized from the available starting materials by the methods described below. If a substituent itself is substituted by more than one group, it should be understood that these groups may be on the same carbon atom or on different carbon atoms, so long as the structure is stabilized.

The term "alkyl" in the present disclosure is meant to include branched and straight chain saturated aliphatic hydrocarbon groups having the specified number of carbon atoms. For example, the definition of "C₁-C₂₀" in "C₁-C₂₀ alkyl" includes groups having 1, 2, 3, 4, 5 ...or 20 carbon atoms arranged in a straight or branched chain. The term "cycloalkyl" refers to a monocyclic saturated aliphatic hydrocarbon group having the specified number of carbon atoms. For example, " C₃~C₇ cycloalkyl" includes cyclopropyl, cyclobutyl, cyclopentyl, cyclohexyl or cycloheptyl.

The term "alkoxy" as used herein refers to a group in which an alkyl group is directly attached to oxygen, i.e. a group with an -O-alkyl structure, such as -OCH₃, -OCH₂CH₃, - OCH₂CH₂CH₃, -O-CH₂CH(CH₃)₂, -OCH₂CH₂CH₂CH₃, -O-CH(CH₃)₂, etc.

The term "heterocyclyl" as used herein refers to a non-aromatic heterocyclic group containing one or more heteroatoms selected from O, N and S, for example: piperidinyl, tetrahydropyrrolyl (pyrrolidinyl), morpholino, piperazinyl, etc. The attachment of heterocyclic substituents can be achieved by carbon atoms or heteroatoms.

The term "heteroaryl" as used herein refers to an aromatic ring containing one or more heteroatoms selected from O, N or S. Heteroaryl groups within the scope of the present disclosure include, but not limited to: quinolinyl, pyrazolyl, pyrrolyl, thienyl, furanyl, pyridyl, pyrimidinyl, pyrazinyl, triazolyl, imidazolyl, oxazolyl, isoxazolyl, pyridazinyl, benzoyl Furanyl, benzothienyl, benzoxazole, indolyl, etc.; "heteroaryl" can also be understood to include the N-oxide derivatives of any nitrogen-containing heteroaryl groups. The attachment of heteroaryl substituents can be through carbon atoms or through heteroatoms.

The term "substituted" as used herein refers to the replacement of a hydrogen group in a particular structure with a group of the designated substituent.

As understood by the skilled in the art, "halogen" as used herein means chlorine, fluorine, bromine and iodine.

The present disclosure comprises free forms of compounds of Formula (I) or Formula (II), as well as their pharmaceutically acceptable salts and stereoisomers. The term "free form" refers to compound in non-salt form. The pharmaceutically acceptable salts" include not only exemplary salts of the particular compounds described herein, but also typical pharmaceutically acceptable salts of all compounds of Formula (I) or Formula (II) in free form. The free forms of specific salts of the compounds can be isolated using the techniques known in the art. For example, the free form can be regenerated by treating the salt with an appropriate dilute aqueous base such as dilute aqueous NaOH, dilute aqueous potassium carbonate, dilute aqueous ammonia, and dilute aqueous sodium bicarbonate. The free forms differ somewhat from their respective salt forms in certain physical properties such as solubility in polar solvents, but for the purposes of the invention, such salts of acid or base are otherwise pharmaceutically equivalent to their respective free forms.

The pharmaceutically acceptable salts of the present disclosure can be synthesized from the compounds containing a basic or acidic moiety in the present disclosure by conventional chemical methods. Generally, salts of basic compounds can be prepared by ion exchanged chromatography or by reacting the free base with a stoichiometric or excess amount of inorganic or organic acid in the desired salt form in a suitable solvent or combination of solvents. Similarly, salts of acidic compounds can be formed by reaction with a suitable inorganic or organic base.

Accordingly, pharmaceutically acceptable salts of the compounds of the present disclosure include conventional non-toxic salts of the compounds of the present disclosure formed by reacting a basic compound of the present disclosure with inorganic or organic acid. For example, conventional non-toxic salts include those derived from inorganic acids such as hydrochloric acid, hydrobromic acid, sulfuric acid, sulfamic acid, phosphoric acid, and nitric acid, etc. They also include those derived from organic acids such as acetic acid, propionic acid, succinic acid, glycolic acid, hard Fatty acid, lactic acid, malic acid, tartaric acid, citric acid, ascorbic acid, pamoic acid, maleic acid, hydroxymaleic acid, phenylacetic acid, glutamic acid, benzoic acid, salicylic acid, p-aminobenzenesulfonic acid, 2 - acetoxy - benzoic acid, fumaric acid, benzenesulfonic acid, toluenesulfonic acid, methanesulfonic acid, ethanedisulfonic acid, oxalic acid, isethionic acid, and trifluoroacetic acid, etc.

If the compounds of the present disclosure are acidic, the appropriate "pharmaceutically acceptable salts" refers to the salts prepared with pharmaceutically acceptable non-toxic bases including inorganic and organic bases. The salts derived from inorganic bases include aluminum, ammonium, calcium, copper, iron, ferrous, lithium, magnesium, manganese, manganous, potassium, sodium, zinc, etc. Ammonium salts, calcium salts, magnesium salts, potassium salts, and sodium salts are particularly preferred. The salts derived from pharmaceutically acceptable organic non-toxic bases include salts of primary, secondary and tertiary amines. Substituted amines include naturally occurring substituted amines, cyclic amines and basic ion exchange resins such as Amino acid, betaine, caffeine, choline, N, N'-dibenzylethylenediamine, diethylamine, 2-diethylaminoethanol, 2-dimethylaminoethanol, aminoethanol, ethanolamine, ethyl Diamine, N-ethylmorpholine, N-ethylpiperidine, Glucosamine, Glucosamine, Histidine, Hydroxocobalamin, Isopropylamine, Lysine, Methylglucamine, Morpholine, Piperazine, Piperidine, quack, polyamine resin, procaine, purine, theobromine, triethylamine, trimethylamine, tripropylamine, tromethamine, etc.

The preparation of the pharmaceutically acceptable salts described above and other typical pharmaceutically acceptable salts was described in more detail by Berg et al., in "Pharmaceutical Salts," J. Pharm. Sci. 1977:66:1-19.

The following are specific examples to further describe the present disclosure in detail.

### Reference Example 1: Preparation of Reference Compound 3-(imidazo[1,2-b]pyridazin-3-ylethynyl)-2-methyl-N-(3-((4-methylpiperazin-1-yl)methyl)-5-(trifluoromethyl)phenyl)benzamide (designated as XS116)

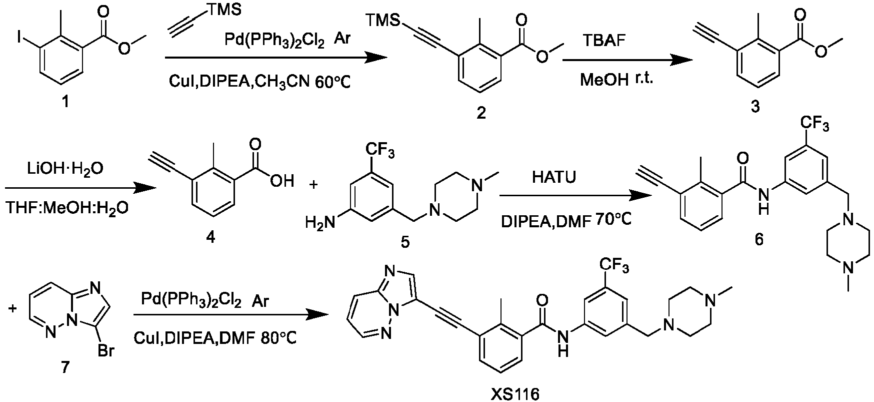

### Step 1: Preparation of methyl 2-methyl-3-((trimethylsilyl)ethynyl)benzoate (Compound 2)

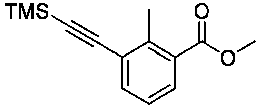

In a 500 mL three-necked flask, 10 g (36 mmol) of Compound 1, 689 mg (3.6 mmol) of cuprous iodide, 1.27 g (1.8 mmol) of bis(triphenylphosphine)palladium dichloride, 150 mL anhydrous acetonitrile and 9.3 g (72 mmol) of N, N - diisopropylethylamine were added to react under Ar in a closed system. Then 10.6 g (108 mmol) of trimethylsilylacetylene was injected with a syringe, and the mixture was stirred at 60° C for 6 hours. The reaction solution was filtered through celite, and the solvent was spin-dried to obtain a black mixture, which was directly used in the next reaction.

### Step 2: Preparation of methyl 3-ethynyl-2-methylbenzoate (Compound 3)

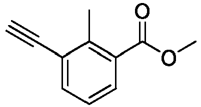

The crude product in the previous step was dissolved in methanol, and added with about 20 mL of 1 mol/L tetrabutylammonium fluoride solution in tetrahydrofuran, and the mixture was stirred at room temperature for 2 hours. The reaction system was spin-dried, and 4 g of yellow-brown oil was obtained by column chromatography (total yield of the two steps was 63%).

¹H NMR (400 MHz, DMSO-*d*₆) δ 7.80 (s, 1H), 7.78 (s, 1H), 7.58 (s, 1H), 4.27 (s, 1H), 3.85 (s, 3H), 2.37 (s, 3H). LC-MS (ESI) m/z 175.5[M + H]⁺.

### Step 3: Preparation of 3-ethynyl-2-methylbenzoic acid (Compound 4)

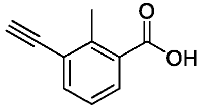

1.5 g (9 mmol) of Compound 3 was dissolved in a mixed solvent of tetrahydrofuran, methanol and water with a volume ratio of 10:1:5, then 1.8 g (40 mmol) of lithium hydroxide hydrate was added, and the mixture was stirred at 60°C for 1 hour. The reaction system was filtered and spin-dried, and then was added with 4M hydrochloric acid solution until the system became acidic. At this time a white solid is precipitated, which was collected by filtration, and dried to obtain 900 mg of white solid (yield: 65%).

¹H NMR (400 MHz, DMSO-d6) δ 13.09 (s, 1H), 7.77 (dd, J = 7.8, 0.9 Hz, 1H), 7.62 (d, J = 7.6 Hz, 1H), 7.29 (t, J = 7.7 Hz, 1H), 4.47 (s, 1H), 2.61 (s, 3H). LC-MS (ESI) m/z 158.9[M - H]⁻ .

### Step 4: Preparation of 3-ethynyl-2-methyl-N-(3-((4-methylpiperazin-1-yl)methyl)-5-(trifluoromethyl)phenyl)benzamide (Compound 6)

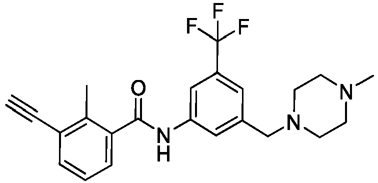

2.3 g of Compound 4 and 3.4 g of Compound 5 were dissolved in 40 mL of N,N-dimethylformamide (DMF), added with 9.12 g (24 mmol) of 2-(7-Azabenzotriazole)-N,N,N',N'-tetramethylurea hexafluorophosphate (HATU) and 2.3 g (18 mmol) of N,N-diisopropylethyl acetate amine; and the mixture was heated and stirred at 70°C for 2 hours. The reaction system was spin-dried, and added with water, extracted with ethyl acetate, rinsed with water, dried with anhydrous sodium sulfate. The solvent was spin-dried, and 3.4 g of yellow oil was obtained by column chromatography (yield: 70%).

¹H NMR (400 MHz, DMSO-*d*₆) δ 10.52 (s, 1H), 8.17 (s, 1H), 8.10 (s, 1H), 8.00(d, J = 8.0 Hz, 1H), 7.91 (m, 1H), 7.47 (d, J = 8.0 Hz, 1H), 7.34 (s, 1H), 4.51 (s, 1H),3.53 (s, 2H), 2.46 (s, 3H), 2.39 (s, 4H), 2.33 (s, 4H), 2.15 (s, 3H). LC-MS (ESI) m/z 416.3 [M + H] ⁺ .

### Step 5: Preparation of 3-(imidazo[1,2-b]pyridazin-3-ylethynyl)-2-methyl-N-(3-((4-methylpiperazin-1-yl)methyl) )-5-(trifluoromethyl)phenyl)benzamide (XS116)

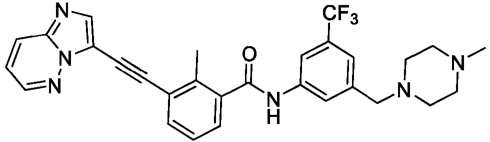

210 mg (0.51 mmol) of Compound 6 and120 mg (0.61 mmol) Compound 7 were dissolved in 10 mL of anhydrous N, N-dimethylformamide (DMF), then added with 19 mg (0.1 mmol) of cuprous iodide, 35 mg (0.05 mmol) of bis(triphenylphosphine)palladium dichloride and 131 mg (1.02 mmol) of N,N-diisopropylethylamine, reacted under Ar in a closed system. The mixture was heated and stirred at 80°C and reacted overnight. The reaction solution was filtered through celite, the solvent was spin-dried, and 70 mg of yellow-white solid was obtained by column chromatography (yield: 26%).

¹H NMR (400 MHz, Chloroform-d) δ 8.50 (dd, *J* = 4.4, 1.6 Hz, 1H), 8.07 (s, 1H), 8.02 (dd, *J* = 9.2*,* 1.7 Hz, 1H), 7.94 (s, 1H), 7.81 (d, *J =* 4.5 Hz, 2H), 7.73 (dd, *J =* 7.7, 1.4 Hz, 1H), 7.50 (d, *J* = 7.8 Hz, 1H), 7.41 (s, 1H), 7.32 (t, *J* = 7.7 Hz, 1H), 7.16 (dd, *J* = 9.2, 4.4 Hz, 1H), 3.62 (s, 2H), 2.76 (s, 3H), 2.64 (s, 8H), 2.41 (s, 3H). LC-MS (ESI) m/z 533.3[M + H]⁺ .

### Reference Example 2: Preparation of Reference Compound 3-(imidazo[1,2-a]pyrimidin-3-ylethynyl)-2-methyl-N-(3-((4-methylpiperazin-1-yl)methyl)-5-(trifluoromethyl)phenyl)benzamide (designated as XS2-161)

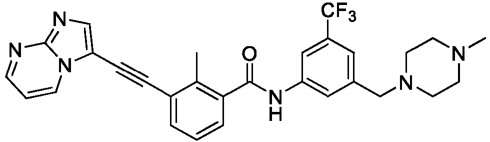

The synthetic method is according to Example 1.

¹H NMR (400 MHz, Chloroform-d) δ 9.06 (s, 1H), 8.64 (dd, J = 4.1, 2.0 Hz, 1H), 8.60 (dd, J = 6.8, 2.0 Hz, 1H), 8.12 (s, 1H), 8.03 (s, 1H), 7.90 (s, 1H), 7.58 (dd, J = 7.8, 1.3 Hz, 1H), 7.50 (dd, J = 7.8, 1.3 Hz, 1H), 7.40 (s, 1H), 7.25 (t, J = 7.7 Hz, 1H), 7.09 (dd, J = 6.8, 4.1 Hz, 1H), 3.70 (s, 2H), 3.11 (q, J = 7.3 Hz, 4H), 2.91 (d, J = 5.0 Hz, 4H), 2.73 (s, 3H), 2.61 (s, 3H). LC-MS (ESI) m/z 533.2[M + H]⁺ .

### Reference Example 3: Preparation of Reference Compound 3-(imidazo[1,2-b]pyridazin-3-ylethynyl)-2-methyl-N-(3-(trifluoromethyl)phenyl)benzamide (designated as XS2-106 )

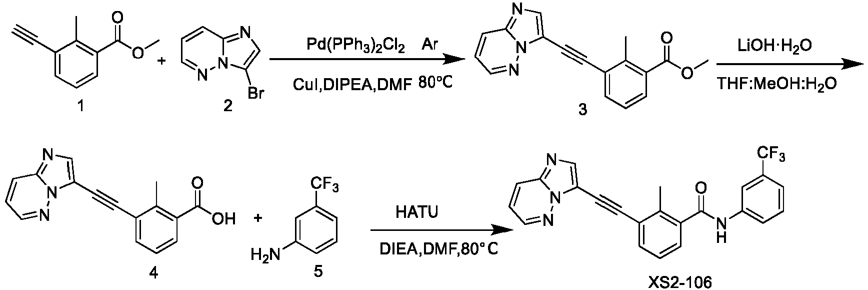

### Step 1: Preparation of methyl 3-(imidazo[1,2-b]pyridazin-3-ylethynyl)-2-methylbenzoate (Compound 3)

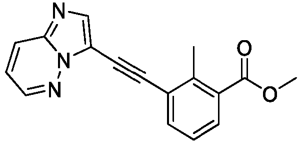

2.5 g (14 mmol) of Compound 1 and 3.4 g (17 mmol) of Compound 2 were dissolved in 40 mL of anhydrous N, N-dimethylformamide (DMF), and then added with 533 mg (2.8 mmol) of cuprous iodide, 982 mg (1.4 mmol) of bis(triphenylphosphine)palladium and 3.6 g (28 mmol) of N, N-diisopropylethylamine, and then reacted under Ar in a closed system. The mixture was heated and stirred at 80°C and reacted overnight. The reaction solution was filtered through celite, the solvent was spin-dried, and 1.38 g of yellow powdery solid was obtained by column chromatography (yield: 34%).

¹H NMR (400 MHz, DMSO-d6) δ 8.74 (d, J = 4.3 Hz, 1H), 8.27 (d, J = 8.9 Hz, 2H), 7.83 (d, J = 7.7 Hz, 1H), 7.75 (d, J = 7.5 Hz, 1H), 7.44 - 7.41 (m, 1H), 7.39 (d, J = 7.6 Hz, 1H), 3.85(s, 3H) ^{,} 2.76 (s, 3H). LC-MS (ESI) m/z 292.3[M + H]⁺ .

### Step 2: Preparation of 3-(imidazo[1,2-b]pyridazin-3-ylethynyl)-2-methylbenzoic acid (Compound 4)

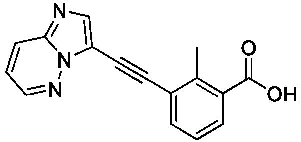

1.38 g (4.7 mmol) of Compound 3 was dissolved in a mixed solvent of tetrahydrofuran, methanol and water with a volume ratio of 10:1:5, then 995 mg (24 mmol) of lithium hydroxide hydrate was added, and the mixture was stirred at 60°C for 1 hour. The reaction system was filtered and spin-dried, and then added with 4M of hydrochloric acid solution until the system became acidic. At this time a solid is precipitated, which was collected by filtration, and dried to obtain 1.05g of yellow solid (yield: 81%).

¹H NMR (400 MHz, DMSO-*d*₆) δ 13.14 (s, 1H), 8.72 (d, J = 3.9 Hz, 1H), 8.32 - 8.19 (m, 2H), 7.83 (d, J = 7.6 Hz, 1H), 7.75 (d, J = 7.3 Hz, 1H), 7.44 - 7.34 (m, 2H), 2.76 (s, 3H). LC-MS (ESI) m/z 276.8[M - H]⁻ .

### Step 3: Preparation of 3-(imidazo[1,2-b]pyridazin-3-ylethynyl)-2-methyl-N-(3-(trifluoromethyl)phenyl)benzamide (XS2- 106)

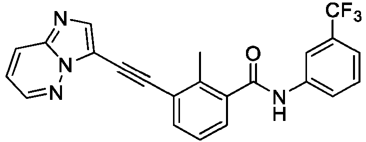

100 mg (0.36 mmol) of Compound 4 and 48 mg (0.3 mmol) of Compound 5 were dissolved in 10 mL of N, N-dimethylformamide (DMF), then added with 137 mg (0.36 mmol) of 2-(7-Azabenzotriazole)-N, N, N', N'-tetramethylurea hexafluorophosphate (HATU) and 77 mg (0.6 mmol) of N, N-diisopropylethyl acetate amine; and the mixture was heated and stirred at 80°C and reacted overnight. The reaction system was spin-dried, and 51 mg of yellow-white solid was obtained by column chromatography (yield 40%).

¹H NMR (400 MHz, Chloroform-d) δ 8.51 (d, J = 4.1 Hz, 1H), 8.03 (dd, J = 23.3, 14.3 Hz, 3H), 7.89 (d, J = 7.4 Hz, 1H), 7.73 (d, J = 6.6 Hz, 2H), 7.50 (ddd, J = 24.8, 16.6, 7.8 Hz, 3H), 7.32 (t, J = 7.7 Hz, 1H), 7.17 (dd, J = 9.1, 4.4 Hz, 1H), 2.76 (s, 3H). LC-MS (ESI) m/z 419.2[M - H]⁻ .

### Reference Example 4: Preparation of Reference Compound N-(3-fluoro-5-(trifluoromethyl)phenyl)-3-(imidazo[1,2-b]pyridazin-3-ylethynyl)-2-methylbenzamide (designated as XS2-109)

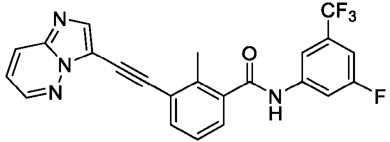

The synthetic method is according to Example 3.

¹H NMR (400 MHz, Chloroform-d) δ 8.53 (d, J = 4.2 Hz, 1H), 8.08 (s, 2H), 7.91 (d, J = 10.1 Hz, 1H), 7.81 (s, 1H), 7.74 (d, J = 7.8 Hz, 1H), 7.59 (s, 1H), 7.50 (d, J = 7.5 Hz, 1H), 7.33 (t, J = 7.7 Hz, 1H), 7.19 (s, 1H), 7.16 (d, J = 8.2 Hz, 1H), 2.75 (s, 3H). LC-MS (ESI) m/z 437.6[M - H]⁻ .

### Reference Example 5: Preparation of Reference Compound N-(3-chloro-5-(trifluoromethyl)phenyl)-3-(imidazo[1,2-b]pyridazin-3-ylethynyl)-2-methylbenzamide (designated as XS2-112)

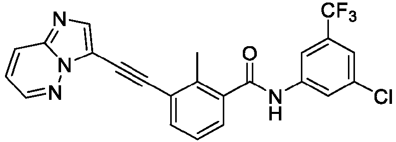

The synthetic method is according to Example 3.

¹H NMR (400 MHz, Chloroform-d) δ 8.50 (dd, J = 4.4, 1.5 Hz, 1H), 8.06 (d, J = 4.2 Hz, 2H), 8.01 (dd, J = 9.2, 1.6 Hz, 1H), 7.85 (s, 1H), 7.80 (s, 1H), 7.74 (d, J = 6.7 Hz, 1H), 7.48 (d, J = 6.9 Hz, 1H), 7.44 (s, 1H), 7.32 (t, J = 7.7 Hz, 1H), 7.16 (dd, J = 9.2, 4.4 Hz, 1H), 2.75 (s, 3H). LC-MS (ESI) m/z 455.5[M + H]⁺ .

### Example 6: Preparation of 2-methyl-3-((6-morpholinoimidazo[1,2-b]pyridazin-3-yl)ethynyl)-N-(3-(trifluoromethyl)phenyl)benzamide (designated as XS3-23)

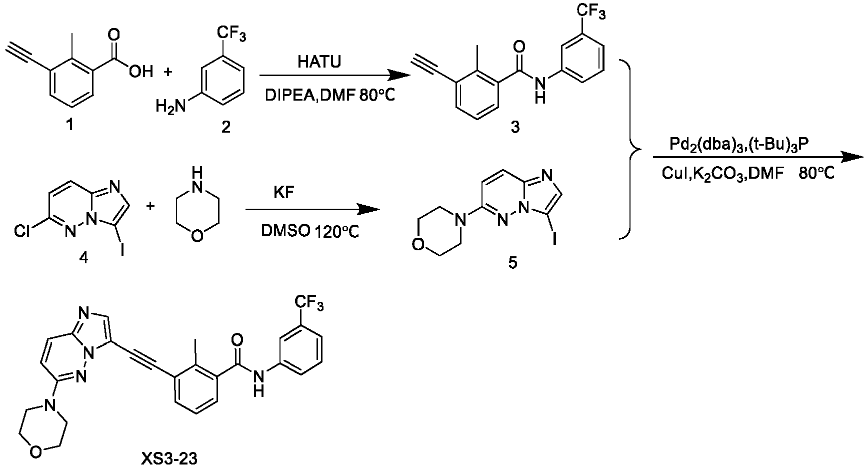

### Step 1: Preparation of 3-ethynyl-2-methyl-N-(3-(trifluoromethyl)phenyl)benzamide (Compound 3)

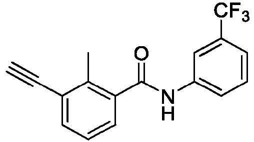

120 mg (0.74 mmol) of Compound 1 and 101 mg (0.62mmol) of Compound 2 were dissolved in 15 mL of N, N-dimethylformamide (DMF), and then added with 353 mg (0.93 mmol) of 2-(7-Azabenzotriazole)-N, N, N', N'-tetramethylurea hexafluorophosphate (HATU) and 160 mg (1.24 mmol) of N, N-diisopropylethyl acetate amine; and then the mixture was heated and stirred at 80°C for 2 hours. The reaction system was spin-dried, and added with water, and then extracted with ethyl acetate, rinsed with water, dried with anhydrous sodium sulfate. The solvent was spin-dried, and 150mg of yellow oil was obtained by column chromatography (yield: 80%).

¹H NMR (400 MHz, DMSO-*d*₆) δ 10.75 (s, 1H), 8.26 (d, J = 2.0 Hz, 1H), 7.95 - 7.90 (m, 1H), 7.63 - 7.58 (m, 2H), 7.53 (dd, J = 7.7, 1.4 Hz, 1H), 7.49 - 7.45 (m, 1H), 7.35 (t, J = 7.7 Hz, 1H), 4.52 (s, 1H) ^{,} 2.47 (s, 3H). LC-MS (ESI) m/z 304.1 [M + H]⁺ .

### Step 2: Preparation of 4-(3-iodoimidazo[1,2-b]pyridazin-6-yl)morpholine (Compound 5)

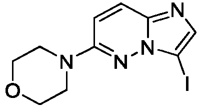

300 mg (1.07 mmol) of Compound 4 was dissolved in 15 mL of dimethyl sulfoxide (DMSO), and then added with 280 mg (3.22 mmol) of morpholine and 744 mg (12.84 mg) of potassium fluoride. The mixture was heated and stirred at 120°C for 3 hours. The reaction system was filtrated and spin-dried, and 260 mg of yellow powdery solid was obtained by column chromatography (yield: 74%).

¹H NMR (400 MHz, Chloroform-d) δ 7.70 (d, J = 9.8 Hz, 1H), 7.64 (s, 1H), 6.82 (d, J = 9.9 Hz, 1H), 3.89 (t, J = 4.8 Hz, 4H), 3.57 (t, J = 4.9 Hz, 4H). LC-MS (ESI) m/z 331.0 [M + H]⁺ .

### Step 3: Preparation of 2-Methyl-3-((6-morpholinoimidazo[1,2-b]pyridazin-3-yl)ethynyl)-N-(3-(trifluoromethyl)phenyl)benzamide (XS3-23)

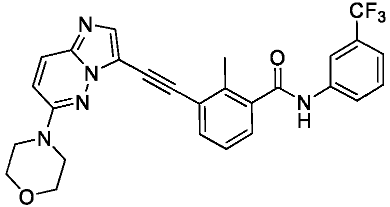

138 mg (0.45 mmol) of Compound 3 and 300 mg (0.91 mmol) of Compound 5 were dissolved in 10 mL of anhydrous N, N-dimethylformamide (DMF), and then added with 7 mg (0.036 mmol) of cuprous iodide, 21 mg (0.023 mmol) of Benzylacetone dipalladium, 9 mg (0.045 mmol) of tri-tert-butylphosphorus and 124 mg (0.9 mmol) of potassium carbonate, and then react under Ar in a closed system. The mixture was heated and stirred at 80°C and reacted overnight. The reaction solution was filtered through celite, and the solvent was spin-dried, and 120 mg of yellow-white solid was obtained by column chromatography (yield: 52%).

¹H NMR (400 MHz, Chloroform-d) δ 8.06 - 7.98 (m, 2H), 7.91 (d, J = 8.1 Hz, 1H), 7.87 - 7.74 (m, 2H), 7.64 (d, J = 7.7 Hz, 1H), 7.54 (t, J = 8.0 Hz, 1H), 7.47 (d, J = 6.5 Hz, 2H), 7.30 (d, J = 7.6 Hz, 1H), 6.90 (d, J = 9.5 Hz, 1H), 3.87 (t, J = 4.7 Hz, 4H), 3.57 (t, J = 4.8 Hz, 4H), 2.73 (s, 3H). LC-MS (ESI) m/z 504.2 [M - H]⁻.

### Example 7: Preparation of N-(3-chloro-5-(trifluoromethyl)phenyl)-2-methyl-3-((6-morpholinoimidazo[1,2-b]pyridazin-3 -yl)ethynyl)benzamide (designated as XS3-61)

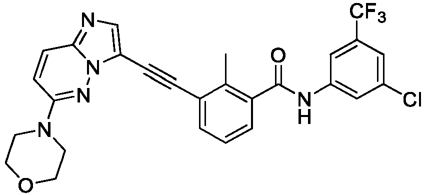

The synthetic method is according to Example 6.

¹H NMR (400 MHz, DMSO-*d*₆) δ 10.92 (s, 1H), 8.13 (d, J = 2.5 Hz, 2H), 7.98 (d, J = 10.0 Hz, 2H), 7.70 (dd, J = 7.7, 1.4 Hz, 1H), 7.61 (s, 1H), 7.56 (dd, J = 7.7, 1.4 Hz, 1H), 7.42 (t, J = 7.7 Hz, 1H), 7.32 (d, J = 9.8 Hz, 1H), 3.74 (t, J = 4.8 Hz, 4H), 3.53 (t, J = 4.8 Hz, 4H), 2.63 (s, 3H). LC-MS (ESI) m/z 539.8 [M + H]⁺ .

### Example 8: Preparation of N-(3-fluoro-5-(trifluoromethyl)phenyl)-2-methyl-3-((6-morpholinoimidazo[1,2-b]pyridazin-3-yl)ethynyl)benzamide (designated as XS4-80)

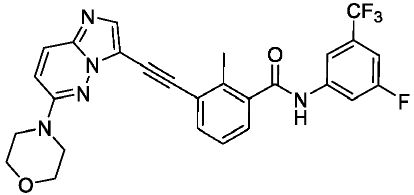

The synthetic method is according to Example 6.

¹H NMR (400 MHz, DMSO-*d*₆) δ 10.94 (s, 1H), 8.02 - 7.96 (m, 2H), 7.96 - 7.89 (m, 2H), 7.70 (dd, *J =* 7.8*,* 1.4 Hz, 1H), 7.55 (dd, *J =* 7.8, 1.4 Hz, 1H), 7.42 (dd, *J =* 8.9, 6.3 Hz, 2H), 7.31 (d, *J =* 10.0 Hz, 1H), 3.74 (t, *J =* 4.8 Hz, 4H), 3.53 (t, *J =* 4.8 Hz, 4H), 2.63 (s, 3H). HRMS (ESI) for C₂₇H₂₁F₄N₅O₂ [M + H]⁺: calcd 524.1704, found 524.1686.

### Example 9: Preparation of 2 -methyl-N-(3-methyl-5-(trifluoromethyl)phenyl)-3-((6-morpholinoimidazo[1,2-b]pyridazin-3-yl )ethynyl)benzamide (designated as XS4-81)

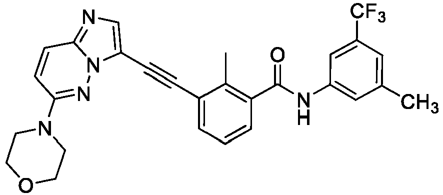

The synthetic method is according to Example 6.

¹H NMR (400 MHz, DMSO-*d*₆) δ 10.68 (s, 1H), 8.05 - 7.90 (m, 3H), 7.80 (s, 1H), 7.68 (dd, *J* = 7.7, 1.4 Hz, 1H), 7.52 (dd, *J* = 7.7, 1.4 Hz, 1H), 7.40 (t, *J* = 7.7 Hz, 1H), 7.34 - 7.28 (m, 2H), 3.74 (dd, *J* = 5.8, 3.9 Hz, 4H), 3.53 (t, *J* = 4.8 Hz, 4H), 2.62 (s, 3H), 2.40 (s, 3H). HRMS (ESI) for C₂₈H₂₄F₃N₅O₂ [M + H]⁺: calcd 520.1955, found 520.1939.

### Example 10: Preparation of N-(3-methoxy-5-(trifluoromethyl)phenyl)-2-methyl-3-((6-morpholinoimidazo[1,2-b]pyridazin-3-yl)ethynyl)benzamide (designated as XS4-72)

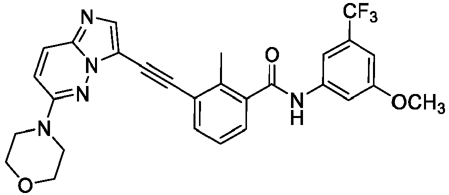

The synthetic method is according to Example 6.

¹H NMR (400 MHz, DMSO-*d*₆) δ 10.72 (s, 1H), 7.97 (d, *J=* 9.9 Hz, 1H), 7.93 (s, 1H), 7.81 (s, 1H), 7.68 (dd, *J* = 7.8, 1.4 Hz, 1H), 7.62 (t, *J* = 2.2 Hz, 1H), 7.53 (dd, *J* = 7.8, 1.4 Hz, 1H), 7.41 (t, *J* = 7.7 Hz, 1H), 7.31 (d, *J* = 9.9 Hz, 1H), 7.01 (t, *J* = 2.0 Hz, 1H), 3.84 (s, 3H), 3.74 (dd, *J =* 5.8, 3.8 Hz, 4H), 3.53 (t, *J =* 4.8 Hz, 4H), 2.62 (s, 3H). HRMS (ESI) for C₂₇H₂₁ClF₃N₅O₂ [M + H]⁺: calcd 536.1904, found 536.1919.

### Example 11: Preparation of N-(3,5-bis(trifluoromethyl)phenyl)-2-methyl-3-((6-morpholinoimidazo[1,2-b]pyridazin-3-yl)ethynyl)benzamide (designated as XS4-76)

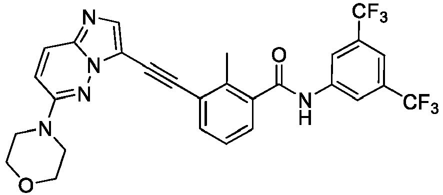

The synthetic method is according to Example 6.

¹H NMR (400 MHz, DMSO-*d*₆) δ 11.07 (s, 1H), 8.44 (s, 2H), 7.98 (d, *J =* 10.0 Hz, 2H), 7.85 (s, 1H), 7.71 (dd, *J =* 7.8, 1.4 Hz, 1H), 7.59 (dd, *J =* 7.8*,* 1.4 Hz, 1H), 7.43 (t, *J =* 7.7 Hz, 1H), 7.32 (d, *J =* 9.5 Hz, 1H), 3.74 (t, *J =* 4.8 Hz, 4H), 3.53 (t, *J* = 4.8 Hz, 4H), 2.64 (s, 3H). HRMS (ESI) for C₂₈H₂₁F₆N₅O₂ [M + H]⁺: calcd 574.1672, found 574.1676.

### Example 12: Preparation of 2-methyl-3-((6-morpholinoimidazo[1,2-b]pyridazin-3-yl)ethynyl)-N-(3-nitro-5-(trifluoromethyl)phenyl)benzamide (designated as XS4-77)

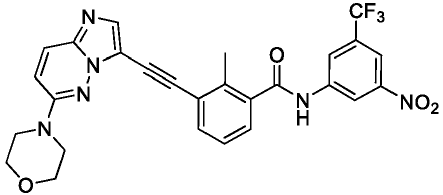

The synthetic method is according to Example 6.

¹H NMR (400 MHz, DMSO-*d*₆) δ 11.20 (s, 1H), 8.95 (t, *J* = 2.1 Hz, 1H), 8.53 (s, 1H), 8.23 (d, *J=* 2.1 Hz, 1H), 7.97 (d, *J =* 9.9 Hz, 1H), 7.93 (s, 1H), 7.72 (dd, *J* = 7.8, 1.4 Hz, 1H), 7.60 (dd, *J =* 7.7, 1.4 Hz, 1H), 7.44 (t, *J =* 7.7 Hz, 1H), 7.31 (dd, *J* = 10.1, 1.6 Hz, 1H), 3.74 (t, *J =* 4.8 Hz, 4H), 3.53 (t, *J =* 4.9 Hz, 4H), 2.65 (s, 3H). HRMS (ESI) for C₂₇H₂₁F₃N₆O₄ [M + H]⁺: calcd 551.1649, found 551.1667.

### Example 13: Preparation of 2-methyl-N-(3-((4-methylpiperazin-1-yl)methyl)-5-(trifluoromethyl)phenyl)-3-((6-morpholinoimidazo[1,2-b]pyridazin-3-yl)ethynyl)benzamide (designated as XS3-68)

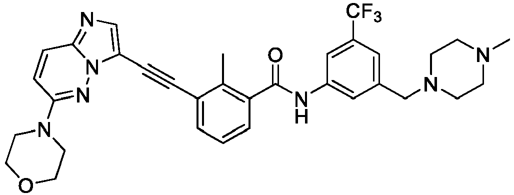

The synthetic method is according to Example 6.

¹H NMR (400 MHz, Chloroform-d) δ 8.22 (s, 1H), 7.98 (s, 1H), 7.82 (s, 1H), 7.77 (s, 1H), 7.70 (d, J = 9.8 Hz, 1H), 7.62 (dd, J = 7.7, 1.4 Hz, 1H), 7.45 - 7.40 (m, 2H), 7.26 (d, J = 7.7 Hz, 1H), 6.86 (d, J = 9.9 Hz, 1H), 3.88 - 3.84 (m, 4H), 3.59 (s, 2H), 3.57 - 3.53 (m, 4H), 2.71 (s, 3H), 2.52 (s, 8H), 2.32 (s, 3H). LC-MS (ESI) m/z 618.3 [M + H]⁺ .

### Example 14: Preparation of (S)-3-((6-(3-hydroxypyrrolidin-1-yl)imidazo[1,2-b]pyridazin-3-yl)ethynyl)-2-methyl-N-(3-(trifluoromethyl)phenyl)benzamide (designated as XS3-35)

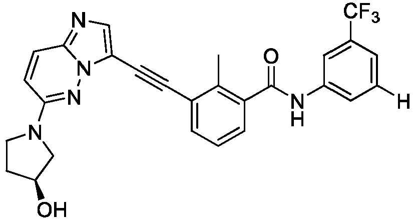

The synthetic method is according to Example 6.

¹H NMR (400 MHz, Chloroform-d) δ 8.16 (s, 1H), 8.05 (s, 1H), 7.91 (d, J = 8.1 Hz, 1H), 7.74 (s, 1H), 7.65 - 7.57 (m, 2H), 7.54 (t, J = 7.9 Hz, 1H), 7.46 (d, J = 7.8 Hz, 1H), 7.41 (d, J = 7.9 Hz, 1H), 7.24 (t, J = 7.6 Hz, 1H), 6.58 (d, J = 8.8 Hz, 1H), 5.03 (d, *J=* 3.7 Hz, 1H), 4.42 (s, 1H), 3.62 - 3.50 (m, 3H), 3.40 (d, *J=* 11.3 Hz, 1H), 2.67 (s, 3H), 2.10 - 1.98 (m, 1H), 1.92 (d, *J* = 12.9 Hz, 1H).

LC-MS (ESI) m/z 504.2 [M - H]⁻.

### Example 15: Preparation of (S)-N-(3-chloro-5-(trifluoromethyl)phenyl)-3-((6-(3-hydroxypyrrolidin-1-yl)imidazo[1,2-b]pyridazin-3-yl)ethynyl)-2-methylbenzamide (designated as XS3-58)

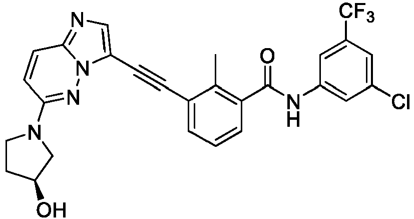

The synthetic method is according to Example 6.

¹H NMR (400 MHz, Chloroform-d) δ 8.27 (s, 1H), 8.08 (s, 1H), 7.86 (s, 1H), 7.72 (s, 1H), 7.64 - 7.59 (m, 2H), 7.44 (d, J = 1.8 Hz, 1H), 7.39 (dd, J = 7.7, 1.4 Hz, 1H), 7.25 (t, J = 7.7 Hz, 1H), 6.60 (d, J = 9.6 Hz, 1H), 4.66 (s, 1H), 3.70 - 3.54 (m, 5H), 2.71 (s, 3H), 2.16 (dd, J = 7.9, 3.9 Hz, 2H). LC-MS (ESI) m/z 540.0 [M + H]⁺ .

### Example 16: Preparation of (S)-3-((6-(3-hydroxypyrrolidin-1-yl)imidazo[1,2-b]pyridazin-3-yl)ethynyl)-2-methyl-N-(3-((4-methylpiperazin-1-yl)methyl)-5-(trifluoromethyl)phenyl)benzamide (designated as XS3-36)

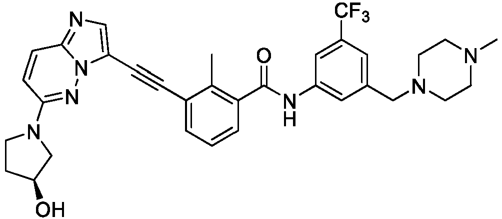

The synthetic method is according to Example 6.

¹H NMR (400 MHz, Chloroform-d) δ 8.18 (s, 1H), 8.02 (s, 1H), 7.79 (s, 1H), 7.72 (s, 1H), 7.63 - 7.58 (m, 2H), 7.41 (d, J = 5.1 Hz, 2H), 7.25 (t, J = 7.7 Hz, 1H), 6.58 (d, J = 9.7 Hz, 1H), 4.64 (dd, J = 4.3, 2.2 Hz, 1H), 3.68 - 3.56 (m, 7H), 2.74 (s, 3H), 2.51 (s, 8H), 2.31 (s, 3H), 2.17 - 2.09 (m, 2H). LC-MS (ESI) m/z 618.3 [M + H]⁺ .

### Example 17: Preparation of 3-((6-(4-(dimethylamino)piperidin-1-yl)imidazo[1,2-b]pyridazin-3-yl)ethynyl)-2-methyl-N-(3-(trifluoromethyl)phenyl)benzamide (designated as XS3-57)

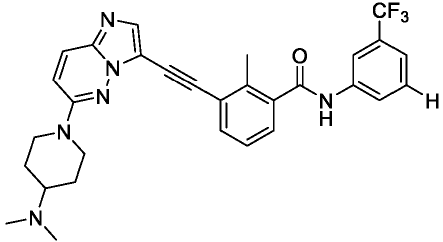

The synthetic method is according to Example 6.

¹H NMR (400 MHz, DMSO-*d*₆) δ 10.78 (s, 1H), 8.27 (d, J = 2.3 Hz, 1H), 7.95 (d, J = 2.6 Hz, 1H), 7.93 (d, J = 3.2 Hz, 1H), 7.90 (s, 1H), 7.68 (dd, J = 7.7, 1.4 Hz, 1H), 7.61 (t, J = 8.1 Hz, 1H), 7.55 (dd, J = 7.7, 1.4 Hz, 1H), 7.48 (d, J = 7.8 Hz, 1H), 7.42 (t, J = 7.7 Hz, 1H), 7.34 (d, J = 10.0 Hz, 1H), 4.30 (d, J = 13.4 Hz, 2H), 2.97 (t, J = 12.0 Hz, 2H), 2.65 (s, 3H), 2.34 (s, 6H), 1.90 (d, J = 12.1 Hz, 2H), 1.57 - 1.41 (m, 3H). LC-MS (ESI) m/z 547.2 [M + H]⁺ .

### Example 18: Preparation of N-(3-chloro-5-(trifluoromethyl)phenyl)-3-((6-(4-(dimethylamino)piperidin-1-yl)imidazo[1,2-b]pyridazin-3-yl)ethynyl)-2-methylbenzamide (designated as XS3-56)

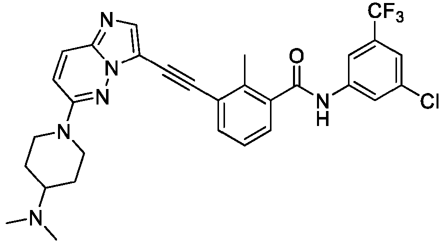

The synthetic method is according to Example 6.

¹H NMR (400 MHz, Chloroform-d) δ 8.30 (s, 1H), 8.09 (d, *J =* 2.3 Hz, 1H), 7.86 (s, 1H), 7.74 (s, 1H), 7.66 - 7.60 (m, 2H), 7.44 (s, 1H), 7.40 (dd, *J* = 7.7, 1.4 Hz, 1H), 7.28 - 7.23 (m, 1H), 6.90 (d, *J =* 9.9 Hz, 1H), 4.26 (d, *J =* 12.9 Hz, 2H), 2.98 (t, *J* = 12.8 Hz, 2H), 2.72 (s, 3H), 2.41 (t, *J =* 11.2 Hz, 1H), 2.33 (s, 6H), 2.01 - 1.93 (m, 2H), 1.66 - 1.54 (m, 2H). LC-MS (ESI) m/z 579.3 [M -H]⁻.

### Example 19: Preparation of 3-((6-(4-(dimethylamino)piperidin-1-yl)imidazo[1,2-b]pyridazin-3-yl)ethynyl)-2-methyl-N-(3-((4-methylpiperazin-1-yl)methyl)-5-(trifluoromethyl)phenyl)benzamide (designated as XS3-67)

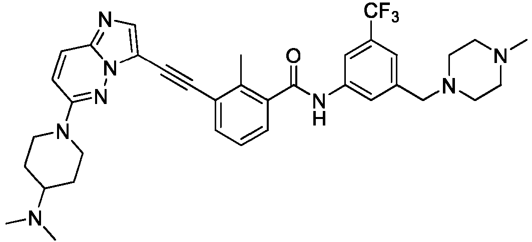

The synthetic method is according to Example 6.

¹H NMR (400 MHz, DMSO-*d*₆) δ 10.75 (s, 1H), 8.15 (s, 1H), 7.92 (d, *J =* 9.8 Hz, 2H), 7.89 (s, 1H), 7.67 (d, *J =* 6.4 Hz, 1H), 7.54 (d, *J =* 6.9 Hz, 1H), 7.40 (t, *J =* 7.7 Hz, 1H), 7.37 (s, 1H), 7.32 (d, *J =* 10.0 Hz, 1H), 4.26 (d, *J =* 13.0 Hz, 2H), 3.54 (s, 2H), 2.96 (t, *J* = 12.1 Hz, 2H), 2.64 (s, 3H), 2.45 - 2.29 (m, 8H), 2.18 (s, 6H), 2.16 (s, 3H), 2.00 (q, *J =* 7.5 Hz, 1H), 1.83 (d, *J* = 11.5 Hz, 2H), 1.44 (q, *J=* 10.5, 9.3 Hz, 2H). LC-MS (ESI) m/z 658.3 [M +H]⁺ .

### Example 20: Preparation of N-(3-chloro-5-(trifluoromethyl)phenyl)-2-methyl-3-((6-(4-methylpiperazin-1-yl)imidazo[1,2-b]pyridazin-3-yl)ethynyl)benzamide (designated as XS3-51)

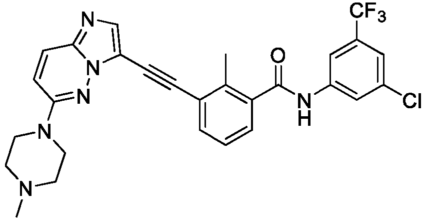

The synthetic method is according to Example 6.

¹H NMR (400 MHz, Chloroform-d) δ 8.08 (s, 2H), 7.86 (s, 1H), 7.83 (d, J = 3.4 Hz, 1H), 7.78 - 7.72 (m, 1H), 7.66 (d, J = 7.6 Hz, 1H), 7.46 (d, J = 7.7 Hz, 1H), 7.43 (s, 1H), 7.32 (d, J = 7.7 Hz, 1H), 6.89 (d, J = 9.9 Hz, 1H), 3.80 (s, 4H), 3.13 (s, 3H), 2.74 (s, 3H), 2.53 (d, J = 21.6 Hz, 4H). LC-MS (ESI) m/z 553.2 [M +H]⁺.

### Example 21: Preparation of (R)-N-(3-chloro-5-(trifluoromethyl)phenyl)-3-((6-(3,4-dimethylpiperazin-1-yl)imidazo[1,2-b]pyridazin-3-yl)ethynyl)-2-methylbenzamide (designated as XS3-52)

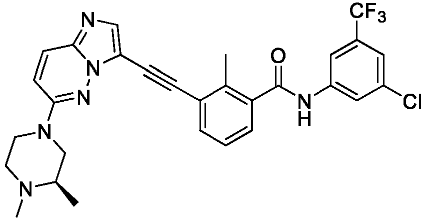

The synthetic method is according to Example 6.

¹H NMR (400 MHz, DMSO-*d*₆) δ 10.93 (s, 1H), 8.13 (s, 2H), 8.06 (s, 1H), 7.97 (s, 1H), 7.71 (d, J = 7.2 Hz, 1H), 7.62 (s, 1H), 7.58 (d, J = 7.3 Hz, 1H), 7.44 (t, J = 7.6 Hz, 1H), 7.39 (d, J = 9.5 Hz, 1H), 3.13-3.06 (m, 4H), 2.91 (s, 2H), 2.75 (m, 1H), 2.65 (s, 3H), 2.46 (s, 3H), 1.24 (s, 3H). LC-MS (ESI) m/z 567.2 [M +H]⁺ .

### Example 22: Preparation of N-(3-chloro-5-(trifluoromethyl)phenyl)-3-((6-(4-hydroxypiperidin-1-yl)imidazo[1,2-b]pyridazin-3-yl)ethynyl)-2-methylbenzamide (designated as XS3-55)

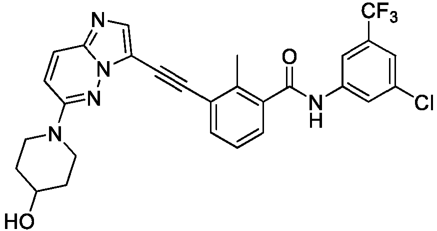

The synthetic method is according to Example 6.

¹H NMR (400 MHz, DMSO-*d*₆) δ 10.93 (s, 1H), 8.14 (d, *J* = 1.8 Hz, 2H), 7.94 - 7.88 (m, 2H), 7.69 (dd, *J = 7.7,* 1.4 Hz, 1H), 7.61 (s, 1H), 7.56 (dd, *J =* 7.7, 1.4 Hz, 1H), 7.42 (t, *J =* 7.7 Hz, 1H), 7.32 (d, *J =* 10.0 Hz, 1H), 4.71 (d, *J =* 4.2 Hz, 1H), 4.00 - 3.93 (m, 2H), 3.74 (m, 1H), 3.25 (t, *J =* 13.1 Hz, 2H), 2.64 (s, 3H), 1.82 (d, *J* = 12.8 Hz, 2H), 1.49 - 1.40 (m, 2H). LC-MS (ESI) m/z 552.1[M -H]⁻.

### Example 23: Preparation of 3-((6-(4-acetylpiperazin-1-yl)imidazo[1,2-b]pyridazin-3-yl)ethynyl)-N-(3-chloro-5-(trifluoro) methyl)phenyl)-2-methylbenzamide (designated as XS3-54)

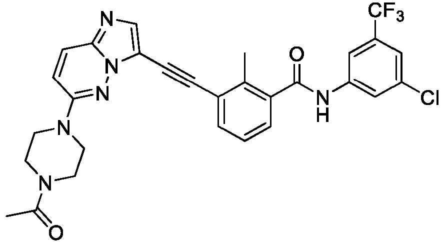

The synthetic method is according to Example 6.

¹H NMR (400 MHz, DMSO-*d*₆) δ 10.93 (s, 1H), 8.14 (d, J = 1.7 Hz, 2H), 7.98 (d, J = 9.9 Hz, 1H), 7.93 (s, 1H), 7.71 (dd, J = 7.7, 1.3 Hz, 1H), 7.61 (d, J = 2.0 Hz, 1H), 7.57 (dd, J = 7.7, 1.4 Hz, 1H), 7.43 (t, J = 7.7 Hz, 1H), 7.34 (d, J = 10.0 Hz, 1H), 3.66 - 3.53 (m, 8H), 2.65 (s, 3H), 2.04 (s, 3H). LC-MS (ESI) m/z581.5 [M +H]+ .

### Reference Example 24: Preparation of Reference Compound N-(3-chloro-5-(trifluoromethyl)phenyl)-2-methyl-3-((6-((2-morpholinoethyl)amino)imidazo[1,2-b]pyridazin-3-yl)ethynyl)benzamide (designated as XS3-53)

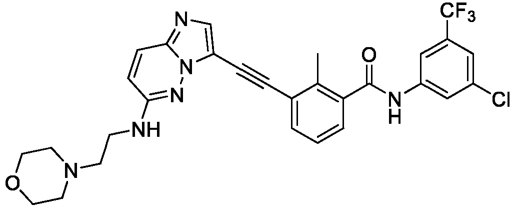

The synthetic method is according to Example 6.

¹H NMR (400 MHz, Chloroform-d) δ 8.28 (s, 1H), 8.08 (s, 1H), 7.87 (s, 1H), 7.66 (d, J = 7.7 Hz, 2H), 7.47 (d, J = 7.5 Hz, 1H), 7.44 (s, 1H), 7.34 (d, J = 7.9 Hz, 1H), 7.31 (s, 1H), 6.73 (s, 2H), 4.01 (s, 4H), 3.78 (s, 2H), 3.19 (s, 2H), 3.05 (s, 4H), 2.71 (s, 3H). LC-MS (ESI) m/z583.2 [M +H]⁺.

### Example 25: Preparation of 3-((6-(4-hydroxypiperidin-1-yl)imidazo[1,2-b]pyridazin-3-yl)ethynyl)-2-methyl-N-(3-(((4-methylpiperazin-1-yl)methyl)-5-(trifluoromethyl)phenyl)benzamide (designated as XS3-81)

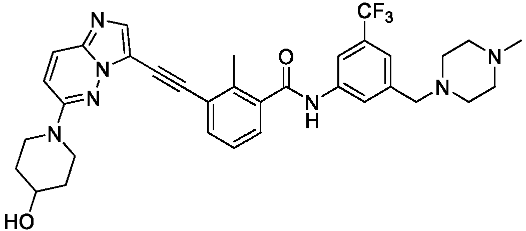

The synthetic method is according to Example 6.

¹H NMR (400 MHz, Chloroform-d) δ 8.36 (s, 1H), 8.01 (s, 1H), 7.80 (s, 1H), 7.73 (s, 1H), 7.62 (d, *J =* 9.9 Hz, 1H), 7.59 (dd, *J =* 7.8, 1.4 Hz, 1H), 7.43 - 7.38 (m, 2H), 7.24 (t, *J =* 7.7 Hz, 1H), 6.88 (d, *J =* 9.9 Hz, 1H), 4.0 - 3.91 (m, 3H), 3.59 (s, 2H), 3.28 (t, *J =* 13.1 Hz, 2H), 2.70 (s, 3H), 2.51 (s, 8H), 2.31 (s, 3H), 1.99 - 1.95 (m, 2H), 1.69 -1.61 (m, 2H).LC-MS (ESI) m/z632.3[M +H]⁺ .

### Example 26: Preparation of N-(3-chloro-5-(trifluoromethyl)phenyl)-2-methyl-3-((6-(4-morpholinopiperidin-1-yl)imidazo[1,2-b]pyridazin-3-yl)ethynyl)benzamide (designated as XS3-130)

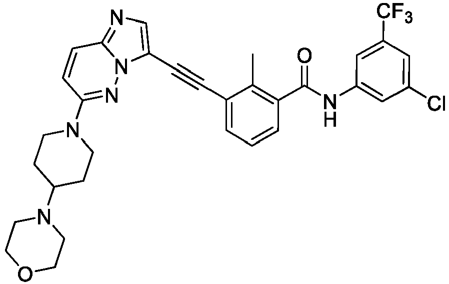

The synthetic method is according to Example 6.

¹H NMR (400 MHz, DMSO-*d*₆) δ 10.93 (s, 1H), 8.13 (s, 2H), 7.92 (d, *J=* 9.8 Hz, 2H), 7.69 (d, *J* = 7.3 Hz, 1H), 7.61 (s, 1H), 7.56 (d, *J* = 7.4 Hz, 1H), 7.42 (t, *J* = 7.7 Hz, 1H), 7.33 (d, *J* = 9.8 Hz, 1H), 4.26 (d, *J =* 13.0 Hz, 2H), 3.54 (s, 4H), 2.97 (t, *J =* 12.3 Hz, 2H), 2.64 (s, 3H), 2.45 (s, 5H), 1.86 (d, *J =* 12.8 Hz, 2H), 1.45 (d, *J =* 12.2 Hz, 2H). HRMS (ESI) for C₃₂H₃₀ClF₃N₆O₂ [M + H]⁺: calcd 623.2144, found 623.2126.

### Example 27: Preparation of N-(3-chloro-5-(trifluoromethyl)phenyl)-2-methyl-3-((6-(4-(oxetan-3-yl)piperazin-1-yl)imidazo[1,2-b]pyridazin-3-yl)ethynyl)benzamide (designated as XS3-138)

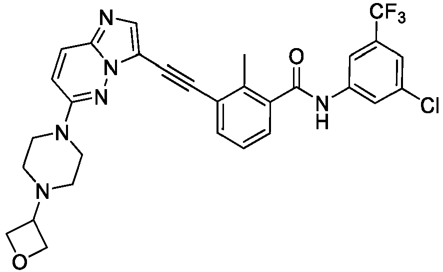

The synthetic method is according to Example 6.

¹H NMR (400 MHz, DMSO-*d*₆) δ 10.92 (s, 1H), 8.13 (s, 2H), 7.98 - 7.87 (m, 2H), 7.69 (d, *J =* 8.2 Hz, 1H), 7.61 (s, 1H), 7.56 (d, *J =* 6.9 Hz, 1H), 7.42 (t*, J =* 7.7 Hz, 1H), 7.34 (d, *J =* 9.9 Hz, 1H), 4.55 (t, *J* = 6.5 Hz, 2H), 4.47 (t, *J =* 6.1 Hz, 2H), 3.60 (t, *J* = 5.1 Hz, 4H), 3.45 (m, 1H), 2.63 (s, 3H), 2.40 (t, *J =* 5.0 Hz, 4H). HRMS (ESI) for C₃₀H₂₆ClF₃N₆O₂ [M + H]⁺: calcd 595.1831, found 595.1811.

### Reference Example 28: Preparation of Reference Compound N-(3-chloro-5-(trifluoromethyl)phenyl)-3-((6-((2-(dimethylamino)ethyl)(methyl)amino)imidazo[1,2-b]pyridazin-3-yl)ethynyl)-2-methylbenzamide (designated as XS3-134)

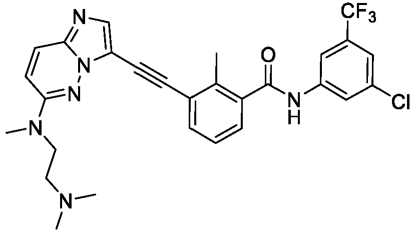

The synthetic method is according to Example 6.

¹H NMR (400 MHz, DMSO-*d*₆) δ 10.92 (s, 1H), 8.13 (d, *J =* 1.8 Hz, 2H), 7.90 - 7.82 (m, 2H), 7.68 (dd, *J = 7.7,* 1.4 Hz, 1H), 7.60 (d, *J =* 1.8 Hz, 1H), 7.55 (dd, *J* = 7.8, 1.3 Hz, 1H), 7.41 (t, *J* = 7.7 Hz, 1H), 7.14 (d, *J* = 10.0 Hz, 1H), 3.64 (t, *J* = 6.7 Hz, 2H), 3.09 (s, 3H), 2.63 (s, 3H), 2.45 (t, *J* = 6.7 Hz, 2H), 2.16 (s, 6H). HRMS (ESI) for C₂₈H₂₆ClF₃N₆O [M + H]⁺: calcd 555.1881, found 555.1862.

### Reference Example 29: Preparation of Reference Compound N-(3-chloro-5-(trifluoromethyl)phenyl)-3-((6-((2-methoxyethyl)amino)imidazo[1,2-b] Pyridazin-3-yl)ethynyl)-2-methylbenzamide (designated as XS3-135)

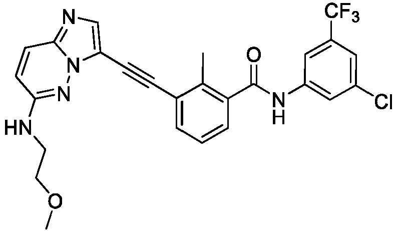

The synthetic method is according to Example 6.

¹H NMR (600 MHz, DMSO-*d*₆) δ 10.93 (s, 1H), 8.14 - 8.09 (m, 2H), 7.81 - 7.73 (m, 2H), 7.67 (dd, *J* = 7.7, 1.3 Hz, 1H), 7.59 (d, *J =* 1.9 Hz, 1H), 7.54 (dd, *J* = 7.7, 1.3 Hz, 1H), 7.41 (t, *J =* 7.7 Hz, 1H), 7.27 (t, *J =* 5.5 Hz, 1H), 6.82 (d, *J* = 9.6 Hz, 1H), 3.54 (t, *J* = 5.5 Hz, 2H), 3.47 - 3.45 (m, 2H), 3.26 (s, 3H), 2.63 (s, 3H). HRMS (ESI) for C₂₆H₂₁ClF₃N₅O₂ [M + H]⁺: calcd 528.1409, found 528.1394.

### Reference Example 30: Preparation of Reference Compound N-(3-chloro-5-(trifluoromethyl)phenyl)-3-((6-((4-hydroxybutyl)amino)imidazo[1,2-b]pyridazin-3-yl)ethynyl)-2-methylbenzamide (designated as XS3-139)

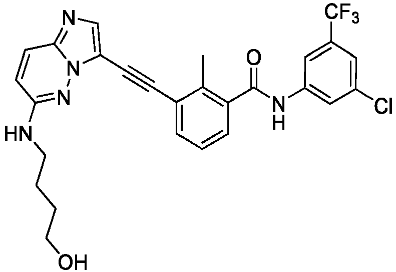

The synthetic method is according to Example 6.

¹H NMR (400 MHz, DMSO-*d*₆) δ 10.92 (s, 1H), 8.12 (s, 2H), 7.76 (d, *J =* 9.6 Hz, 2H), 7.68 (dd, *J* = 7.7, 1.4 Hz, 1H), 7.61 (s, 1H), 7.55 (dd, *J* = 7.7, 1.4 Hz, 1H), 7.41 (t, *J =* 7.7 Hz, 1H), 7.16 (t, *J =* 5.3 Hz, 1H), 6.77 (d, *J =* 8.9 Hz, 1H), 4.38 (t, *J* = 5.1 Hz, 1H), 3.43 - 3.37 (m, 2H), 3.30 - 3.25 (m, 2H), 2.65 (s, 3H), 1.70 - 1.59 (m, 2H), 1.53 - 1.46 (m, 2H). HRMS (ESI) for C₂₇H₂₃ClF₃N₅O₂ [M + H]⁺: calcd 542.1565, found 542.1552.

### Example 31: Preparation of 3-((6-(4-aminopiperidin-1-yl)imidazo[1,2-b]pyridazin-3-yl)ethynyl)-N-(3-chloro-5-(trifluoromethyl)phenyl)-2-methylbenzamide (designated as XS3-131)

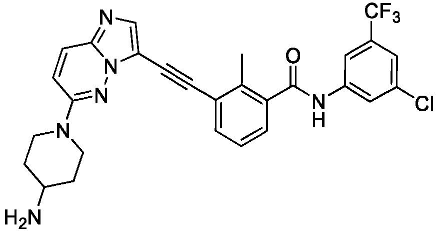

The synthetic method is according to Example 6.

¹H NMR (400 MHz, DMSO-*d*₆) δ 10.96 (s, 1H), 8.15 (s, 2H), 7.96 (d, *J =* 9.9 Hz, 1H), 7.92 (s, 1H), 7.70 (dd, *J* = 7.8, 1.3 Hz, 1H), 7.61 (d, *J* = 2.3 Hz, 1H), 7.57 (dd, *J* = 7.7, 1.3 Hz, 1H), 7.43 (t, *J* = 7.7 Hz, 1H), 7.34 (d, *J* = 10.0 Hz, 1H), 4.23 (d, *J* = 13.4 Hz, 2H), 3.17 (s, 2H), 3.12 - 3.02 (m, 2H), 2.65 (s, 3H), 2.63 (m, 1H), 1.89 (d, *J =* 11.2 Hz, 2H), 1.48 (q, *J* = 11.7, 10.8 Hz, 2H). HRMS (ESI) for C₂₈H₂₄ClF₃N₆O [M + H]⁺: calcd 553.1725, found 553.1708.

### Example 32: Preparation of (R)-3-((6-(3-aminopiperidin-1-yl)imidazo[1,2-b]pyridazin-3-yl)ethynyl)-N-(3-chloro-5-(trifluoromethyl)phenyl)-2-methylbenzamide (designated as XS3-137)

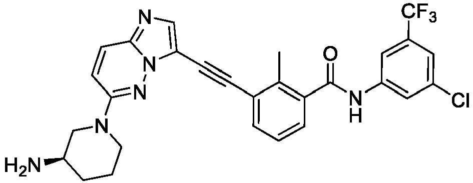

The synthetic method is according to Example 6.

¹H NMR (400 MHz, DMSO-*d*₆) δ 10.93 (s, 1H), 8.13 (s, 2H), 7.92 - 7.85 (m, 2H), 7.69 (d, *J =* 7.6 Hz, 1H), 7.60 (s, 1H), 7.55 (d, *J =* 7.6 Hz, 1H), 7.42 (t, *J =* 7.7 Hz, 1H), 7.28 (d, *J =* 10.0 Hz, 1H), 4.04 (dd, *J* = 10.6, 6.5 Hz, 2H), 2.98 (m, 1H), 2.78 - 2.69 (m, 2H), 2.65 (s, 3H), 1.86 (m, 1H), 1.74 (m, 1H), 1.61 - 1.43 (m, 2H). HRMS (ESI) for C₂₈H₂₄ClF₃N₆O [M + H]⁺: calcd 553.1725, found 553.1706.

### Example 33: Preparation of (S)-3-((6-(3-aminopiperidin-1-yl)imidazo[1,2-b]pyridazin-3-yl)ethynyl)-N-(3-chloro-5-(trifluoromethyl)phenyl)-2-methylbenzamide (designated as XS3-136)

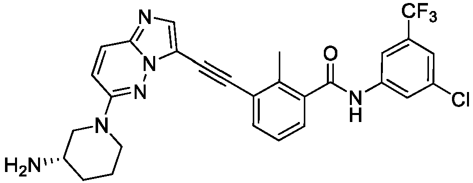

The synthetic method is according to Example 6.

¹H NMR (400 MHz, DMSO-*d*₆) δ 10.93 (s, 1H), 8.13 (s, 2H), 7.95 - 7.84 (m, 2H), 7.69 (d, *J =* 7.6 Hz, 1H), 7.60 (s, 1H), 7.55 (d, *J =* 7.6 Hz, 1H), 7.42 (t, *J = 7.7* Hz, 1H), 7.28 (d, *J =* 10.0 Hz, 1H), 4.04 (dd, *J* = 11.5, 5.9 Hz, 2H), 3.04 - 2.93 (m, 2H), 2.80 - 2.70 (m, 2H), 2.65 (s, 3H), 1.86 (d, *J* = 12.0 Hz, 1H), 1.74 (m, 1H), 1.53 (q, *J* = 11.8 Hz, 1H). HRMS (ESI) for C₂₈H₂₄ClF₃N₆O [M + H]⁺: calcd 553.1725, found 553.1703.

### Example 34: Preparation of N-(3-chloro-5-(trifluoromethyl)phenyl)-3-(6-(4-hydroxy-4-methylpiperidin-1-yl)imidazo[1,2-b]pyridazin-3-yl)ethynyl)-2-methylbenzamide (designated as XS3-153)

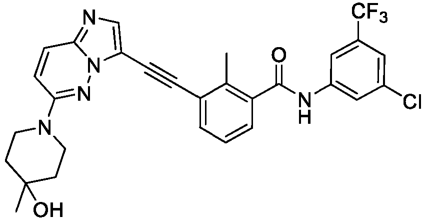

The synthetic method is according to Example 6.

¹H NMR (400 MHz, DMSO-*d*₆) δ 10.93 (s, 1H), 8.13 (t, *J =* 2.1 Hz, 2H), 7.90 (d, *J =* 10.2 Hz, 2H), 7.69 (dd, *J* = 7.7, 1.4 Hz, 1H), 7.61 (d, *J =* 1.8 Hz, 1H), 7.56 (dd, *J =* 7.7, 1.4 Hz, 1H), 7.42 (t, *J =* 7.7 Hz, 1H), 7.32 (d, *J* = 10.0 Hz, 1H), 4.39 (s, 1H), 3.92 - 3.83 (m, 2H), 3.44 - 3.37 (m, 2H), 2.64 (s, 3H), 1.55 (t, *J* = 5.6 Hz, 4H), 1.15 (s, 3H). HRMS (ESI) for C₂₉H₂₅ClF₃N₅O₂ [M + H]⁺: calcd 568.1722, found 568.1699.

### Example 35: Preparation of 2-methyl-N-(3-(4-methyl-1H-imidazol-1-yl)-5-(trifluoromethyl)phenyl)-3-((6-morpholinoimidazo[1,2-b]pyridazin-3-yl)ethynyl)benzamide (designated as XS3-91)

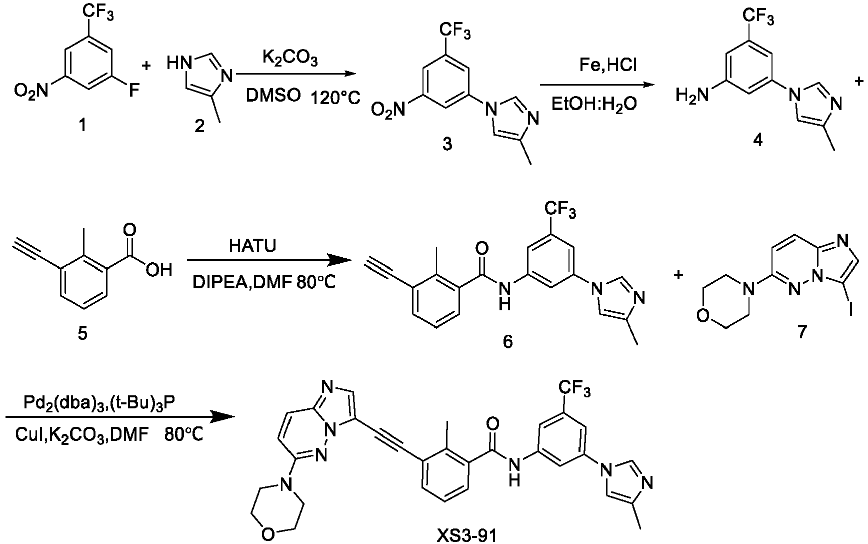

### Step 1: Preparation of 4-methyl-1-(3-nitro-5-(trifluoromethyl)phenyl)-1H-imidazole (Compound 3)

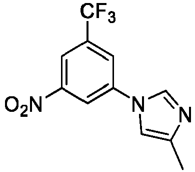

1 g (4.5 mmol) of Compound 1 and 800 mg (9.5 mmol) of Compound 2 were dissolved in 20 mL of dimethyl sulfoxide (DMSO), and then added with 850 mg (7 mmol) of potassium carbonate. The mixture was heated and stirred at 120°C, and reacted overnight. The reaction system was spin-dried, and 430 mg of yellow-white solid was obtained by column chromatography (yield: 36%).

¹H NMR (400 MHz, Chloroform-d) δ 8.46 (q, J = 1.9 Hz, 2H), 8.02 - 7.97 (m, 2H), 7.17 (s, 1H), 2.36 (d, J = 1.0 Hz, 3H). LC-MS (ESI) m/z 272.1[M +H]⁺ .

### Step 2: Preparation of 3-(4-methyl-1H-imidazol-1-yl)-5-(trifluoromethyl)aniline (Compound 4)

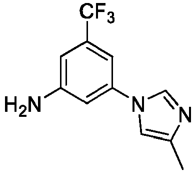

430 mg (1.59 mmol) of Compound 3 was dissolved in a mixed solvent of ethanol: water with a volume ratio of 7:3, added with hydrochloric acid solution to make the reaction system weakly acidic, and then added with 444 mg (7.93 mmol) of iron powder. The mixture was heated and stirred at 70°C for 2 hours. The reaction solution was filtered through celite, the reaction system was spin-dried, and the crude product was directly used in the next reaction.

### Step 3: Preparation of 3-ethynyl-2-methyl-N-(3-(4-methyl-1H-imidazol-1-yl)-5-(trifluoromethyl)phenyl)benzamide (Compound 6 )

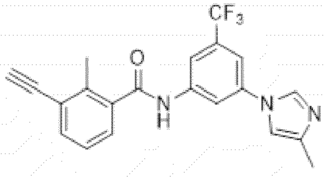

270 mg (1.12 mmol) of Compound 4 and 215 mg (1.3 mmol) of Compound 5 were dissolved in 15 mL of N,N-dimethylformamide (DMF), and then added with 638 mg (1.68 mmol) of 2-(7-azabenzone) Triazole)-N,N,N',N'-tetramethylurea hexafluorophosphate (HATU) and 216 mg (1.68 mmol) of N,N-diisopropylethylamine (DIPEA). The mixture was heated and stirred at 80°C and reacted overnight. The reaction system was spin-dried, and added with water, and then extracted with ethyl acetate, rinsed with water, dried with anhydrous sodium sulfate. The solvent was spin-dried, and 220mg of yellow oil was obtained by column chromatography (yield: 51%).

¹H NMR (400 MHz, Chloroform-d) δ 9.41 (s, 1H), 8.22 (s, 1H), 7.91 (s, 1H), 7.73 (s, 1H), 7.55 (d, J = 7.6 Hz, 1H), 7.43 (d, J = 7.2 Hz, 1H), 7.34 (s, 1H), 7.19 (t, J = 7.7 Hz, 1H), 7.09 (s, 1H), 3.34 (s, 1H), 2.58 (s, 3H), 2.25 (s, 3H). LC-MS (ESI) m/z 384.1[M +H]⁺ .

### Step 4: Preparation of 2-methyl-N-(3-(4-methyl-1H-imidazol-1-yl)-5-(trifluoromethyl) phenyl)-3-((6-morpholinoimidazo[1,2-b]pyridazin-3-yl)ethynyl)benzamide (designated as XS3-91)

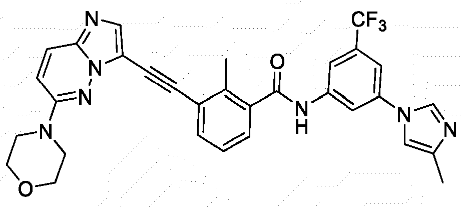

150 mg (0.39 mmol) of Compound 6 and 130 mg (0.47 mmol) of Compound 7 were dissolved in 10 mL of anhydrous N,N-dimethylformamide (DMF), and then added with 6 mg (0.03 mmol) of cuprous iodide, 17mg (0.019mmol) of tris(dibenzylideneacetone) dipalladium, 8 mg (0.039 mmol) of tri-tert-butylphosphorus, and 107 mg (0.78 mmol) of potassium carbonate, and then reacted under Ar in a closed system. The mixture was heated and stirred at 80°C and reacted overnight. The reaction solution was filtered through celite, and the solvent was spin-dried, and 40 mg of yellow-white solid was obtained by column chromatography (yield: 18%).

¹H NMR (400 MHz, DMSO-*d*₆) δ 10.92 (s, 1H), 8.20 (d, J = 1.6 Hz, 2H), 8.10 (s, 1H), 7.98 (d, J = 9.9 Hz, 1H), 7.93 (s, 1H), 7.76 (s, 1H), 7.72 - 7.69 (m, 1H), 7.57 (d, J = 7.4 Hz, 1H), 7.48 (s, 1H), 7.44 (d, J = 7.7 Hz, 1H), 7.32 (d, J = 10.0 Hz, 1H), 3.77 - 3.72 (m, 4H), 3.54 (t, J = 4.9 Hz, 4H), 2.65 (s, 3H), 2.19 (d, J = 1.0 Hz, 3H). LC-MS (ESI) m/z 586.6[M +H]⁺ .

### Example 36: Preparation of 3-((6-(4-hydroxypiperidin-1-yl)imidazo[1,2-b]pyridazin-3-yl)ethynyl)-2-methyl-N-(3-(4-methyl-1H-imidazol-1-yl)-5-(trifluoromethyl)phenyl)benzamide (designated as XS3-87)

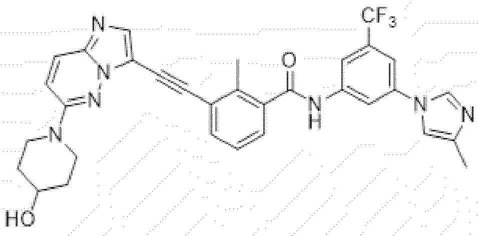

The synthetic method is according to Example 35.

¹H NMR (400 MHz, Chloroform-d) δ 8.25 (s, 1H), 7.97 (s, 1H), 7.88 (s, 1H), 7.83 (s, 1H), 7.75 - 7.70 (m, 2H), 7.68 (d, J = 7.7 Hz, 1H), 7.47 (d, J = 7.5 Hz, 1H), 7.42 (s, 1H), 7.33 (t, J = 7.7 Hz, 1H), 7.13 (s, 1H), 6.93 (d, J = 9.9 Hz, 1H), 4.05 - 3.97 (m, 3H), 3.37 - 3.29 (m, 2H), 2.78 (s, 3H), 2.33 (d, J = 1.0 Hz, 3H), 2.03 (d, J = 9.6 Hz, 2H), 1.69 (d, J = 9.5 Hz, 3H). LC-MS (ESI) m/z 600.0[M +H]⁺ .

### Example 37: IC₅₀ determination of compounds against TRKs kinases

Kinase activity detection: Applying Z'-LYTE^{™} technology (detection by fluorescence, enzyme-coupled format, based on the difference in sensitivity of phosphorylated and non-phosphorylated peptides to proteolytic cleavage), based on the principle of fluorescence resonance energy transfer (FRET), using Z'-LYTE^{™} FRET peptide substrates, and the inhibitory activity of compounds on TRKs (TRK1, TRK2, TRK3) kinases (American Life Technologies, PV3144, PV3616, PV3617) was detected through secondary reaction.

Enzymatic reaction: In a 384-well plate, added 5 µL of enzyme-substrate system [50 mM of 4-hydroxyethylpiperazineethanesulfonic acid (HEPES) pH 7.5, 0.01% BRIJ-35, 10 mM of magnesium chloride (MgCl2), 1 mM of ethylene glycol bis(2-aminoethyl ether)tetraacetic acid (EGTA), 2 µM of Tyr 01 peptide substrate], and transferred 5 nL of compound (concentration gradient) using an Echo520 ultra-micro liquid pipetting system. After shaking at room temperature for 10-20 minutes, transferred 200 nL, 12.5 nL, 25 nL ATP using the echo520 ultra-micro liquid pipetting system (final concentrations were 400 uM, 25 uM, 50 uM respectively). Shaked and mixed, then centrifuged, and reacted at 30°C in the dark for 1.5 hours.

Detection reaction: Added 2.5 µL of Development Solution (1:128 dilution) to each well and incubated at 37°C for 1 hour in the dark, then added 5 µL of Stop Reagent.

Plate reading: detected the fluorescence signal (excitation wavelength was 400 nm, emission wavelength were 460 nm, 535 nm) through a Perkin Elmer EnVision Multimode Plate Reader.

Calculation: Calculated the inhibition rate of each well through the full active well and the control signal well. The data analysis method was as follows: 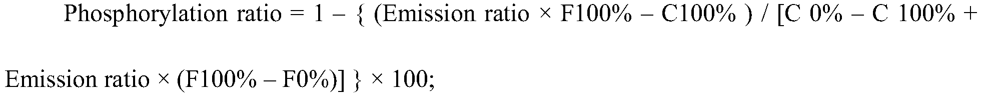 Phosphorylation ratio = 1 - { (Emission ratio × F100% - C100% ) / [C 0% - C 100% + Emission ratio × (F100% - F0%)] } × 100; 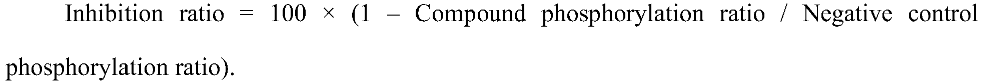 Inhibition ratio = 100 × (1 - Compound phosphorylation ratio / Negative control phosphorylation ratio).

The IC₅₀ value was calculated by medical drawing software (GraphPad Prism5.0).

The results of the kinase activity test are shown in Table 1.

**Table 1 Test results of compounds effect on kinase activity (IC₅₀: nM)**

| Compound No. | TRKA | TRKB | TRKC |
|---|---|---|---|
| XS116 (Reference Compound) | ** | ** | ** |
| XS2-161 (Reference Compound) | *** | **** | *** |
| XS2-106 (Reference Compound) | *** | **** | |
| XS2-109 (Reference Compound) | *** | **** | *** |
| XS2-112 (Reference Compound) | *** | *** | *** |
| XS3-23 | ** | ** | ** |
| XS3-61 | ** | ** | ** |
| XS4-80 | ** | | ** |
| XS4-81 | ** | | ** |
| XS4-72 | ** | ** | ** |
| XS4-76 | ** | ** | ** |
| XS4-77 | ** | ** | ** |
| XS3-68 | * | * | * |
| XS3-35 | * | ** | * |
| XS3-58 | ** | ** | ** |
| XS3-36 | ** | ** | * |
| XS3-57 | * | * | * |
| XS3-56 | ** | ** | ** |
| XS3-67 | * | ** | * |
| XS3-51 | ** | ** | * |
| XS3-52 | ** | ** | ** |
| XS3-55 | * | ** | ** |
| XS3-54 | * | ** | * |
| XS3-53 (Reference Compound) | * | ** | * |
| XS3-81 | * | * | * |
| XS3-130 | ** | ** | * |
| XS3-138 | ** | * | * |
| XS3-134 (Reference Compound) | ** | ** | ** |
| XS3-135 (Reference Compound) | ** | ** | ** |
| XS3-139 (Reference Compound) | ** | ** | ** |
| XS3-131 | ** | ** | ** |
| XS3-137 | ** | ** | ** |
| XS3-136 | ** | ** | ** |
| XS3-153 | ** | ** | ** |
| XS3-91 | * | * | * |
| XS3-87 | * | * | * |

| | | | |
|---|---|---|---|
| IC₅₀: <10 nM=*; 10-100 nM=**; 100-1000 nM=***; >1 uM=****. | | | |

It can be seen from the data in Table 1 that the Alkynylphenylbenzamide compounds of the present disclosure have strong inhibitory activity on TRKs kinase.

### Example 38: Study on Cell proliferation inhibitory activity based on Ba/F3-TRKs stable strain

The BaF3 cells (mouse pre-B cells) used in this experiment were purchased from the Japanese Cell Bank. All of the monoclonal stable strains, BaF3-CD74-NTRK1, BaF3-ETV6-NTRK2 and BaF3-ETV6-NTRK3 were constructed by our laboratory, and were identified by experiments such as positive control, protein expression and gene sequencing.

The brief steps of stable strain construction were as follows: constructed pCDNA3.1(+) plasmid vector carrying genes such as CD74-NTRK1, ETV6-NTRK2, ETV6-NTRK3; the plasmid was electroporated into Ba/F3 cells using Amaxa^{®} Cell Line Nucleofector^{®} Kit V; 48 hours after electroporation, geneticin (G418) with a final concentration of 1000 µg/mL was added to screen for two weeks, and the interleukin 3 (IL3) was removed to continue screening to obtain polyclonal stable strains; and then single clones were selected by limiting dilution method; the stable strains were then identified by the positive drugs, Western Blot (WB), and gene sequencing; the correct monoclonal through identification can be used to study the cell proliferation inhibitory activity of the inhibitors.

Cell proliferation inhibitory activity study: The cells in logarithmic growth phase were seeded into 96-well plates at 8000-12000 cells/well, and the inhibitors at different concentrations of (0-10 µM) were added the next day, and the culture was continued for 72 hours; then 10 µL of Cell Counting Kit-8 Cell Counting Reagent (CCK-8 Reagent) was added to each well, and continued to incubate for 1-3 hours; then its absorbance at 450nm and 650nm were measured with a super microplate reader. The median inhibitory concentration (IC₅₀) was calculated using medical graphing software (GraphPad Prism 8.0.0).

The test results are shown in Table 2.

**Table 2 Test results of compounds effect on cell viability (IC₅₀: nM)**

| Compound No. | CD74-NTRK1 | ETV6-NTRK2 | ETV6-NTRK3 |
|---|---|---|---|
| XS116 (Reference Compound) | *** | *** | *** |
| XS2-161 (Reference Compound) | **** | **** | **** |
| XS2-106 (Reference Compound) | *** | *** | |
| XS2-112 (Reference Compound) | *** | *** | *** |
| XS3-23 | ** | ** | ** |
| XS3-61 | ** | | ** |
| XS4-80 | ** | ** | ** |
| XS4-81 | ** | ** | * |
| XS4-72 | ** | ** | * |
| XS4-76 | ** | ** | ** |
| XS4-77 | ** | ** | * |
| XS3-68 | * | ** | * |
| XS3-35 | ** | *** | ** |
| XS3-58 | *** | *** | ** |
| XS3-36 | ** | ** | ** |
| XS3-57 | ** | *** | ** |
| XS3-56 | *** | *** | ** |
| XS3-67 | *** | *** | ** |
| XS3-51 | *** | *** | ** |
| XS3-52 | ** | *** | ** |
| XS3-55 | ** | ** | ** |
| XS3-54 | ** | ** | ** |
| XS3-53 (Reference Compound) | ** | *** | ** |
| XS3-81 | ** | * | * |
| XS3-130 | ** | ** | ** |
| XS3-138 | ** | ** | * |
| XS3-134 (Reference Compound) | *** | *** | ** |
| XS3-135 (Reference Compound) | *** | *** | ** |
| XS3-139 (Reference Compound) | ** | ** | ** |
| XS3-131 | *** | *** | ** |
| XS3-137 | *** | *** | ** |
| XS3-136 | *** | *** | ** |
| XS3-153 | ** | ** | ** |
| XS3-87 | * | ** | * |
| XS3-91 | ** | ** | * |

| | | | |
|---|---|---|---|
| IC₅₀: <10 nM= *; 10-100 nM= **; 100-1000 nM= ***; >1 uM= ****. | | | |

It can be seen from the data in Table 2 that the Alkynylphenylbenzamide compounds of the present disclosure have strong inhibitory activity on the cell proliferation of the Ba/F3-TRKs stable strains.

### Example 39: Study on the inhibitory activity of drug-resistant cell proliferation based on Ba/F3-TRKs stable strain

BaF3 cells (mouse pre-B cells) used in this experiment were purchased from Japan Cell Bank, all of the monoclonal stable strains, BaF3-CD74-NTRK1-G667C, BaF3-CD74-NTRK1-F589L, BaF3-CD74-NTRK1-G595R, BaF3-CD74-NTRK1-G667A, BaF3-CD74-NTRK1-V573M, BaF3-ETV6-NTRK2-G639R, BaF3-ETV6-NTRK2-G709C, BaF3-ETV6-NTRK2-V617M, BaF3-ETV6-NTRK2-F633L, BaF3-ETV6-NTRK3-G696C, BaF3-ETV6-NTRK3-G696A, BaF3-ETV6-NTRK3-G623R, BaF3-ETV6-NTRK3-G623E, BaF3-ETV6-NTRK3-F617L and BaF3-ETV6-NTRK3-V601M were constructed by our laboratory, and were identified by experiments such as positive control, protein expression and gene sequencing.

The brief steps for stable strains construction were as follows: constructed pCDNA3.1 (+) plasmid vector carrying genes such as BaF3-CD74-NTRK1-G667C, BaF3-CD74-NTRK1-F589L, BaF3-CD74-NTRK1-G595R, BaF3-CD74-NTRK1-G667A, BaF3-CD74-NTRK1-V573M, BaF3-ETV6-NTRK2-G639R, BaF3-ETV6-NTRK2-G709C, BaF3-ETV6-NTRK2-V617M, BaF3-ETV6-NTRK2-F633L, BaF3-ETV6-NTRK3-G696C, BaF3-ETV6-NTRK3-G696A, BaF3-ETV6-NTRK3-G623R, BaF3-ETV6-NTRK3-G623E, BaF3-ETV6-NTRK3-F617L and BaF3-ETV6-NTRK3-V601M; the plasmid was electroporated into Ba/F3 cells using Amaxa^{®} Cell Line Nucleofector^{®} Kit V; 48 hours after of electroporation, geneticin (G418) with a final concentration of 1000 µg/mL was added to screen for two weeks, and the interleukin 3 (IL3) was removed to continue screening to obtain polyclonal stable strains; and then single clones were selected by limiting dilution method; the stable strains were then identified by the positive drugs, Western Blot (WB), and gene sequencing; the correct monoclonal through identification can be used to study the cell proliferation inhibitory activity of the inhibitors.

Cell proliferation inhibitory activity study: The cells in logarithmic growth phase were seeded into 96-well plates at 8000-12000 cells/well, and the inhibitors at different concentrations of (0-10 µM) were added the next day, and the culture was continued for 72 hours; then 10 µL of Cell Counting Kit-8 Cell Counting Reagent (CCK-8 Reagent) was added to each well, and continued to incubate for 1-3 hours; then its absorbance at 450nm and 650nm were measured with a super microplate reader. The median inhibitory concentration (IC₅₀) was calculated using medical graphing software (GraphPad Prism 8.0.0).

The test results are shown in Table 3.

**Table 3 Test results of compounds effect on drug-resistant cell viability (IC₅₀: nM)**

| Compound No. | XS3-55 | XS3-68 | XS3-81 | XS3-87 |
|---|---|---|---|---|
| BaF3-CD74-NTRK1-G667C | * | * | * | * |
| BaF3-CD74-NTRK1-G667A | * | * | * | * |
| BaF3-CD74-NTRK1-G595R | **** | *** | *** | *** |
| BaF3-CD74-NTRK1-F589L | *** | *** | *** | ** |
| BaF3-CD74-NTRK1-V573M | ** | ** | * | * |
| BaF3-ETV6-NTRK2-G639R | *** | ** | ** | ** |
| BaF3-ETV6-NTRK2-F633L | *** | *** | *** | ** |
| BaF3-ETV6-NTRK2-V617M | * | * | * | * |
| BaF3-ETV6-NTRK2-G709C | * | * | ** | * |
| BaF3-ETV6-NTRK3-G696C | * | * | * | * |
| BaF3-ETV6-NTRK3-G696A | * | * | * | * |
| BaF3-ETV6-NTRK3-G623R | *** | ** | ** | ** |
| BaF3-ETV6-NTRK3-G623E | * | * | * | * |
| BaF3-ETV6-NTRK3-F617L | ** | ** | ** | * |
| BaF3-ETV6-NTRK3-V601M | * | * | * | * |

| | | | | |
|---|---|---|---|---|
| IC₅₀: <10 nM= *; 10-100 nM= **; 100-1000 nM= ***; >1 uM= ****. | | | | |

It can be seen from the data in Table 3 that the Alkynylphenylbenzamide compounds of the present disclosure have strong inhibitory activity on the proliferation of drug-resistant cells of the Ba/F3-TRKs stable strains.

### Example 40: IC₅₀ determination of Compound XS3-55 kinase selectivity

Kinase activity detection: Applying Z'-LYTE^{™} technology (detection by fluorescence, enzyme-coupled format, based on the difference in sensitivity of phosphorylated and non-phosphorylated peptides to proteolytic cleavage), based on the principle of fluorescence resonance energy transfer (FRET), using Z'-LYTE^{™} FRET peptide substrates, and the inhibitory activity of Compound XS3-55 and control molecules XS4-128 and Ponatinib against Bcr-Abl, SRC, RET, PDGFRA, PDGFRB, VEGFR2 and KIT kinases was detected through secondary reaction.

Wherein, the structural formula of Compound XS4-128 is as follows:

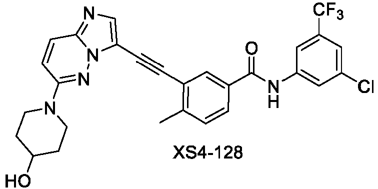

Its preparation method is as follows:

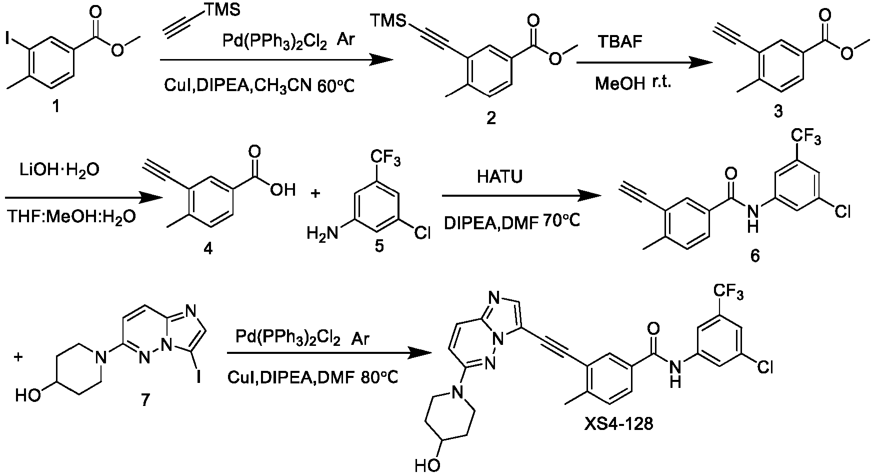

### Step 1: Preparation of methyl 4-methyl-3-((trimethylsilyl)ethynyl)benzoate (Compound 2)

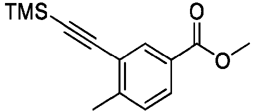

In a 100mL three-necked flask, 1 g (3.6 mmol) of Compound 1, 69 mg (0.36 mmol) of cuprous iodide, 127mg (0.18 mmol) of bis(triphenylphosphine) palladium dichloride, 50mL of anhydrous acetonitrile and 934 mg (7.2 mmol) of N, N - diisopropylethylamine were added to react under Ar in a closed system. Then 1.06 g (10.8 mmol) of trimethylsilylacetylene was injected with a syringe, and the mixture was stirred at 60° C for 6 hours. The reaction solution was filtered through celite, and the solvent was spin-dried to obtain a black mixture, which was directly used in the next reaction.

### Step 2: Preparation of methyl 3-ethynyl-4-methylbenzoate (Compound 3)

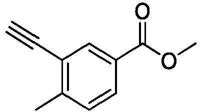

The crude product of the previous step was dissolved in methanol, and added with about 2 mL of 1 mol/L tetrabutylammonium fluoride solution in tetrahydrofuran, and the mixture was stirred at room temperature for 2 hours. The reaction system was spin-dried, and 470 mg of yellow-brown oil was obtained by column chromatography (total yield of two steps was 75%).

¹H NMR (400 MHz, DMSO-*d*₆) δ 8.06 (d, *J =* 1.5 Hz, 1H), 7.76 (dd, *J =* 7.5, 1.5 Hz, 1H), 7.25 (dd, *J =* 7.5, 1.0 Hz, 1H), 4.25 (s, 1H), 3.86 (s, 3H), 2.39 (s, 3H). LC-MS (ESI) m/z 175.5[M + H]⁺.

### Step 3: Preparation of 3-ethynyl-4-methylbenzoic acid (Compound 4)

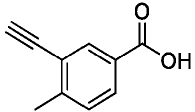

400 mg (2.29 mmol) of Compound 3 was dissolved in a mixed solvent of tetrahydrofuran, methanol and water with a volume ratio of 10:1:5, then 482 mg (11.4 mmol) of lithium hydroxide hydrate was added, and the mixture was stirred at 60°C for 1 hour. The reaction system was filtered and spin-dried, and then added with 4M hydrochloric acid solution until the system became acidic. At this time a white solid is precipitated, which was collected by filtration, and dried to obtain 350 mg of white solid (yield: 96%).

¹H NMR (400 MHz, DMSO-*d*₆) δ 13.02 (s, 1H), 7.93 (d, *J =* 1.8 Hz, 1H), 7.85 (dd, *J =* 7.9, 1.9 Hz, 1H), 7.43 (d, *J =* 8.0 Hz, 1H), 4.48 (s, 1H), 2.45 (s, 3H). LC-MS (ESI) m/z 160.9 [M + H]⁺.

### Step 4: Preparation of N-(3-chloro-5-(trifluoromethyl)phenyl)-3-ethynyl-4-methylbenzamide (Compound 6)

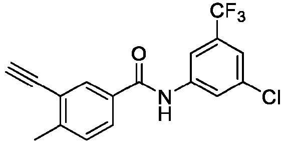

300 mg (1.9 mmol) of Compound 4 and 305 mg (1.6 mmol) of Compound 5 were dissolved in 20 mL of N,N-dimethylformamide (DMF), added with 912 mg (2.4 mmol) of 2-(7-Azabenzotriazole)-N,N,N',N'-tetramethylurea hexafluorophosphate (HATU) and 413 mg (3.2 mmol) of N,N-diisopropylethyl acetate amine, and the mixture was heated and stirred at 70°C for 2 hours. The reaction system was spin-dried, and added with water, extracted with ethyl acetate, rinsed with water, dried over anhydrous sodium sulfate. The solvent was spin-dried, and 340 mg of yellow oil was obtained by column chromatography (yield: 63%).

¹H NMR (400 MHz, DMSO-*d*₆) δ 10.88 (s, 1H), 8.11 (d, *J* = 1.8 Hz, 2H), 7.64 - 7.57 (m, 2H), 7.54 (dd, *J* = 7.7, 1.4 Hz, 1H), 7.36 (t, *J* = 7.7 Hz, 1H), 4.51 (s, 1H), 2.47 (s, 3H). MS (ESI) m/z 335.8 [M - H]⁻.

### Step 5: Preparation of N-(3-chloro-5-(trifluoromethyl)phenyl)-3-((6-(4-hydroxypiperidin-1-yl)imidazo[1,2-b]pyridazin-3-yl)ethynyl)-4-methylbenzamide (XS4-128)

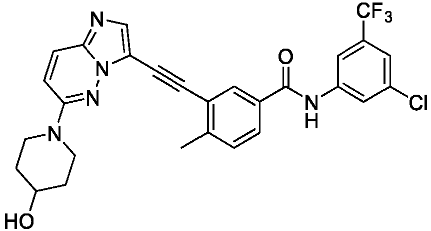

70 mg (0.2 mmol) of Compound 6 and 83 mg (1.2 mmol) of Compound 7 were dissolved in 10 mL of anhydrous N,N-dimethylformamide (DMF), and then added with 8 mg (0.016 mmol) of cuprous iodide, 19 mg (0.01 mmol) of bis(triphenylphosphine)palladium dichloride and 145 mg (0.4 mmol) of N,N-diisopropylethylamine, and reacted under Ar in a closed system. The mixture was heated and stirred at 80°C and reacted overnight. The reaction solution was filtered through celite, the solvent was spin-dried, and 80 mg of yellow-white solid was obtained by column chromatography (yield: 73%).

¹H NMR (400 MHz, DMSO-*d*₆) δ 10.66 (s, 1H), 8.25 (s, 2H), 8.16 (d, *J* = 9.2 Hz, 2H), 7.96 (s, 1H), 7.90 (dd, *J* = 8.0, 1.9 Hz, 1H), 7.55 (s, 1H), 7.52 (d, *J* = 8.1 Hz, 1H), 7.34 (s, 1H), 4.75 (d, *J=* 4.2 Hz, 1H), 3.98 (d, *J=* 14.5 Hz, 2H), 3.80 - 3.71(m , 1H), 3.26 (t, *J=* 11.5 Hz, 2H), 2.61 (s, 3H), 1.89 - 1.78(m, 2H), 1.54 - 1.40 (m, 2H). HRMS (ESI) for C₂₈H₂₃ClF₃N₅O₂ [M + H]⁺: calcd 554.1565, found 554.1562.

Enzymatic reaction: In a 384-well plate, added 5 µL of enzyme-substrate system [50 mM 4-hydroxyethylpiperazineethanesulfonic acid (HEPES) pH 7.5, 0.01% BRIJ-35, 10 mM of magnesium chloride (MgCl2), 1 mM of ethylene glycol bis(2-aminoethyl ether)tetraacetic acid (EGTA), 2 µM of Tyr 01 peptide substrate], transferred 5 nL of compound (concentration gradient) using an Echo520 Ultra-Micro Liquid Pipetting System. After shaking at room temperature for 10-20 minutes, transferred 200 nL, 12.5 nL, 25 nL ATP using the Echo520 Ultra-Micro Liquid Pipetting System (final concentrations are 400 uM, 25 uM, 50 uM respectively). Shaked and mixed, then centrifuged, and reacted at 30°C in the dark for 1.5 hours.

Detection reaction: Added 2.5 µL of Development Solution (1:128 dilution) to each well and incubated at 37°C for 1 hour in the dark, then added 5 µL of Stop Reagent.

Plate reading: detected the fluorescence signal (excitation wavelength was 400 nm, emission wavelength was 460 nm, 535 nm) through a Perkin Elmer EnVision Multimode Plate Reader.

Calculation: Calculated the inhibition rate of each well through the full active well and the control signal well. The data analysis method was as follows: 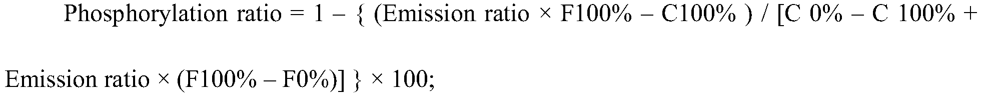 Phosphorylation ratio = 1 - { (Emission ratio × F100% - C100% ) / [C 0% - C 100% + Emission ratio × (F100% - F0%)] } × 100; 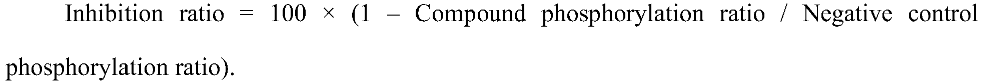 Inhibition ratio = 100 × (1 - Compound phosphorylation ratio / Negative control phosphorylation ratio).

The IC₅₀ value was calculated by medical drawing software (GraphPad Prism5.0).

The results of the kinase activity test are shown in Table 4.

**Table 4 Test Results of compounds kinase selective activity detection IC₅₀: nM)**

| Kinase | XS3-55 | XS4-128 | Ponatinib |
|---|---|---|---|
| Bcr-Abl | >10000 | 128.8 | 3.9 |
| SRC | 526.3 | 20.3 | 2.0 |
| RET | >10000 | 10.7 | 3.3 |
| VEGFR2 | 832.7 | 18.9 | 3.3 |
| Kit | 1669 | 93.5 | 25.1 |
| PDGFRA | 5887 | 19.9 | 3.9 |
| PDGFRB | >10000 | 118.9 | 18.0 |

It can be seen from the data in Table 4 that the representative compound XS3-55 of the Alkynylphenylbenzamide compounds of the present disclosure has weak inhibitory activity on a variety of representative tyrosine kinases other than TRKA, TRKB and TRKC. It has good kinase selectivity, and its kinase selectivity is much better than that of compounds XS4-128 and Ponatinib. Therefore, the Alkynylphenylbenzamide compounds of the present disclosure have good selectivity and low toxic and side effects.

### Example 41: Pharmacokinetic Evaluation

Pharmacokinetic and oral bioavailability tests were performed in SD rats. According to the drug's solubility, the drug was administered orally and intravenously as a single dose. Animal blood samples were collected at different time points (0, 0.5, 1, 2, 4, 6, 8, 24 hours), and added with heparin for anticoagulation, then centrifugated to obtain the supernatant. Blood samples were analyzed by HPLC-MS, and DAS2.1 was used for data analysis to detect half-life (T1/2), maximum blood concentration (Cmax), peak time (Tmax), area under the curve (AUC), and bioavailability (BA) and other pharmacokinetic data. The pharmacokinetic data results of compound XS3-55 and compound 9o (European Journal of Medicinal Chemistry 179(2019) 470-482.) are shown in Table 5:

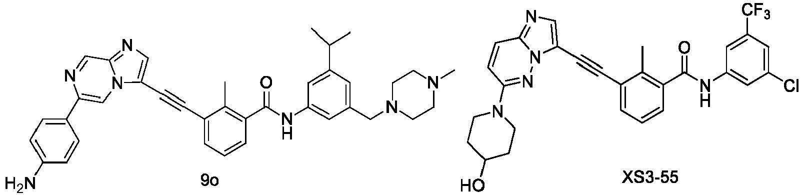

**Table 5**

| Compound | Compound 9o | | Compound XS3-55 | |
|---|---|---|---|---|
| Administration way | Intravenous injection | Oral | Intravenous injection | Oral |
| Dose (mg/kg) | 5 | 25 | 2.0 | 10.0 |
| half-life (h) | 2.03 | 4.77 | 16.07 | 15.19 |
| Tmax (h) | 0.083 | 2 | 0.08 | 4.00 |
| Cmax (ng/mL) | 1968.37 | 63.3 | 46123.74 | 44066.54 |
| AUC (0-t)(h*ng/mL) | 804.65 | 431.49 | 358128.95 | 560764.28 |
| AUC (0-∞) (h*ng/mL) | 810.45 | 445.18 | 548798.57 | 878346.33 |
| Clearance rate (mL/h/kg) | 1260.51 | Nd | 3.65 | nd |
| BA (%) | | 10.72 | | 31.32 |

Compound XS3-55 had strong inhibitory activity on TRKs kinase, and had strong inhibitory activity on the proliferation of wild-type and drug-resistant cells of Ba/F3-TRKs stable strain as well. It also had good oral absorption properties. Under the oral dose of 10 mg/kg in rats, the half-life of compound XS3-55 was 15.19 hours, the Cmax was as high as 44066.54 ng/mL, and the AUC was as high as 878346.33 h*ng/mL, the pharmacokinetic properties were significantly higher than that of the control compound 9o.

### Example 42: In vivo antitumor activity of compound XS3-55

The in vivo antitumor efficacy of compound XS3-55 was evaluated in a Ba/F3-CD74-TRKAG^{667C} allograft mouse model by oral administration. The cultured BaF3-CD74-TRKAG^{667C} cells were collected and centrifuged. After washed twice with normal saline, the cells were adjusted to a density of 1 × 10⁷ cells/mL and placed on ice, and injected subcutaneously into the right axilla of CB17-SCID female mice (purchased from Viton Lever, Beijing, 6-8 weeks old) as soon as possible, with 200 µL each injection. After 9 days of modeling, when the tumor volume grew to about 200 mm³, the mice were randomly divided into groups and started to be administered, including a control group with 8 mice and 4 groups of compound XS3-55 doses (50, 25, 12.5, 6.25 mg/kg) with 6 mice in each group.

The administration method was as follows: according to the dosage, an appropriate amount of Compound XS3-55 powder was weighed and dissolved in a mixed solvent of 2% dimethyl sulfoxide (DMSO) + 20% hydrogenated castor oil + 8% absolute ethanol + 70% normal saline, to obtain light yellow to yellow transparent liquid, orally administered once a day. The control group was given the same volume of mixed solvent orally. Body weight and tumor volume were recorded every two days.

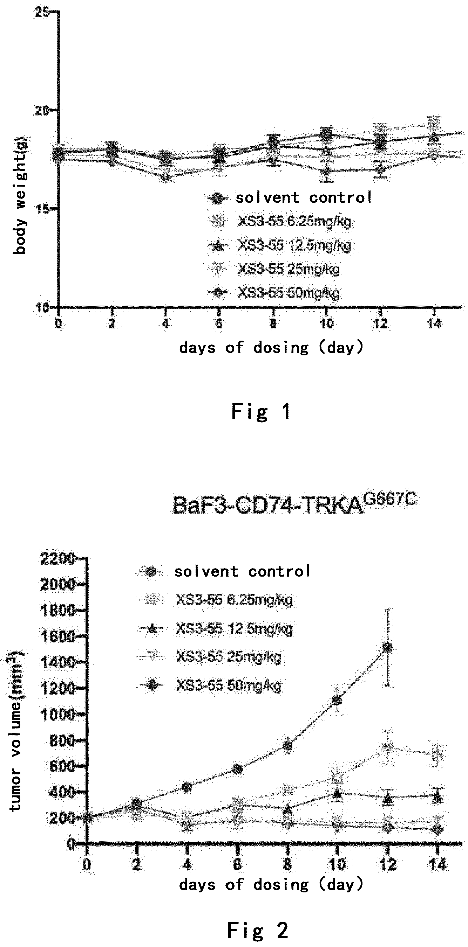

The results are shown in Fig. 1 and Fig. 2: Compound XS3-55 was administered once daily for two weeks, inhibited the growth of the CD74 TRKAG^{667C} mutation-bearing allograft mouse model in a dose-dependent manner. Significant reductions in tumor size were observed after 2 days of treatment at the lowest dose of 6.25 mg/kg. After treatment for 12 days, Compound XS3-55 exhibited excellent in vivo antitumor efficacy at doses of 6.25 mg/kg/day, 12.5 mg/kg/day, 25 mg/kg/day and 50 mg/kg/day, with TGIs being 50.9%, 76.3%, 89.2 and 91.6%, respectively; while the control mice died on day 14. Meanwhile, the in vivo studies showed that four different doses of compound XS3-55 had no obvious adverse effect on mouse body weight (Fig.1), which indicated that Compound XS3-55 had a good safety profile.

## Claims

1. Alkynylphenylbenzamide compounds with the structure shown in Formula (I) or their pharmaceutically acceptable salts, or stereoisomers, 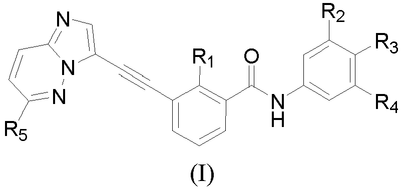
wherein, R₁ is selected from: C₁-C₂₀ alkyl;
R₂ is selected from: H, halogen, C₁-C₂₀ alkyl, C₁-C₂₀ alkoxy and halogen-substituted C₁-C₂₀ alkyl;
R₃ is selected from: H, difluoromethyl, difluoroethyl, trifluoromethyl and trifluoroethyl;
R₄ is selected from: H, halogen, nitro, C₁-C₈ alkyl, halogen-substituted C₁-C₈ alkyl, C₁-C₈ alkoxy, halogen-substituted C₁-C₈ alkoxy, -(CH₂)ₓNR₁₇R₁₈, 5-6 membered heterocyclyl group containing 1-3 N ring atoms and substituted or unsubstituted by 1-5 R₁₉, and 5-6-membered heteroaryl containing 1-3 N ring atoms and substituted or unsubstituted by 1-5 R₁₉, wherein, x is an integer from 1 to 5; R₁₇ and R₁₈ together with the attached nitrogen atom form a 1-5 R₁₉ substituted or unsubstituted morpholinyl, pyrrolidinyl, piperidinyl or piperazinyl; each R₁₉ is independently selected from: C₁-C₅ alkyl;
R₅ is -NR₆R₇;
wherein, R₆ and R₇ are independently selected from: -(CH₂)ₘNR₈R₉, -(CH₂)ₙCR₁₀R₁₁R₁₂, and -(CH₂)ₚOR₁₂; or R₆ and R₇ together with the attached nitrogen atom form a 3-15 membered monocyclic ring, fused ring, spiro ring or bridged ring containing 1-3 heteroatoms and substituted or unsubstituted by 1-5 R₁₃; wherein, the heteroatoms are selected from: O, N, S;
R₈ and R₉ are independently selected from: H, and C₁-C₅ alkyl; or R₈ and R₉ together with the attached nitrogen atom form a 3-10 membered monocyclic ring, fused ring, spiro ring or bridged ring containing 1-3 heteroatoms and substituted or unsubstituted by 1-5 R₁₃, wherein, the heteroatoms are selected from: O, and N;
R₁₀ and R₁₁ together with the attached carbon atoms form a 3-10 membered monocyclic ring, fused ring, spiro ring or bridged ring containing 1-3 heteroatoms and substituted or unsubstituted by 1-5 R₁₃, wherein, the heteroatoms are selected from: O, and N;
R₁₂ is selected from: H, and C₁-C₅ alkyl;
each R₁₃ is independently selected from: H, C₁-C₅ alkyl, C₁-C₅ alkanoyl, hydroxy, hydroxy-substituted C₁-C₅ alkyl, amino-substituted C₁-C₅ alkyl, amino-substituted C₁-C₅ alkoxy, C₃-C₇ cycloalkyl-substituted C₁-C₃ alkyl, -NR₁₅R₁₆, and 3-10 membered monocyclic ring, fused ring, spiro ring or bridged ring containing 1-3 heteroatoms and substituted or unsubstituted by 1-5 R₁₄, wherein, the heteroatoms are selected from: O, and N;
R₁₄, R₁₅ and R₁₆ are independently selected from: H, and C₁-C₅ alkyl;
m, n and p are each independently selected from: an integer from 0 to 5.

2. The Alkynylphenylbenzamide compounds or their pharmaceutically acceptable salts or stereoisomers according to claim 1,
wherein R₄ is selected from: H, halogen, nitro, C₁-C₄ alkyl, halogen-substituted C₁-C₄ alkyl, C₁-C₄ alkoxy, halogen-substituted C₁-C₄ alkoxy, -(CH₂)ₓNR₁₇R₁₈, and 1-3 R₁₉ substituted or unsubstituted imidazolyl, wherein, x is 1, 2 or 3; R₁₇ and R₁₈ together with the attached nitrogen atom form a piperazinyl substituted or unsubstituted by 1-3 R₁₉; each R₁₉ is independently selected from: C₁-C₅ alkyl; or
wherein R₄ is selected from: H, halogen, nitro, methyl, ethyl, propyl, methoxy, ethoxy, propoxy, trifluoromethyl, trifluoroethyl, 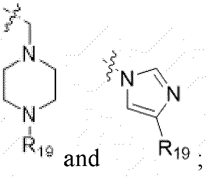 each R₁₉ is independently selected from: methyl, ethyl, and propyl.

3. The Alkynylphenylbenzamide compounds or their pharmaceutically acceptable salts or stereoisomers according to claim 1, wherein
R₆ and R₇ are independently selected from: -(CH₂)ₘNR₈R₉, and -(CH₂)ₚOR₁₂; or R₆ and R₇ together with the attached nitrogen atom form a 1-3 R₁₃ substituted or unsubstituted morpholinyl, pyrrolidinyl, piperidinyl or piperazinyl ;
R₈ and R₉ are independently selected from: H, and C₁-C₅ alkyl; or R₈ and R₉ together with the attached nitrogen atom form a 1-5 R₁₃ substituted or unsubstituted morpholinyl, pyrrolidinyl, piperidinyl or piperazinyl;
R₁₂ is selected from: H, and C₁-C₅ alkyl;
each R₁₃ is independently selected from: H, C₁-C₅ alkyl, C₁-C₅ alkanoyl, hydroxy, -NR₁₅R₁₆, oxetanyl substituted or unsubstituted by 1-2 R₁₄, and morpholinyl substituted or unsubstituted by 1-4 R₁₄;
R₁₄, R₁₅ and R₁₆ are independently selected from: H, and C₁-C₃ alkyl;
m and p are each independently selected from: 1, 2, 3, 4 and 5.

4. The Alkynylphenylbenzamide compounds or their pharmaceutically acceptable salts or stereoisomers according to claim 1, wherein
R₅ is selected from any one of the following groups:

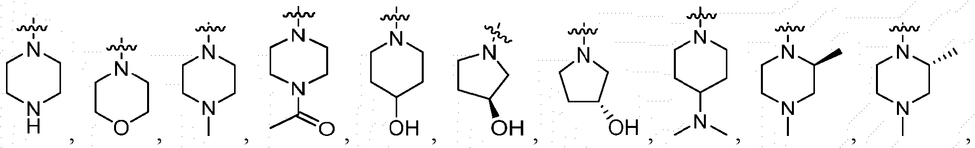

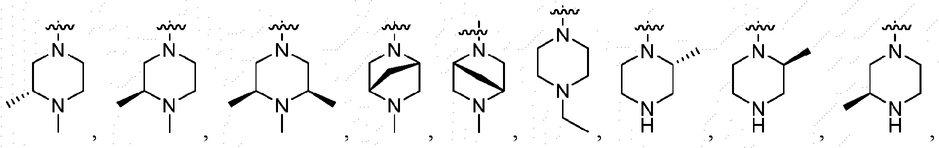

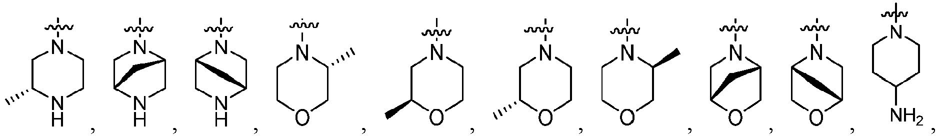

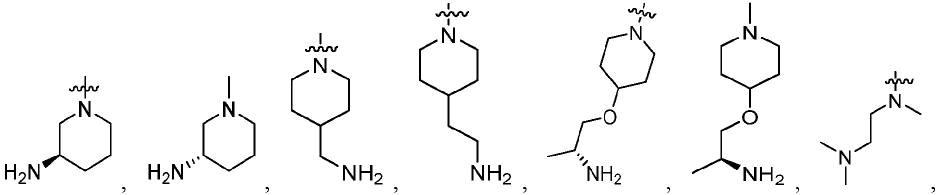

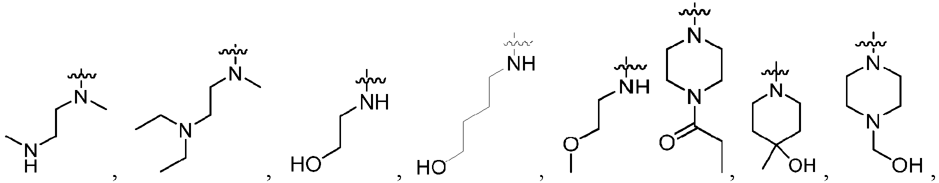

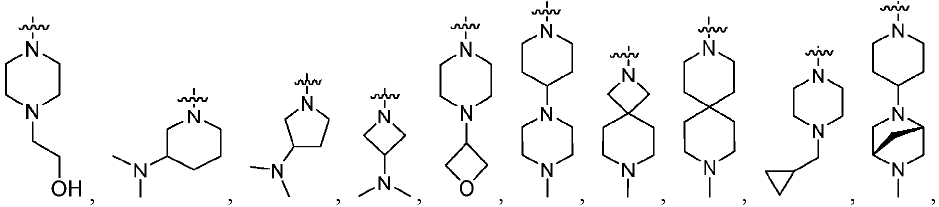

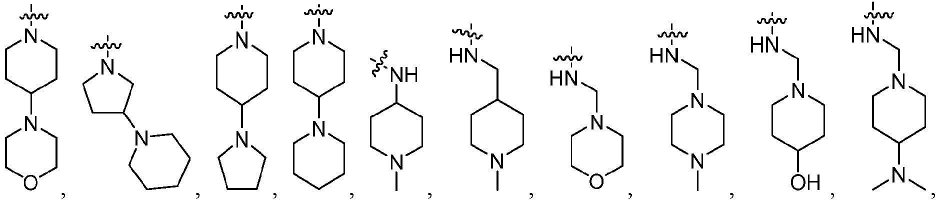

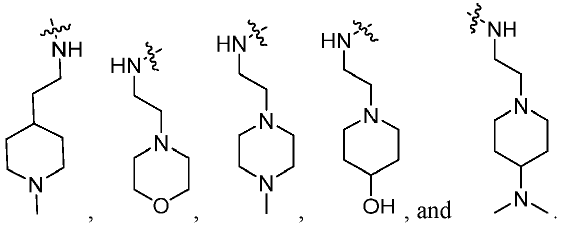

5. The Alkynylphenylbenzamide compounds or their pharmaceutically acceptable salts or stereoisomers according to claim 1,
wherein R₄ is halogen; R₅ is -NR₆R₇; R₆, R₇ are independently selected from: -(CH₂)ₘNR₈R₉, and -(CH₂)ₚOR₁₂; or R₆ and R₇ together with the attached nitrogen atom form a 1-3 R₁₃ substituted or unsubstituted morpholinyl, pyrrolidinyl, piperidinyl or piperazinyl; R₈ and R₉ are independently selected from: H, and C₁-C₃ alkyl; or R₈ and R₉ together with the attached nitrogen atom form a 1-2 R₁₃ substituted or unsubstituted morpholinyl, pyrrolidinyl, piperidinyl or piperazinyl; R₁₂ is selected from: H, and C₁-C₃ alkyl; each R₁₃ is independently selected from: H, C₁-C₃ alkyl, acetyl, hydroxyl, -NR₁₅R₁₆, oxetanyl, and morpholinyl; R₁₅ and R₁₆ are independently selected from: H, and C₁-C₃ alkyl; m and p are each independently selected from: 2, 3 and 4; or
wherein R₄ is Cl, R₅ is selected from:

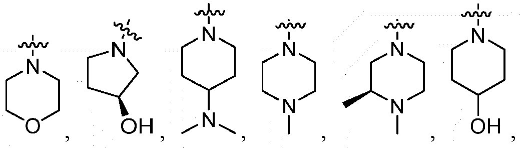

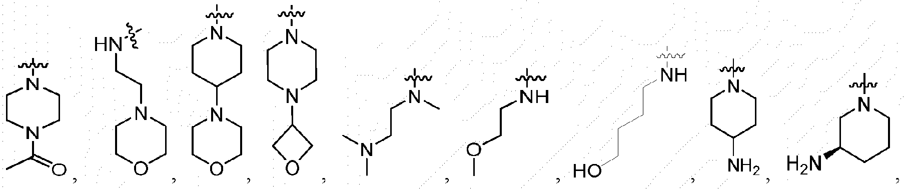

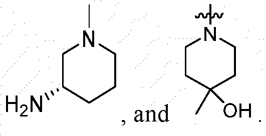

6. The Alkynylphenylbenzamide compounds or their pharmaceutically acceptable salts or stereoisomers according to claim 1, wherein
R₄ is selected from: H, halogen, methyl, methoxy, trifluoromethyl, nitro, and 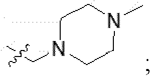 R₅ is 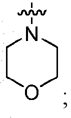 or
R₄ is selected from H, and 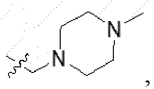 R₅ is selected from 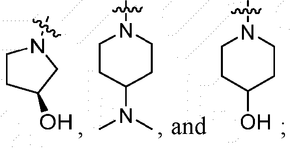 or
R₄ is 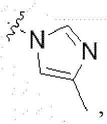 R₅ is selected from

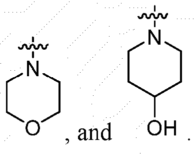

7. The Alkynylphenylbenzamide compounds or their pharmaceutically acceptable salts or stereoisomers according to claim 1, wherein
R₁ is selected from: C₁-C₄ alkyl; and/or,
R₂ is selected from: H, halogen, C₁-C₄ alkyl, halogen-substituted C₁-C₄ alkyl, and C₁-C₄ alkoxy.

8. The Alkynylphenylbenzamide compounds or their pharmaceutically acceptable salts or stereoisomers according to claim 7, wherein
R₁ is selected from: methyl, ethyl, isopropyl, and tert-butyl; and/or,
R₂ is selected from: hydrogen, fluorine, methyl, ethyl, isopropyl, tert-butyl, difluoromethyl, difluoroethyl, trifluoromethyl and trifluoroethyl.

9. The Alkynylphenylbenzamide compounds or their pharmaceutically acceptable salts or stereoisomers according to claim 1, wherein the Alkynylphenylbenzamide compounds have the structure shown in Formula (II): 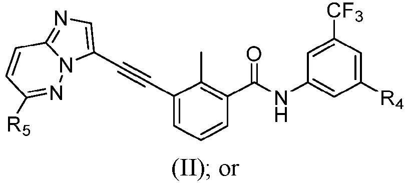 wherein the Alkynylphenylbenzamide compounds are selected from the following compounds:

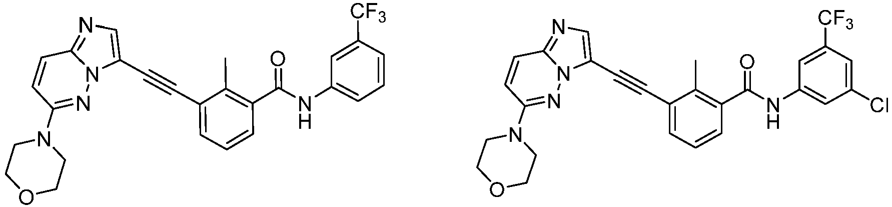

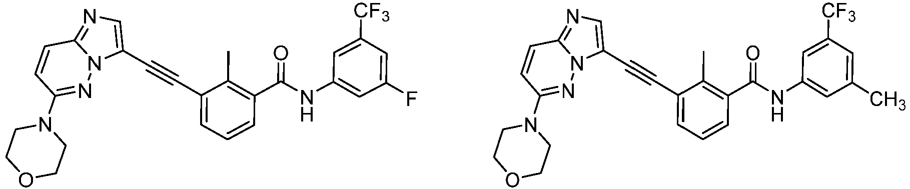

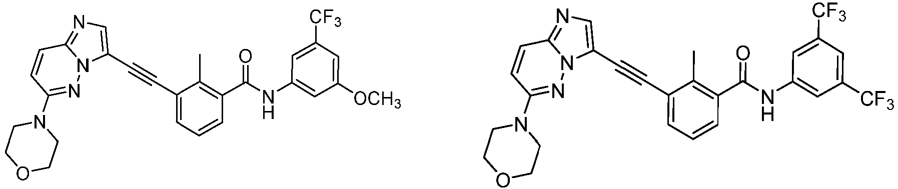

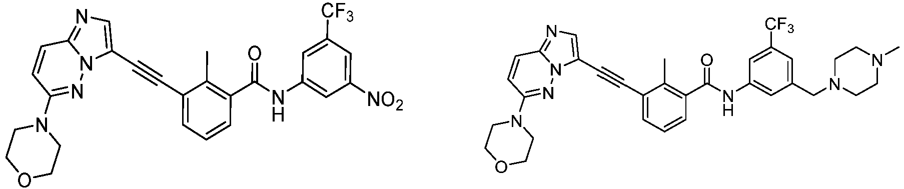

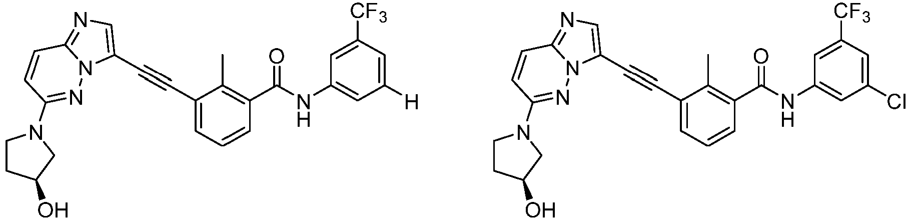

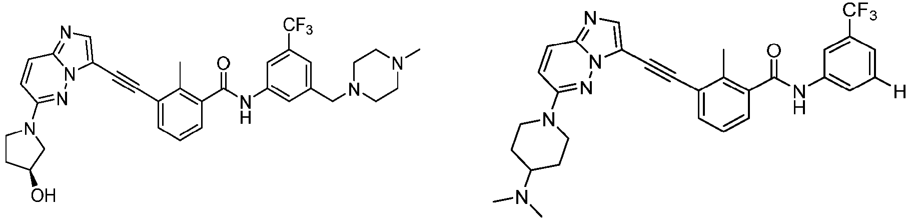

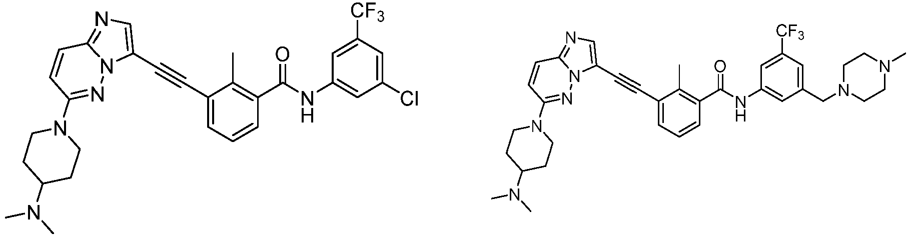

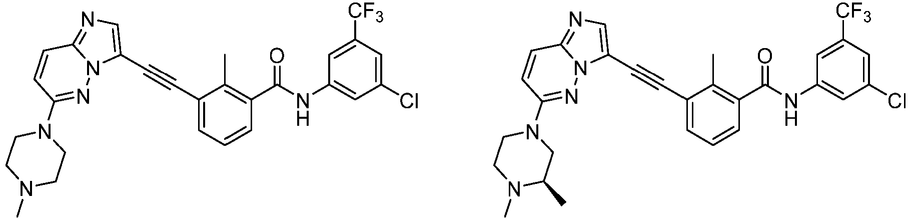

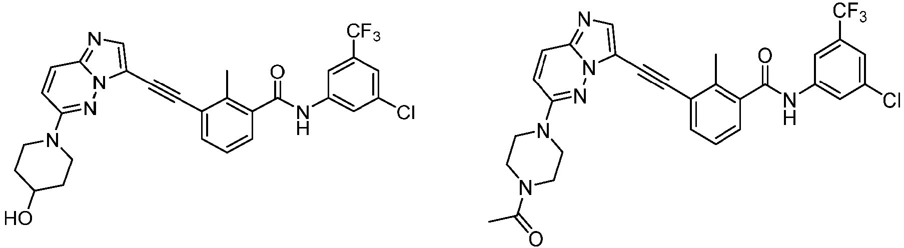

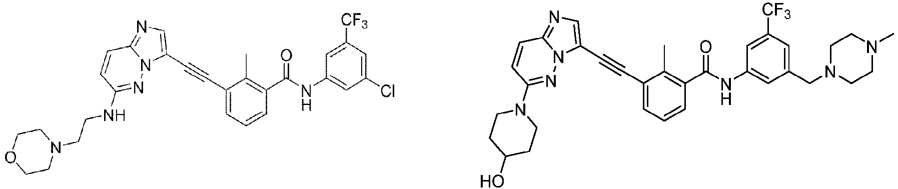

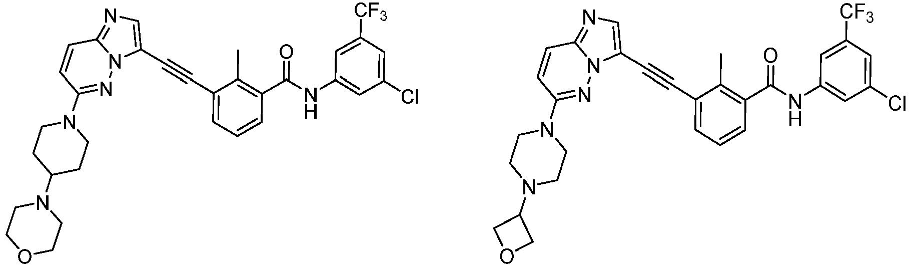

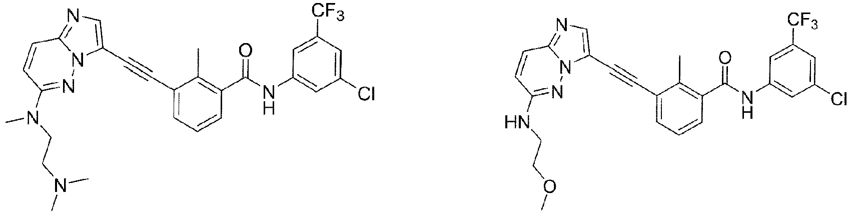

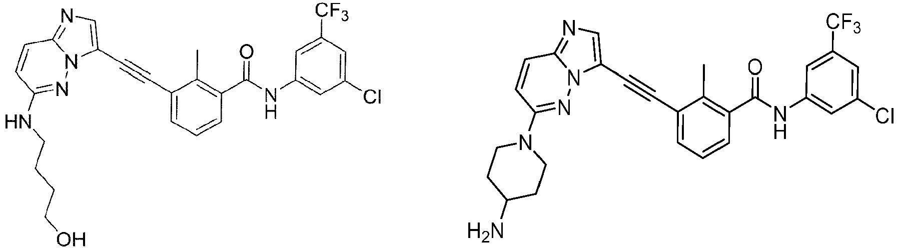

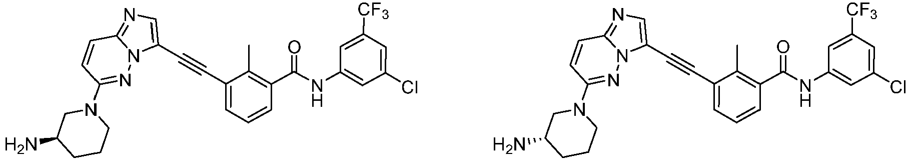

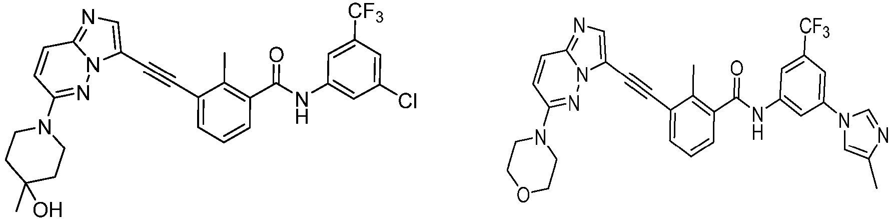

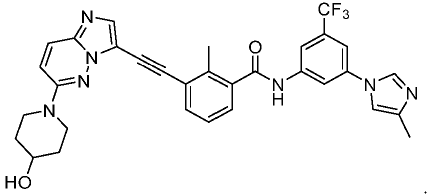

10. Use of the Alkynylphenylbenzamide compounds or their pharmaceutically acceptable salts or stereoisomers according to any one of claims 1-9 in the preparation of a TRK inhibitor.

11. Use of the Alkynylphenylbenzamide compounds or their pharmaceutically acceptable salts or stereoisomers according to any one of claims 1-9 in the preparation of a medicament for preventing and/or treating diseases mediated by TRK tyrosine kinase.

12. The use of claim 11, wherein the disease mediated by TRK tyrosine kinase is cancer, preferably non-small cell lung cancer, breast cancer, colon cancer, prostate cancer, thyroid cancer, malignant melanoma, neuroblastoma and breast-like secretory carcinoma.

13. A pharmaceutical composition for preventing and/or treating tumors, comprising an active ingredient and a pharmaceutically acceptable adjuvant and/or carrier, wherein the active ingredient comprises the Alkynylphenylbenzamide compounds or their pharmaceutically acceptable salts or stereoisomers according to any one of claims 1-9.

## Patentansprüche

1. Alkinylphenylbenzamid-Verbindungen mit der in der Formel (I) gezeigten Struktur oder pharmazeutisch annehmbaren Salze oder Stereoisomere davon: 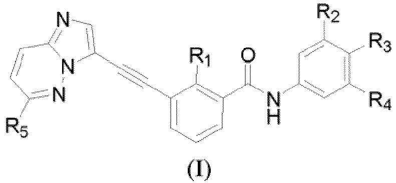 worin R₁ aus C₁-C₂₀-Alkyl ausgewählt ist;
R₂ aus Folgendem ausgewählt ist: H, Halogen, C₁-C₂₀-Alkyl, C₁-C₂₀-Alkoxy und Halogen-substituiertem C₁-C₂₀-Alkyl;
R₃ aus Folgendem ausgewählt ist: H, Difluormethyl, Difluorethyl, Trifluormethyl und Trifluorethyl;
R₄ aus Folgendem ausgewählt ist: H, Halogen, Nitro, C₁-C₈-Alkyl, Halogen-substituiertem C₁-C₈-Alkyl, C₁-C₈-Alkoxy, Halogen-substituiertem C₁-C₈-Alkoxy, -(CH₂)ₓNR₁₇R₁₈, einer 5- bis 6-gliedrigen Heterocyclyl-Gruppe, die 1 bis 3 N-Ringatome enthält und unsubstituiert oder mit 1 bis 5 R₁₉ substituiert ist, und 5- bis 6-gliedrigem Heteroaryl, das 1 bis 3 N-Ringatome enthält und mit 1 bis 5 R₁₉ substituiert oder unsubstituiert ist, worin x eine ganze Zahl von 1 bis 5 ist; wobei R₁₇ und R₁₈ zusammen mit dem gebundenen Stickstoffatom ein mit 1 bis 5 R₁₉ substituiertes oder unsubstituiertes Morpholinyl, Pyrrolidinyl, Piperidinyl oder Piperazinyl bilden und die R₁₉ jeweils unabhängig voneinander aus C₁-C₅-Alkyl ausgewählt sind;
R₅ -NR₆R₇ ist;
worin R₆ und R₇ jeweils unabhängig voneinander aus Folgendem ausgewählt sind: -(CH₂)ₘNR₈R₉, -(CH₂)ₙCR₁₀R₁₁R₁₂ und -(CH₂)ₚOR₁₂, oder R₆ und R₇ zusammen mit dem gebundenen Stickstoffatom einen 3- bis 15-gliedrigen monozyklischen Ring, kondensierten Ring, Spiro-Ring oder verbrückten Ring bilden, der 1 bis 3 Heteroatome enthält und unsubstituiert oder mit 1 bis 5 R₁₃ substituiert ist; wobei die Heteroatome aus den folgenden ausgewählt sind: O, N, S;
R₈ und R₉ jeweils unabhängig voneinander aus Folgendem ausgewählt sind: H und C₁-C₅-Alkyl, oder R₈ und R₉ zusammen mit dem gebundenen Stickstoffatom einen 3- bis 10-gliedrigen monozyklischen Ring, kondensierten Ring, Spiro-Ring oder verbrückten Ring bilden, der 1 bis 3 Heteroatome enthält und unsubstituiert oder mit 1 bis 5 R₁₃ substituiert ist, wobei die Heteroatome aus den folgenden ausgewählt sind: O und N;
R₁₀ und R₁₁ zusammen mit den gebundenen Kohlenstoffatomen einen 3- bis 10-gliedrigen monozyklischen Ring, kondensierten Ring, Spiro-Ring oder verbrückten Ring bilden, der 1 bis 3 Heteroatome enthält und unsubstituiert oder mit 1 bis 5 R₁₃ substituiert ist, wobei die Heteroatome aus den folgenden ausgewählt sind: O und N;
R₁₂ aus Folgendem ausgewählt ist: H und C₁-C₅-Alkyl;
die R₁₃ jeweils unabhängig voneinander aus Folgendem ausgewählt sind: H, C₁-C₅-Alkyl, C₁-C₅-Alkanoyl, Hydroxy, Hydroxy-substituiertem C₁-C₅-Alkyl, Amino-substituiertem C₁-C₅-Alkyl, Amino-substituiertem C₁-C₅-Alkoxy, C₃-C₇-Cycloalkyl-substituiertem C₁-C₃-Alkyl, -NR₁₅R₁₆ und einem 3- bis 10-gliedrigen monozyklischen Ring, kondensierten Ring, Spiro-Ring oder verbrückten Ring, der 1 bis 3 Heteroatome enthält und unsubstituiert oder mit 1 bis 5 R₁₄ substituiert ist, wobei die Heteroatome aus den folgenden ausgewählt sind: O und N;
R₁₄, R₁₅ und R₁₆ jeweils unabhängig voneinander aus Folgendem ausgewählt sind: H und C₁-C₅-Alkyl; und
m, n und p jeweils unabhängig voneinander aus Folgendem ausgewählt sind: einer ganzen Zahl von 0 bis 5.

2. Alkinylphenylbenzamid-Verbindungen oder pharmazeutisch annehmbare Salze oder Stereoisomere davon nach Anspruch 1,
worin R₄ aus Folgendem ausgewählt ist: H, Halogen, Nitro, C₁-C₄-Alkyl, Halogen-substituiertem C₁-C₄-Alkyl, C₁-C₄-Alkoxy, Halogen-substituiertem C₁-C₄-Alkoxy, -(CH₂)ₓNR₁₇R₁₈, und unsubstituiertem oder mit 1 bis 3 R₁₉ substituiertem Imidazolyl, worin x = 1, 2 oder 3 ist, R₁₇ und R₁₈ zusammen mit dem gebundenen Stickstoffatom ein Piperazinyl bilden, das unsubstituiert oder mit 1 bis 3 R₁₉ substituiert ist, die R₁₉ jeweils unabhängig voneinander aus Folgendem ausgewählt sind: C₁-C₅-Alkyl; oder
worin R₄ aus Folgendem ausgewählt ist: H, Halogen, Nitro, Methyl, Ethyl, Propyl, Methoxy, Ethoxy, Propoxy, Trifluormethyl, Trifluorethyl, 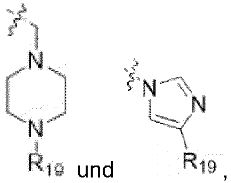 worin die R₁₉ jeweils unabhängig voneinander aus Folgendem ausgewählt sind: Methyl, Ethyl und Propyl.

3. Alkinylphenylbenzamid-Verbindungen oder pharmazeutisch annehmbare Salze oder Stereoisomere davon nach Anspruch 1, worin
R₆ und R₇ jeweils unabhängig voneinander aus Folgendem ausgewählt sind: -(CH₂)ₘNR₈R₉ und -(CH₂)ₚOR₁₂, oder R₆ und R₇ zusammen mit dem gebundenen Stickstoffatom ein unsubstituiertes oder mit 1 bis 3 R₁₃ substituiertes Morpholinyl, Pyrrolidinyl, Piperidinyl oder Piperazinyl bilden;
R₈ und R₉ jeweils unabhängig voneinander aus Folgendem ausgewählt sind: H und C₁-C₅-Alkyl, oder R₈ und R₉ zusammen mit dem gebundenen Stickstoffatom ein unsubstituiertes oder mit 1 bis 5 R₁₃ substituiertes Morpholinyl, Pyrrolidinyl, Piperidinyl oder Piperazinyl bilden;
R₁₂ aus Folgendem ausgewählt ist: H und C₁-C₅-Alkyl;
die R₁₃ jeweils unabhängig voneinander aus Folgendem ausgewählt sind: H, C₁-C₅-Alkyl, C₁-C₅-Alkanoyl, Hydroxy, -NR₁₅R₁₆, Oxetanyl, das unsubstituiert oder mit 1 bis 2 R₁₄ substituiert ist, und Morpholinyl, das unsubstituiert oder mit 1 bis 4 R₁₄ substituiert ist;
R₁₄, R₁₅ und R₁₆ jeweils unabhängig voneinander aus Folgendem ausgewählt sind: H und C₁-C₃-Alkyl; und
m und p jeweils unabhängig voneinander aus Folgendem ausgewählt sind: 1, 2, 3, 4 und 5.

4. Alkinylphenylbenzamid-Verbindungen oder pharmazeutisch annehmbare Salze oder Stereoisomere davon nach Anspruch 1, worin
R₅ aus den folgenden Gruppen ausgewählt ist:

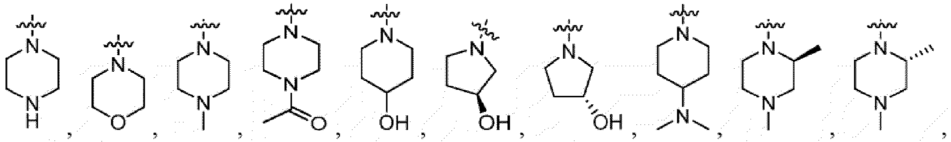

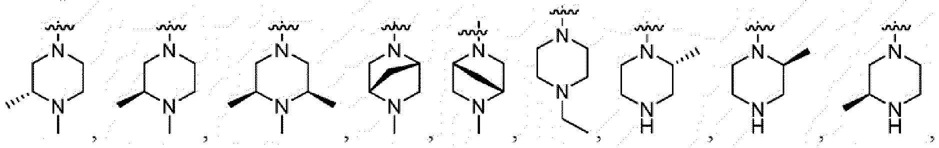

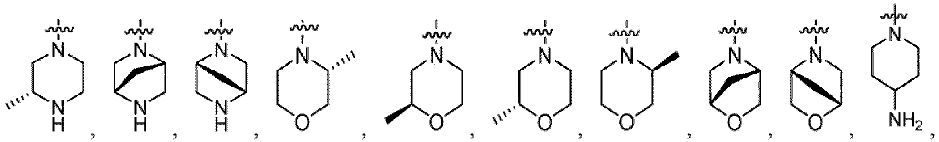

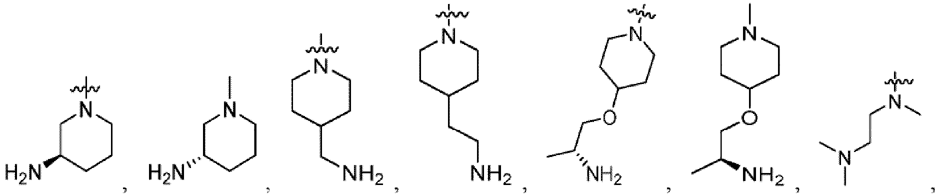

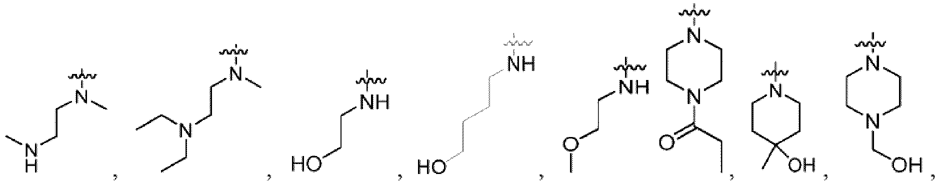

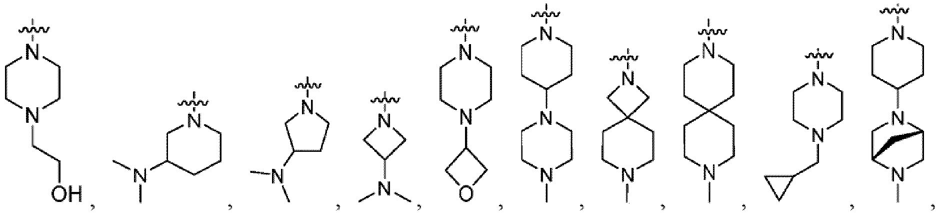

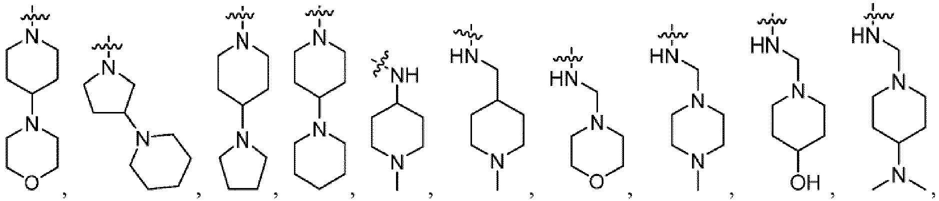

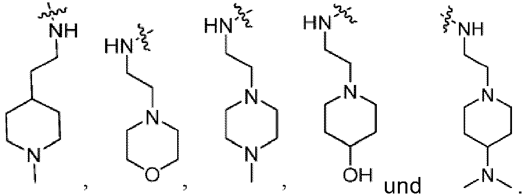

5. Alkinylphenylbenzamid-Verbindungen oder pharmazeutisch annehmbare Salze oder Stereoisomere davon nach Anspruch 1,
worin R₄ Halogen ist, R₅ -NR₆R₇ ist, worin R₆ und R₇ jeweils unabhängig voneinander aus Folgendem ausgewählt sind: -(CH₂)ₘNR₈R₉ und -(CH₂)ₚOR₁₂, oder R₆ und R₇ zusammen mit dem gebundenen Stickstoffatom ein unsubstituiertes oder mit 1 bis 3 R₁₃ substituiertes Morpholinyl, Pyrrolidinyl, Piperidinyl oder Piperazinyl bilden, R₈ und R₉ jeweils unabhängig voneinander aus Folgendem ausgewählt sind: H und C₁-C₃-Alkyl, oder R₈ und R₉ zusammen mit dem gebundenen Stickstoffatom ein unsubstituiertes oder mit 1 bis 2 R₁₃ substituiertes Morpholinyl, Pyrrolidinyl, Piperidinyl oder Piperazinyl bilden, R₁₂ aus Folgendem ausgewählt ist: H und C₁-C₃-Alkyl, die R₁₃ jeweils unabhängig voneinander aus Folgendem ausgewählt sind: H, C₁-C₃-Alkyl, Acetyl, Hydroxyl, -NR₁₅R₁₆, Oxetanyl und Morpholinyl, R₁₅ und R₁₆ jeweils unabhängig voneinander aus Folgendem ausgewählt sind: H und C₁-C₃-Alkyl, m und p jeweils unabhängig voneinander aus Folgendem ausgewählt sind: 2, 3 und 4; oder
worin R₄ Cl ist, R₅ aus Folgendem ausgewählt ist:

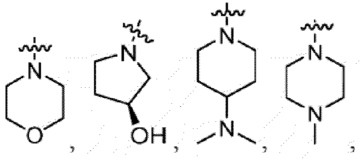

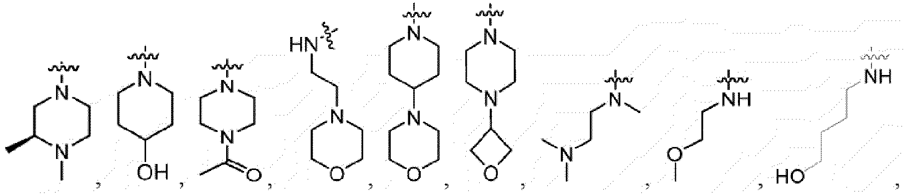

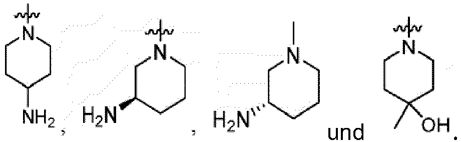

6. Alkinylphenylbenzamid-Verbindungen oder pharmazeutisch annehmbare Salze oder Stereoisomere davon nach Anspruch 1, worin
R₄ aus Folgendem ausgewählt ist: H, Halogen, Methyl, Methoxy, Trifluormethyl, Nitro und 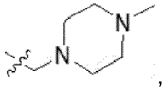
R₅ 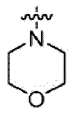 ist; oder
R₄ aus H und 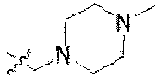 ausgewählt ist, R₅ aus 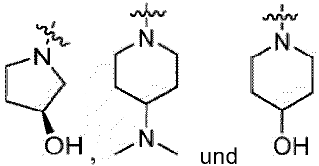 ausgewählt ist; oder
R₄ 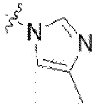 ist, R₅ aus 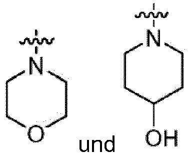 ausgewählt ist.

7. Alkinylphenylbenzamid-Verbindungen oder pharmazeutisch annehmbare Salze oder Stereoisomere davon nach Anspruch 1, worin
R₁ aus Folgendem ausgewählt ist: C₁-C₄-Alkyl; und/oder
R₂ aus Folgendem ausgewählt ist: H, Halogen, C₁-C₄-Alkyl, Halogen-substituiertem C₁-C₄-Alkyl und C₁-C₄-Alkoxy.

8. Alkinylphenylbenzamid-Verbindungen oder pharmazeutisch annehmbare Salze oder Stereoisomere davon nach Anspruch 1, worin
R₁ aus Folgendem ausgewählt ist: Methyl, Ethyl, Isopropyl und tert-Butyl; und/oder
R₂ aus Folgendem ausgewählt ist: Wasserstoff, Fluor, Methyl, Ethyl, Isopropyl, tert-Btyl, Difluormethyl, Difluorethyl, Trifluormethyl und Trifluorethyl.

9. Alkinylphenylbenzamid-Verbindungen oder pharmazeutisch annehmbare Salze oder Stereoisomere davon nach Anspruch 1, wobei die Alkinylphenylbenzamid-Verbindungen die in der Formel (II) gezeigte Struktur aufweisen: 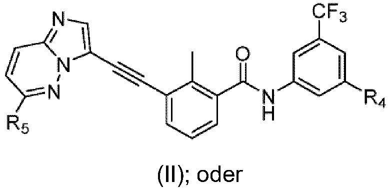 wobei die Alkinylphenylbenzamid-Verbindungen aus den folgenden Verbindungen ausgewählt sind:

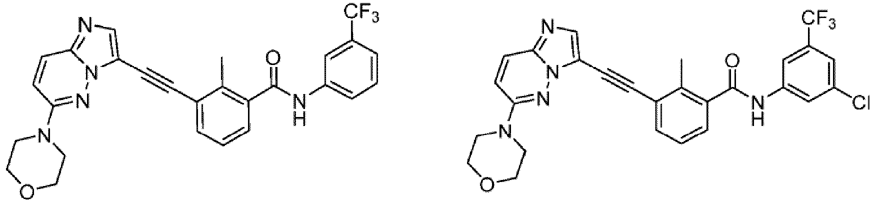

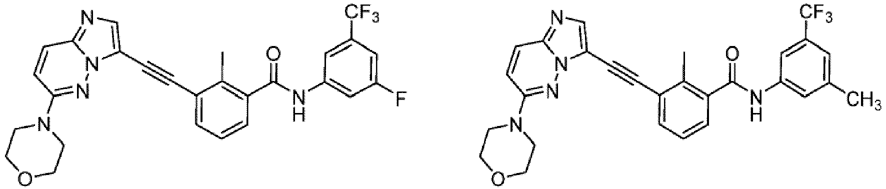

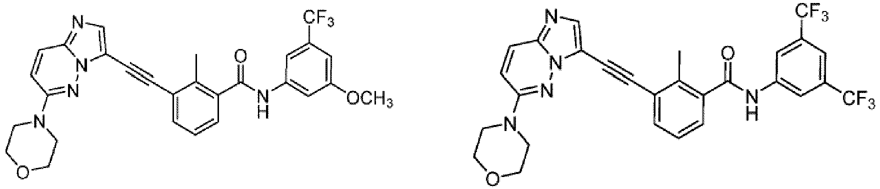

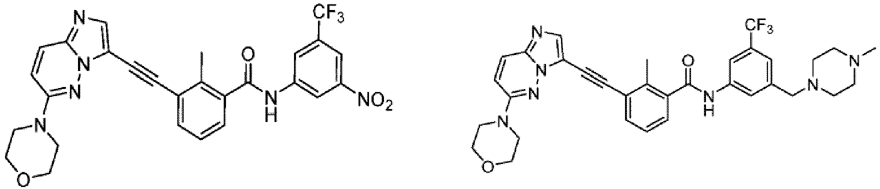

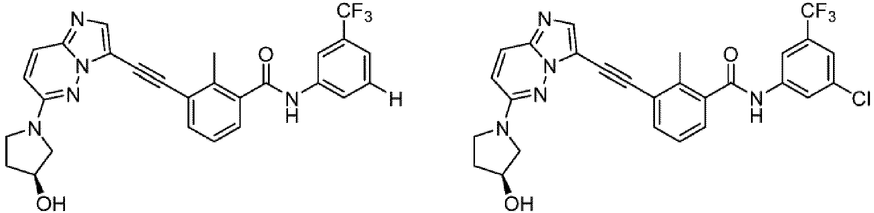

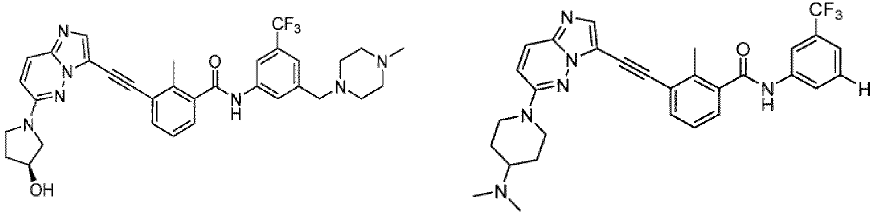

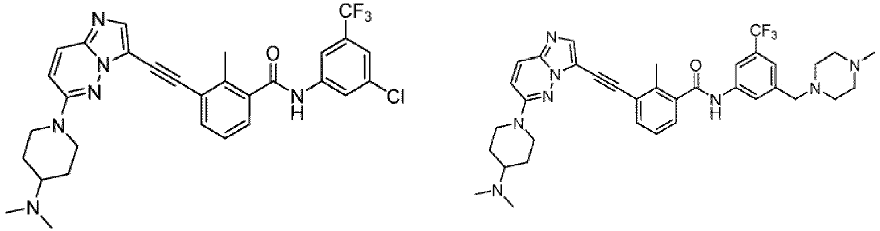

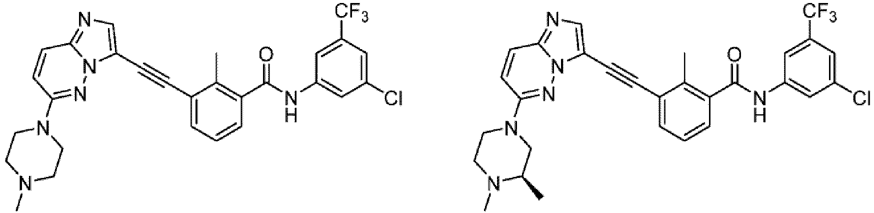

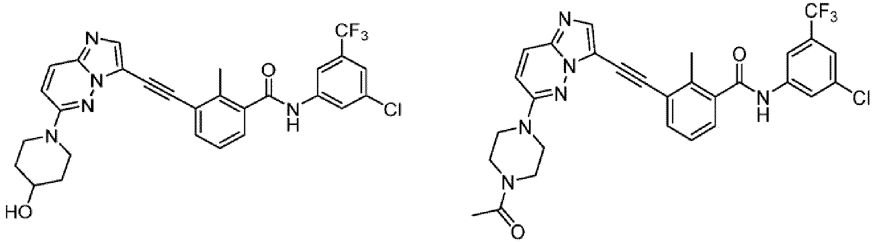

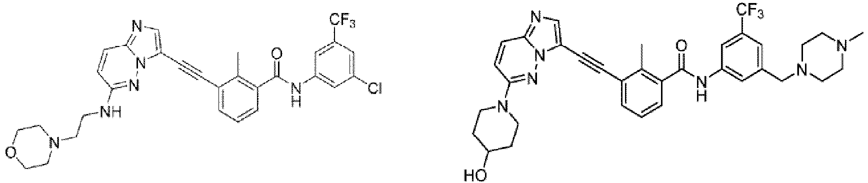

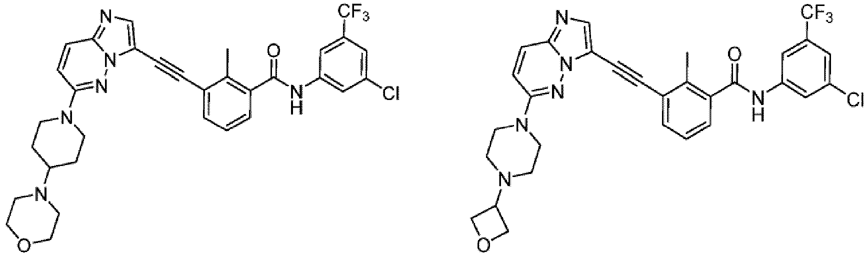

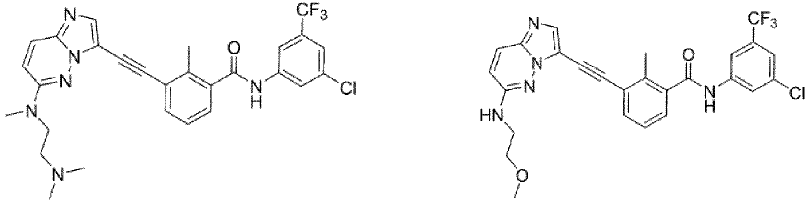

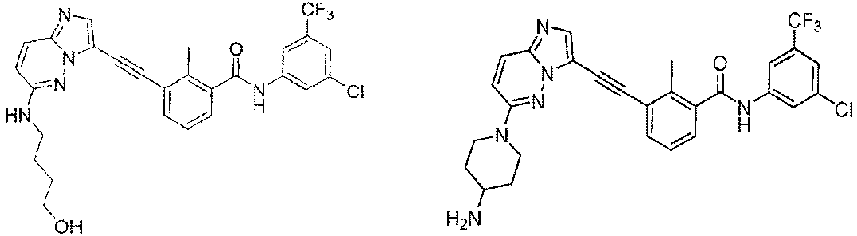

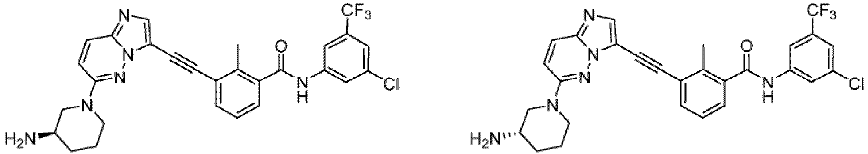

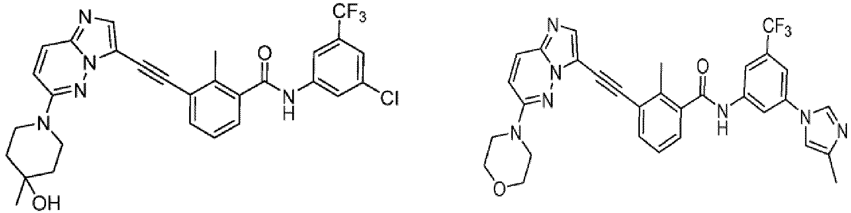

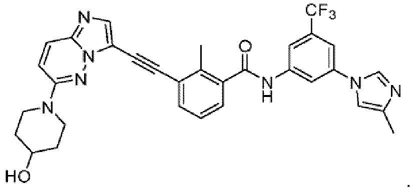

10. Verwendung von Alkinylphenylbenzamid-Verbindungen oder pharmazeutisch annehmbaren Salzen oder Stereoisomeren davon nach einem der Ansprüche 1 bis 9 bei der Herstellung eines TRK-Inhibitors.

11. Verwendung von Alkinylphenylbenzamid-Verbindungen oder pharmazeutisch annehmbaren Salzen oder Stereoisomeren davon nach einem der Ansprüche 1 bis 9 bei der Herstellung eines Medikaments zur Vorbeugung gegen und/oder Behandlung von Erkrankungen, die durch TRK-Tyrosinkinase vermittelt werden.

12. Verwendung nach Anspruch 11, wobei die durch TRK-Tyrosinkinase vermittelte Erkrankung Krebs, vorzugsweise nichtkleinzelliger Lungenkrebs, Brustkrebs, Kolonkrebs, Prostatakrebs, Schilddrüsenkrebs, ein malignes Melanom, ein Neuroblastom oder ein Mamma-analoges sekretorisches Karzinom ist.

13. Pharmazeutische Zusammensetzung zur Vorbeugung gegen und/oder Behandlung von Tumoren, die einen Wirkbestandteil und einen pharmazeutisch annehmbaren Hilfsstoff und/oder Träger umfasst, wobei der Wirkbestandteil Alkinylphenylbenzamid-Verbindungen oder pharmazeutisch annehmbare Salze oder Stereoisomere davon nach einem der Ansprüche 1 bis 9 umfasst.

## Revendications

1. Composés d'alkynylphénylbenzamide avec la structure représentée par la formule (I) ou leurs sels ou stéréoisomères pharmaceutiquement acceptables, 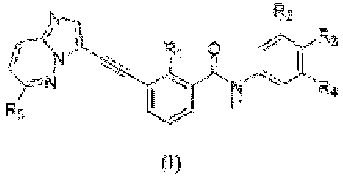
dans lesquels R₁ est choisi parmi : alkyle en C₁₋C₂₀ ;
R₂ est choisi parmi : H, halogène, alkyle en C₁₋C₂₀, alcoxy en C₁₋C₂₀ et alkyle en C₁₋C₂₀ substitué par un halogène ;
R₃ est choisi parmi : H, difluorométhyle, difluoroéthyle, trifluorométhyle et trifluoroéthyle ;
R₄ est choisi parmi : H, halogène, nitro, alkyle en C₁₋C₈, alkyle en C₁₋C₈ substitué par un halogène, alcoxy en C₁₋C₈, alcoxy en C₁-C₈ substitué par un halogène, -(CH₂)ₓNR₁₇R₁₈, un groupe hétérocyclyle de 5 à 6 chaînons contenant de 1 à 3 atomes de cycle N et substitué ou non substitué par 1 à 5 R₁₉, et hétéroaryle de 5 à 6 chaînons contenant de 1 à 3 atomes de cycle N et substitué ou non substitué par 1 à 5 R₁₉, dans lesquels x est un entier de 1 à 5 ; R₁₇ et R₁₈ forment conjointement avec l'atome d'azote auquel ils sont attachés, un morpholinyle, pyrrolidinyle, pipéridinyle ou pipérazinyle substitué ou non substitué par 1 à 5 R₁₉ ; chaque R₁₉ est choisi indépendamment parmi : alkyle en C₁-C₅ ;
R₅ est -NR₆R₇ ;
dans lesquels R₆ et R₇ sont choisis indépendamment parmi : -(CH₂)ₘNR₈R₉, -(CH₂)ₙCR₁₀R₁₁R₁₂ et -(CH₂)ₚOR₁₂ ; ou R₆ et R₇ forment, conjointement avec l'atome d'azote auquel ils sont attachés, un cycle monocyclique de 3 à 15 chaînons, un cycle fusionné, un cycle spiro ou un cycle ponté contenant de 1 à 3 hétéroatomes, et substitués ou non substitués par 1 à 5 R₁₃ ; dans lesquels les hétéroatomes sont choisis parmi : O, N, S ;
R₈ et R₉ sont choisis indépendamment parmi : H, et alkyle en C₁-C₅ ; ou R₈ et R₉ forment, conjointement avec l'atome d'azote auquel ils sont attachés, un cycle monocyclique, un cycle fusionné, un cycle spiro ou un cycle ponté de 3 à 10 chaînons contenant de 1 à 3 hétéroatomes, et substitués ou non substitués par 1 à 5 R₁₃, dans lequel, les hétéroatomes sont choisis parmi : O et N ;
R₁₀ et R₁₁ forment, conjointement avec les atomes de carbone auxquels ils sont attachés, un cycle monocyclique de 3 à 10 chaînons, un cycle fusionné, un cycle spiro ou un cycle ponté contenant de 1 à 3 hétéroatomes, et substitués ou non substitués par 1 à 5 R₁₃, dans lesquels les hétéroatomes sont choisis parmi : O et N ;
R₁₂ est choisi parmi : H, et alkyle en C₁-C₅ ;
chaque R₁₃ est choisi indépendamment parmi : H, alkyle en C₁-C₅, alcanoyle en C₁-C₅, hydroxy, alkyle en C₁-C₅ substitué par un hydroxy, alkyle en C₁-C₅ substitué par un amino, alcoxy en C₁-C₅ substitué par un amino, alkyle en C₁-C₃ substitué par un cycloalkyle en C₃-C₇, -NR₁₅R₁₆ et un cycle monocyclique de 3 à 10 chaînons, un cycle fusionné, un cycle spiro ou un cycle ponté contenant de 1 à 3 hétéroatomes et substitués ou non substitués par 1 à 5 R₁₄, dans lequel les hétéroatomes sont choisis parmi : O et N ;
R₁₄, R₁₅ et R₁₆ sont choisis indépendamment parmi : H, et alkyle en C₁-C₅ ;
m, n et p sont choisis chacun indépendamment parmi : un entier de 0 à 5.

2. Composés d'alkynylphénylbenzamide ou leurs sels ou stéréoisomères pharmaceutiquement acceptables selon la revendication 1,
dans lesquels R₄ est choisi parmi : H, halogène, nitro, alkyle en C₁-C₄, alkyle en C₁-C₄ substitué par un halogène, alcoxy en C₁-C₄, alcoxy en C₁-C₄ substitué par un halogène, - (CH₂ₓNR₁₇R₁₈ et imidazolyle substitué ou non substitué par 1 à 3 R₁₉, dans lesquels x est égal à 1, 2 ou 3 ; R₁₇ et R₁₈ forment, conjointement avec l'atome d'azote auquel ils sont attachés, un pipérazinyle substitué ou non substitué par 1 à 3 R₁₉ ; chaque R₁₉ est choisi indépendamment parmi : alkyle en C₁-C₅ ; ou
dans lesquels R₄ est choisi parmi : H, halogène, nitro, méthyle, éthyle, propyle, méthoxy, éthoxy, propoxy, trifluorométhyle, trifluoroéthyle, 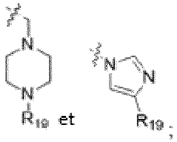 chaque R₁₉ est choisi indépendamment parmi : méthyle, éthyle et propyle.

3. Composés d'alkynylphénylbenzamide ou leurs sels ou stéréoisomères pharmaceutiquement acceptables selon la revendication 1, dans lesquels
R₆ et R₇ sont choisis indépendamment parmi : -(CH₂)ₘNR₈R₉ et - (CH₂)ₚOR₁₂ ; ou R₆ et R₇ forment, conjointement avec l'atome d'azote auquel ils sont attachés, un morpholinyle, pyrrolidinyle, pipéridinyle ou pipérazinyle substitué ou non substitué par 1 à 3 R₁₃ ;
R₈ et R₉ sont choisis indépendamment parmi : H, et alkyle en C₁-C₅ ; ou R₈ et R₉ forment, conjointement avec l'atome d'azote auquel ils sont attachés, un morpholinyle, un pyrrolidinyle, un pipéridinyle ou un pipérazinyle substitué ou non substitué par 1 à 5 R₁₃ ;
R₁₂ est choisi parmi : H, et alkyle en C₁-C₅ ;
chaque R₁₃ est choisi indépendamment parmi : H, alkyle en C₁-C₅, alcanoyle en C₁ -C₅, hydroxy, -NR₁₅R₁₆, oxétanyle substitué ou non substitué par 1-2 R₁₄, et morpholinyle substitué ou non substitué par 1-4 R₁₄ ;
R₁₄, R₁₅ et R₁₆ sont choisis indépendamment parmi : H, et alkyle en C₁-C₃ ;
m et p sont choisis chacun indépendamment parmi : 1, 2, 3, 4 et 5.

4. Composés d'alkynylphénylbenzamide ou leurs sels ou stéréoisomères pharmaceutiquement acceptables selon la revendication 1, dans lesquels
R₅ est choisi dans l'un des groupes suivants :

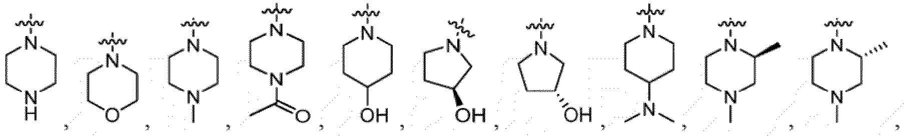

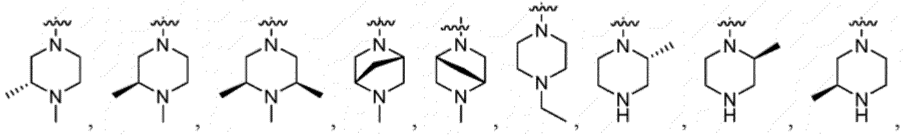

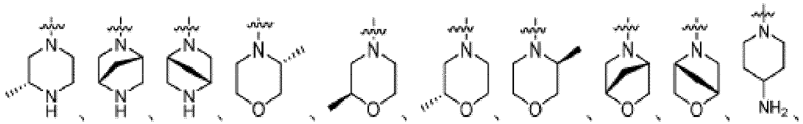

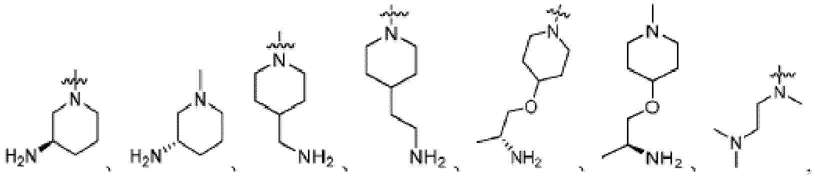

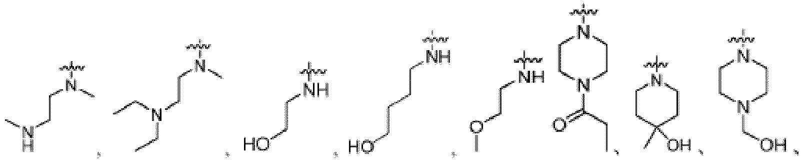

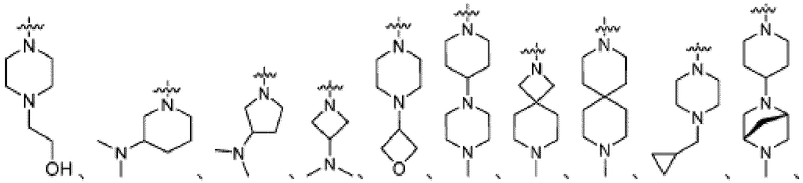

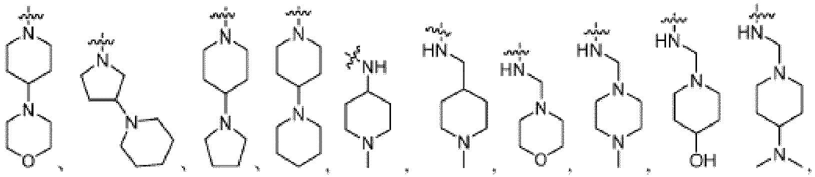

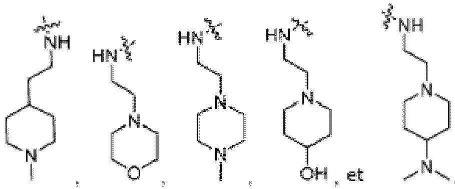

5. Composés d'alkynylphénylbenzamide ou leurs sels ou stéréoisomères pharmaceutiquement acceptables selon la revendication 1,
dans lesquels R₄ est un halogène ; R₅ est -NR₆R₇ ; R₆, R₇ sont choisis indépendamment parmi : -(CH₂)ₘNR₈R₉, et - (CH₂)ₚOR₁₂ ; ou R₆ et R₇ forment, conjointement avec l'atome d'azote auquel ils sont attachés, un morpholinyle, pyrrolidinyle, pipéridinyle ou pipérazinyle substitué ou non substitué par 1 à 3 R₁₃ ; R₈ et R₉ sont choisis indépendamment parmi : H, et alkyle en C₁-C₃ ; ou R₈ et R₉ forment, conjointement avec l'atome d'azote auquel ils sont attachés, un morpholinyle, pyrrolidinyle, pipéridinyle ou pipérazinyle substitué ou non substitué par 1 à 2 R₁₃ ; R₁₂ est choisi parmi : H, et alkyle en C₁-C₃ ; chaque R₁₃ est choisi indépendamment parmi : H, alkyle en C₁-C₃, acétyle, hydroxyle, -NR₁₅R₁₆, oxétanyle et morpholinyle ; R₁₅ et R₁₆ sont choisis indépendamment parmi : H et alkyle en C₁-C₃ ; m et p sont choisis chacun indépendamment parmi : 2, 3 et 4 ; ou
dans lesquels R₄ est Cl, R₅ est choisi parmi :

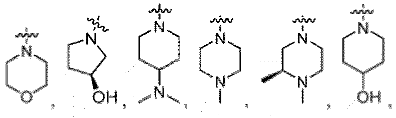

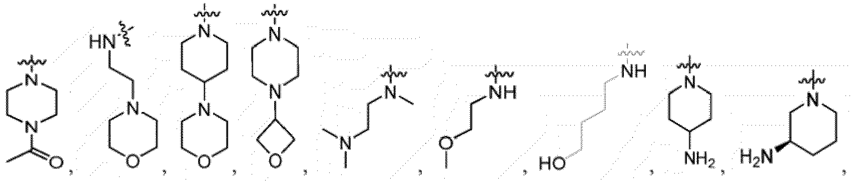

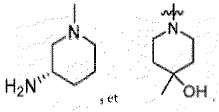

6. Composés d'alkynylphénylbenzamide ou leurs sels ou stéréoisomères pharmaceutiquement acceptables selon la revendication 1, dans lesquels
R₄ est choisi parmi : H, halogène, méthyle, méthoxy, trifluorométhyle, nitro et 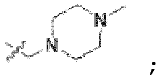
R₅ est 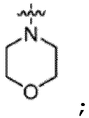 ou
R₄ est choisi parmi H, et 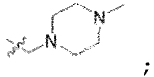 R₅ est choisi parmi 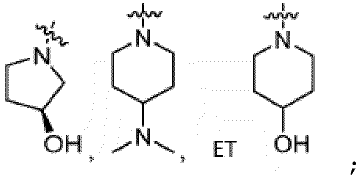
R₄ est 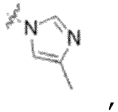 R₅ est choisi parmi

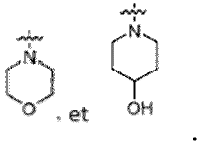

7. Composés d'alkynylphénylbenzamide ou leurs sels ou stéréoisomères pharmaceutiquement acceptables selon la revendication 1, dans lesquels
R₁ est choisi parmi : alkyle en C₁-C₄ ; et/ou
R₂ est choisi parmi : H, halogène, alkyle en C₁-C₄, alkyle en C₁-C₄ substitué par un halogène et alcoxy en C₁-C₄.

8. Composés d'alkynylphénylbenzamide ou leurs sels ou stéréoisomères pharmaceutiquement acceptables selon la revendication 7, dans lesquels
R₁ est choisi parmi : méthyle, éthyle, isopropyle et tert-butyle ; et/ou
R₂ est choisi parmi : hydrogène, fluor, méthyle, éthyle, isopropyle, tert-butyle, difluorométhyle, difluoroéthyle, trifluorométhyle et trifluoroéthyle.

9. Composés d'alkynylphénylbenzamide ou leurs sels ou stéréoisomères pharmaceutiquement acceptables selon la revendication 1, dans lesquels les composés d'alkynylphénylbenzamide présentent la structure représentée dans la formule (II) ; 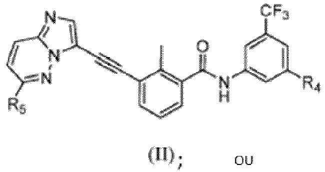 dans lequel les composés d'alkynylphénylbenzamide sont choisis parmi les composés suivants :

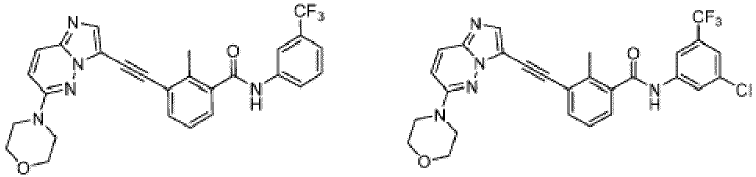

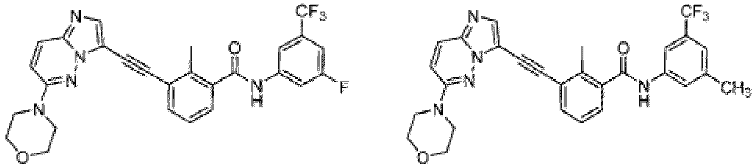

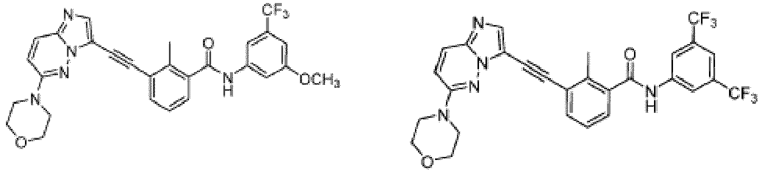

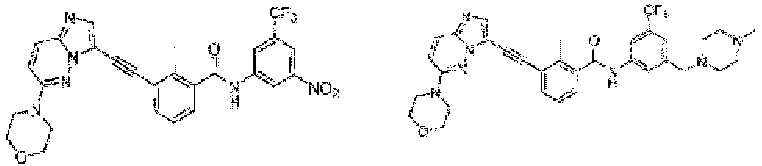

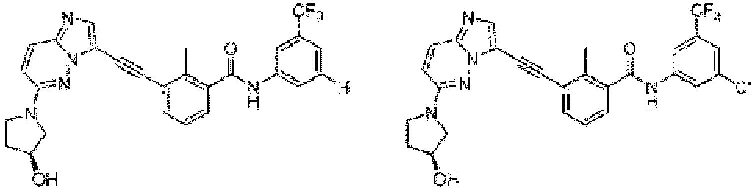

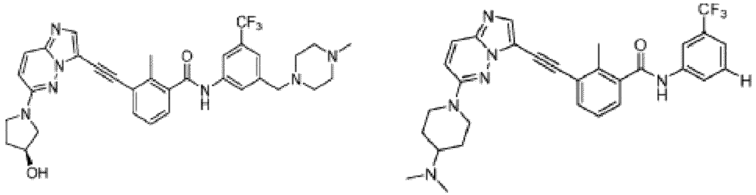

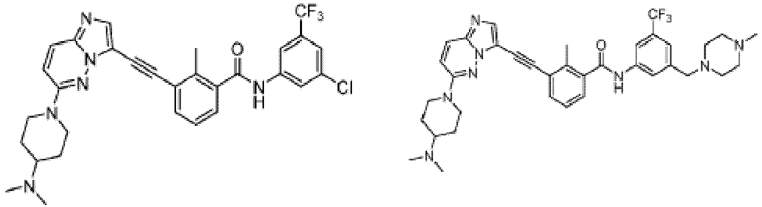

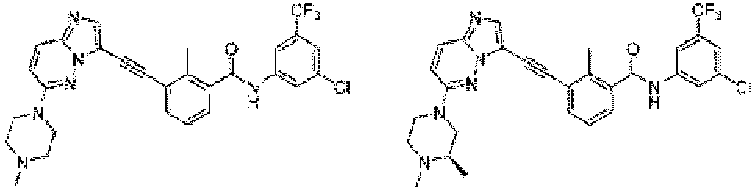

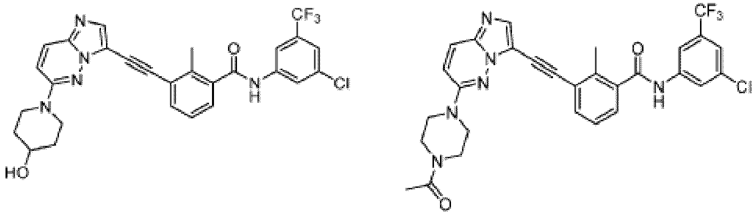

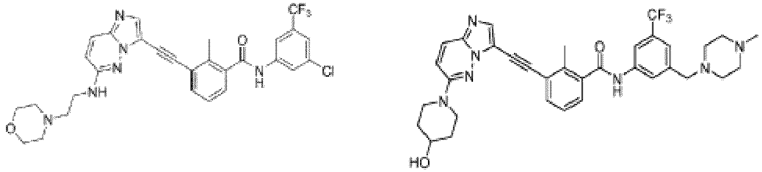

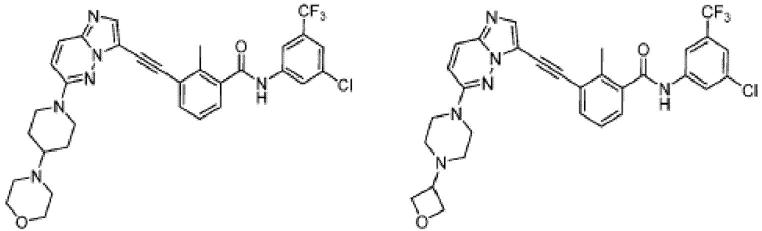

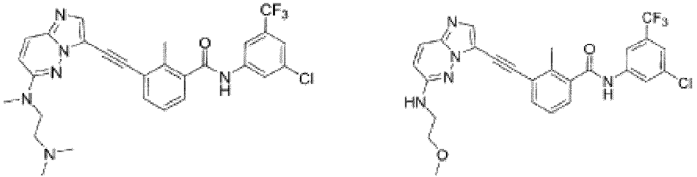

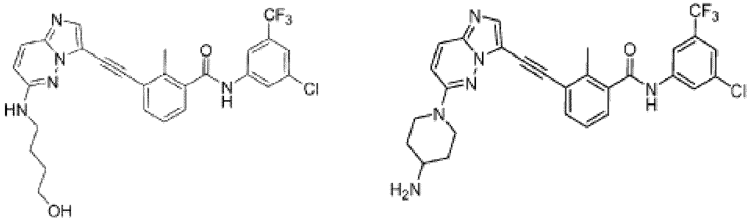

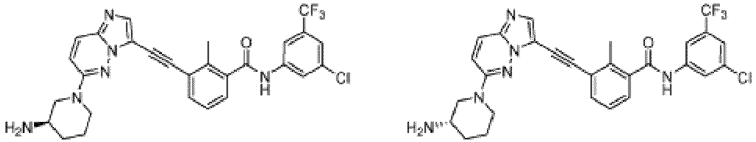

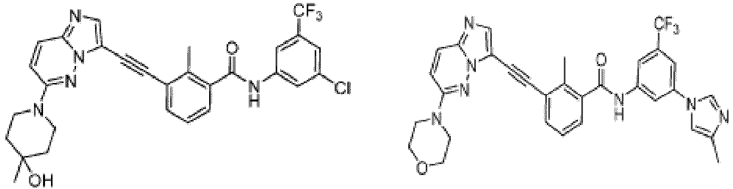

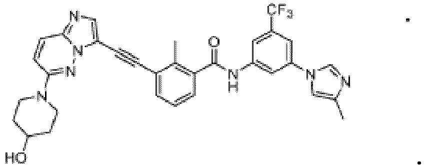

10. Utilisation des composés d'alkylphénylbenzamide ou de leurs sels ou stéréoisomères pharmaceutiquement acceptables selon l'une quelconque des revendications 1 à 9 dans la préparation d'un inhibiteur de TRK.

11. Utilisation des composés d'alkylphénylbenzamide ou de leurs sels ou stéréoisomères pharmaceutiquement acceptables selon l'une quelconque des revendications 1 à 9 dans la préparation d'un médicament pour prévenir et/ou traiter des maladies médiées par la tyrosine kinase TRK.

12. Utilisation selon la revendication 11, dans laquelle la maladie médiée par la tyrosine kinase TRK est le cancer, de préférence le cancer du poumon non à petites cellules, le cancer du sein, le cancer du côlon, le cancer de la prostate, le cancer de la thyroïde, le mélanome malin, le neuroblastome et le carcinome sécrétoire de type mammaire.

13. Composition pharmaceutique pour prévenir et/ou traiter des tumeurs, comprenant un ingrédient actif et un adjuvant et/ou un support pharmaceutiquement acceptable, dans laquelle l'ingrédient actif comprend les composés d'alkylphénylbenzamide ou leurs sels ou stéréoisomères pharmaceutiquement acceptables selon l'une quelconque des revendications 1 à 9.
